# EUROPÄISCHE PATENTANMELDUNG

(11) **EP 4 779 646 A1**
(43) Veröffentlichungstag der Anmeldung: **22.07.2026**
(21) Anmeldenummer: 26150854.3
(22) Anmeldetag: 08.01.2026
(51) Int. Cl.: G16H 10/20

(54) **ERFASSUNGS-ANORDNUNG UND ERFASSUNGS-VERFAHREN ZUM ERFASSEN VON ANTWORTEN EINES BENUTZERS AUF FRAGEN EINES FRAGEBOGENS**

(30) Priorität: 15.01.2025 DE 102025101324
(71) Anmelder: Drägerwerk AG & Co. KGaA, 23558 Lübeck (DE)
(72) Erfinder: Frischke, Meik, 23558 Lübeck (DE); Weber, Nora, 23558 Lübeck (DE); Dennier, Silja, 23558 Lübeck (DE); Franz, Frank, 23558 Lübeck (DE)

(57) **Zusammenfassung**

Die Erfindung betrifft eine Erfassungs-Anordnung (100) und ein Erfassungs-Verfahren, welche die Antworten eines Benutzers (Pt) auf Fragen eines vorgegebenen Fragebogens (Qu) zu erfassen vermögen. Eine Ausgabeeinheit (1, 6) gibt die Fragen aus, und eine Eingabeeinheit (2, 5, 12) erfasst die Antworten des Benutzers (Be). Ein Eingabe-Parameter-Sensor (4, 7, 8, 15, 18) misst für jede Frage einer Fragen-Gruppe, welchen Wert ein zugeordneter Eingabe-Parameter annimmt, während die Eingabeeinheit (2, 5, 12) eine Antwort des Benutzers (Be) auf diese Frage erfasst. Die Erfassungs-Anordnung (100) wendet auf gemessene Eingabe-Parameter-Werte mindestens eine Auswerteregel an und erzeugt Auswertungsdaten, die die Ergebnisse der Anwendung der Auswerteregel umfasst. Die Erfassungs-Anordnung (100) erzeugt Protokolldaten (20) mit den Fragen, den Antworten und den Eingabe-Parameter-Werten sowie eine Darstellungsdatei. Diese Darstellungsdatei umfasst für jede Frage des Fragebogens (Qu) die erfasste Antwort sowie Auswertungsergebnisse.

## Beschreibung

Die Erfindung betrifft eine Erfassungs-Anordnung und ein Erfassungs-Verfahren, welche die Antworten eines Benutzers auf Fragen eines Fragebogens zu erfassen vermögen. Der Fragebogen ist der Erfassungs-Anordnung in einer rechnerauswertbaren Form vorgegeben. Eine Ausgabeeinheit der Erfassungs-Anordnung gibt die Fragen des Fragebogens an einen Benutzer der Erfassungs-Anordnung aus. Eine Eingabeeinheit der Erfassungs-Anordnung erfasst die Antworten, mit denen der Benutzer die Fragen beantwortet hat. Die Möglichkeit wird berücksichtigt, dass ein Benutzer mindestens eine Frage des Fragebogens nicht beantwortet.

Die Aufgabe, Antworten eines Menschen auf Fragen eines Fragebogens zu erfassen, tritt beispielsweise in folgenden beiden Situationen auf:
- Ein Patient soll oder muss sich einer medizinischen Untersuchung und / oder einer medizinischen Behandlung unterziehen. Der Behandlung geht eine Konsultation voraus. Vor der Konsultation füllt der Patient einen vorgegebenen Fragebogen aus.
- Vor einer medizinischen Behandlung führt ein Arzt ein Aufklärungsgespräch mit einem Patienten. Während dieses Gesprächs richtet der Arzt Fragen eines vorgegebenen Fragebogens an den Patienten und erfasst die Antworten des Patienten.

Ein Arzt verwendet die Antworten des Patienten, um zu prüfen, welche Risiken bei der geplanten medizinischen Behandlung bestehen, und um bei Bedarf die geplante medizinische Behandlung so an den Patienten anzupassen, dass die Risiken für den Patienten verringert werden. In beiden Situationen ist es möglich, dass ein tragbarer Rechner, beispielsweise ein Tablet, die Fragen auf einem Bildschirm darstellt und der Patient (erste Situation) bzw. der Arzt (zweite Situation) die Antworten des Patienten in den Rechner eingibt.

Der Erfindung liegt die Aufgabe zugrunde, eine Erfassungs-Anordnung und ein Erfassungs-Verfahren bereitzustellen, welche die Antworten (allgemeiner: die Reaktionen) eines Benutzers auf Fragen eines Fragebogens zu erfassen vermögen. Der Fragebogen ist in einer rechnerauswertbaren Form vorgegeben, und die Fragen des Fragebogens beziehen sich auf eine Person, beispielsweise auf einen Patienten. Eine Ausgabeeinheit der Erfassungs-Anordnung gibt die Fragen des Fragebogens an einen Benutzer der Erfassungs-Anordnung aus, und zwar insbesondere visuell und / oder durch eine Sprachausgabe. Eine Eingabeeinheit der Erfassungs-Anordnung erfasst die Antworten, die der Benutzer eingegeben hat, und zwar insbesondere mittels eines Bildschirms, bevorzugt eines berührungssensitiven Bildschirms, und einer Tastatur und / oder eines Touchpads und / oder per Spracheingabe. Die Erfassungs-Anordnung und das Erfassungs-Verfahren sollen die Gefahr reduzieren, dass ein medizinisches Risiko für die Person nicht entdeckt wird.

Die Aufgabe wird durch eine Erfassungs-Anordnung mit den Merkmalen des Anspruchs 1 und durch ein Erfassungs-Verfahren mit den Merkmalen des Anspruchs 25 gelöst. Vorteilhafte Ausgestaltungen sind in den Unteransprüchen angegeben. Vorteilhafte Ausgestaltungen der erfindungsgemäßen Erfassungs-Anordnung sind, soweit sinnvoll, auch vorteilhafte Ausgestaltungen des erfindungsgemäßen Erfassungs-Verfahrens und umgekehrt.

Die Erfassungs-Anordnung umfasst ein signalverarbeitendes Gerät, bevorzugt ein tragbares signalverarbeitendes Gerät. Das Erfassungs-Verfahren wird unter Verwendung einer solchen Erfassungs-Anordnung durchgeführt.

In einer rechnerauswertbaren Form ist der Erfassungs-Anordnung ein Fragebogen mit mehreren Fragen vorgegeben. Der Fragebogen bezieht sich auf eine Person. Möglich ist, dass der gleiche Fragebogen für eine Vielzahl von Personen wiederverwendet wird. Möglich ist auch, dass ein Fragebogen vorgegeben wird, der für eine bestimmte Person konfiguriert und / oder zugeschnitten oder eigens angefertigt worden ist, beispielsweise durch Streichungen von Fragen aus einem vorgegebenen Maximal-Fragebogen.

Eine Ausgabeeinheit der erfindungsgemäßen Erfassungs-Anordnung vermag die Fragen des Fragebogens an einen Benutzer auszugeben, und zwar in mindestens einer von einem Menschen wahrnehmbaren Form, insbesondere visuell und / oder akustisch. Die Erfassungs-Anordnung ist so ausgestaltet, dass die Ausgabeeinheit mindestens eine, bevorzugt jede Frage des Fragebogens ausgibt. Optional vermag die Ausgabeeinheit die Fragen auf unterschiedliche Weisen auszugeben, insbesondere auf eine Weise, die von einer entsprechenden Benutzervorgabe abhängt. Insbesondere ist in einer Ausgestaltung möglich, dass die Ausgabeeinheit eine Frage auf mindestens zwei verschiedene Weisen ausgeben kann und ein Benutzer auswählen kann, auf welche Weise die Ausgabeeinheit ihm eine Frage tatsächlich ausgeben soll. Möglich ist, dass in jedem Fall alle Fragen des Fragebogens ausgegeben werden. Möglich ist auch, dass es von den eingegebenen und erfassten Antworten auf früher ausgegebene Fragen abhängt, welche Fragen die Ausgabeeinheit in der Folge ausgibt.

Eine Eingabeeinheit vermag die jeweilige Antwort eines Benutzers der Erfassungs-Anordnung auf eine Frage zu erfassen. Diese Frage ist im Fragebogen enthalten und wurde mittels der Ausgabeeinheit ausgegeben. Möglich ist, dass die Eingabeeinheit eine Antwort auf mindestens zwei verschiedene Weisen zu erfassen vermag und ein Benutzer auswählen kann, auf welche Weise er eine Antwort in die Eingabeeinheit eingibt. Möglich ist auch, dass die Eingabeeinheit automatisch erkennt, auf welche Weise eine Antwort eingegeben worden ist. Ein Sonderfall einer erfassten Antwort ist, dass erfasst wird, dass der Benutzer in einem Zeitraum von einer vorgegeben Länge überhaupt keine Antwort eingibt.

Die Eingabeeinheit ist so ausgestaltet, dass der Benutzer die Antwort insbesondere durch eine Eingabe mittels einer Tastatur, einer Auswahleinheit, eines Bildschirmstifts und / oder einer Spracheingabeeinheit eingeben kann. Die Tastatur kann insbesondere eine physikalische Tastatur und / oder eine Bildschirmtastatur und / oder ein Touchpad umfassen. Möglich ist, dass die Eingabeeinheit das Ereignis erfasst, dass ein Benutzer keine Antwort auf eine Frage eingibt. Beispielsweise gibt der Benutzer explizit ein, diese Frage nicht zu beantworten. Oder seit der Ausgabe der Frage ist eine Zeitspanne von einer vorgegebenen Dauer verstrichen, ohne dass die Eingabeeinheit in dieser Zeitspanne eine Antwort auf die Frage erfasst hat.

Anmerkung: Die Bezeichnung "Benutzer" bezieht sich auf einen Menschen, welcher die Erfassungs-Anordnung verwendet, um Antworten auf die Fragen des vorgegebenen Fragebogens einzugeben. Die Fragen des Fragebogens beziehen sich auf eine Person, beispielsweise auf einen Patienten. Der Benutzer selbst kann die Person sein, auf die sich die Fragen beziehen. Möglich ist auch, dass jemand anders als diese Person die oder wenigstens einige der Fragen beantwortet. Möglich ist auch, dass die Antworten insgesamt von mindestens zwei verschiedenen Benutzern eingegeben werden.

Weiterhin ist der Erfassungs-Anordnung mindestens ein Eingabe-Parameter vorgegeben. Bevorzugt sind mehrere Eingabe-Parameter vorgegeben. Der oder jeder Eingabe-Parameter beschreibt jeweils ein messbares Verhalten eines Benutzers der Eingabeeinheit bei der Beantwortung einer Frage - genauer gesagt: sein Verhalten in dem Zeitraum, in dem die Frage ausgegeben wird und der Benutzer eine Antwort auf die Frage in die Eingabeeinheit eingibt oder die Frage nicht beantwortet oder feststeht, dass die Frage nicht beantwortet wird. Der Eingabe-Parameter nimmt für jede Frage jeweils einen Wert an oder kann wenigstens einen Wert annehmen. Ein Eingabe-Parameter kann für verschiedene Fragen unterschiedliche Werte annehmen. Zwei verschiedene Eingabe-Parameter können für dieselbe Frage unterschiedliche Werte annehmen.

Die Erfassungs-Anordnung umfasst mindestens einen Eingabe-Parameter-Sensor, bevorzugt mehrere Eingabe-Parameter-Sensoren. Jeder Eingabe-Parameter-Sensor ist jeweils dem oder mindestens einem Eingabe-Parameter zugeordnet. Möglich ist, dass derselbe Eingabe-Parameter-Sensor mehreren Eingabe-Parametern zugeordnet ist und / oder umgekehrt demselben Eingabe-Parameter mehrere Eingabe-Parameter-Sensoren zugeordnet sind.

Vorgegeben ist eine Fragen-Gruppe. Die Fragen-Gruppe umfasst mindestens eine Frage des vorgegebenen Fragebogens, bevorzugt mehrere Fragen, optional jede Frage des Fragebogens.

Der Eingabe-Parameter-Sensor vermag für jede Frage der vorgegebenen Fragen-Gruppe jeweils automatisch zu messen, welchen Wert der oder jeder dem Sensor zugeordnete Eingabe-Parameter annimmt, während eine Antwort auf diese Frage eingegeben wird. Der Eingabe-Parameter-Sensor führt also diese Messung in einem Beantwortungs-Zeitraum für diese Frage durch. Der Beantwortungs-Zeitraum beginnt mit dem Abschluss der Ausgabe der Frage und endet mit dem Abschluss der Erfassung der Antwort auf diese Frage oder dann, wenn feststeht, dass der Benutzer die Frage nicht beantwortet. In diesem Beantwortungs-Zeitraum erfasst die Eingabeeinheit eine Antwort oder sonstige Reaktion des Benutzers auf die Frage. Der Eingabe-Parameter-Sensor liefert daher für jede Frage der Fragen-Gruppe jeweils mindestens einen Wert, nämlich den gemessenen Wert, den der Eingabe-Parameter im Beantwortungs-Zeitraum angenommen hat. Falls derselbe Eingabe-Parameter-Sensor verschiedenen Eingabe-Parametern zugeordnet ist, so liefert er für jeden Eingabe-Parameter und jede Frage jeweils einen Wert. Möglich ist, dass der Zeitraum, in dem ein Eingabe-Parameter-Sensor einen Eingabe-Parameter misst, mindestens zwei Beantwortungs-Zeiträume überdeckt. Der gemessene Wert gilt dann für jede Frage, die in einem dieser Beantwortungs-Zeiträume beantwortet worden ist. Bei mehreren Eingabe-Parameter-Sensoren liefert jeder Sensor für jede Frage der Fragen-Gruppe und für jeweils mindestens einen Eingabe-Parameter jeweils einen Wert.

Vorteilhafte Ausgestaltungen, die nachfolgend beschrieben werden, spezifizieren unterschiedliche mögliche Eingabe-Parameter und / oder Eingabe-Parameter-Sensoren.

In einer Ausgestaltung gehört mindestens eine Frage des Fragebogens zur Fragen-Gruppe und mindestens eine weitere Frage des Fragebogens nicht zur Fragen-Gruppe. Das Merkmal, dass eine Fragen-Gruppe vorgegeben ist und nicht notwendigerweise jede Frage des Fragebogens zur Fragen-Gruppe gehört, erspart bei dieser Ausgestaltung die Notwendigkeit, dass jeder Eingabe-Parameter bei der Beantwortung jeder Frage des Fragebogens erneut gemessen wird.

Das erfindungsgemäße Erfassungs-Verfahren umfasst den Schritt, dass der oder mindestens ein, bevorzugt jeder Eingabe-Parameter-Sensor für jede Frage der Fragen-Gruppe misst, welchen Wert der oder jeweils ein Eingabe-Parameter im Beantwortungs-Zeitraum für die Frage annimmt.

Anmerkung: Die Formulierung wird verwendet, dass ein Sensor eine physikalische Größe zu messen vermag, beispielsweise die Dauer eines Beantwortungs-Zeitraums oder Korrekturen oder Rückfragen oder einen Vitalparameter des Benutzers. Diese Formulierung bedeutet, dass der Sensor direkt die physikalische Größe zu messen vermag oder mindestens eine andere Größe, die mit der zu messenden Größe korreliert. Die oder eine gemessene andere Größe oder die Kombination der gemessenen anderen Größen sind daher ein Maß für die zu messende physikalische Größe. Die Messung liefert mindestens einen Wert für die gesuchte physikalische Größe.

Ein signalverarbeitender Protokoll-Generierer der Erfassungs-Anordnung vermag automatisch Protokolldaten zu erzeugen. Die Protokolldaten umfassen in einer rechnerauswertbaren Form für jede ausgegebene Frage des Fragebogens die folgenden Informationen:
- eine Kennzeichnung der Frage und
- entweder eine Kennzeichnung der Antwort, welche die Eingabeeinheit erfasst hat, oder eine Kennzeichnung, dass keine Antwort auf diese Frage erfasst worden ist.

Weiterhin umfassen die Protokolldaten in einer rechnerauswertbaren Form für jede Frage der Fragen-Gruppe jeweils eine Kennzeichnung des oder jedes Eingabe-Parameter-Werts, den der Eingabe-Parameter-Sensor für diese Frage gemessen hat - genauer gesagt: im Beantwortungs-Zeitraum für diese Frage gemessen hat. Bevorzugt umfassen die Protokolldaten idealerweise dann, wenn die Fragen-Gruppe n Fragen umfasst, mindestens n Kennzeichnungen der mindestens n Eingabe-Parameter-Werten. Möglich ist, dass die Protokolldaten für dieselbe Frage mindestens zwei gemessene Eingabe-Parameter-Werte umfasst.

In einer rechnerauswertbaren Form ist mindestens eine Auswerteregel vorgegeben. Die oder jede Auswerteregel hängt von jeweils mindestens einem Eingabe-Parameter ab, bezieht sich also auf den Eingabe-Parameter. Dieselbe Auswerteregel kann sich auf mehrere Eingabe-Parameter beziehen. Falls mehrere Auswerteregeln vorgegeben sind, können sich zwei vorgegebene Auswerteregeln auf denselben Eingabe-Parameter oder auf zwei verschiedene Eingabe-Parameter beziehen. Bezieht sich die Auswerteregel auf einen einzigen Eingabe-Parameter und wird auf einen gemessenen Wert dieses Eingabe-Parameters angewendet, so liefert die Auswerteregel ein Auswertungsergebnis. Bezieht sie sich auf mindestens zwei Eingabe-Parameter und wird auf eine Kombination der gemessenen Werte dieser Eingabe-Parameter angewendet, so liefert die Auswerteregel ebenfalls ein Auswertungsergebnis. Beispielsweise liefert die Auswerteregel ein gewichtetes Mittel über die Werte des Eingabe-Parameters, die für die Fragen der Fragen-Gruppe gemessen worden sind, oder entscheidet, bei welchen Fragen der Eingabe-Parameter einen Wert angenommen hat, der außerhalb eines für diesen Eingabe-Parameter vorgegebenen Soll-Wertbereichs liegt. Eine Anwendung einer Auswerteregel liefert also ein Auswertungsergebnis, wobei das Auswertungsergebnis sich auf mindestens eine Frage der Fragen-Gruppe bezieht und eine automatisch generierte Bewertung des Nutzerverhaltens bei der Beantwortung dieser Frage bedeutet.

Eine signalverarbeitende Erfassungs-Auswerteeinheit der Erfassungs-Anordnung vermag automatisch die oder mindestens eine, bevorzugt jede Auswerteregel auf die gemessenen Werte der Eingabe-Parameter, von denen die Auswerteregel jeweils abhängt, anzuwenden. Die Erfassungs-Auswerteeinheit vermag Auswertungsdaten zu erzeugen. Diese Auswertungsdaten umfassen in einer rechnerauswertbaren Form das jeweilige Auswertungsergebnis, das die Erfassungs-Auswerteeinheit bei der Anwendung einer Auswerteregel erzielt hat. Bevorzugt umfassen die Auswertungsdaten jedes Auswertungsergebnis.

Ein Darstellungs-Generierer der Erfassungs-Anordnung vermag automatisch mindestens eine Darstellungsdatei zu erzeugen. Die oder jede erzeugte Darstellungsdatei umfasst in einer rechnerauswertbaren Form für jede Frage der Fragen-Gruppe folgende Information:
- die Frage,
- die erfasste Antwort auf diese Frage oder die Aussage, dass keine Antwort auf diese Frage erfasst worden ist, sowie
- das oder mindestens ein, bevorzugt jedes Auswertungsergebnis, welche sich auf diese Frage bezieht, und optional
- den oder mindestens einen, bevorzugt jeden gemessenen Eingabe-Parameter-Wert oder eine Information über den Eingabe-Parameter-Wert, der bei der Beantwortung dieser Frage gemessen wurde.

Um die Darstellungsdatei zu erzeugen, verwendet der Darstellungs-Generierer die Protokolldaten und die Auswertungsdaten. Die Darstellungsdatei wird so erzeugt, dass sie wie folgt ausgestaltet ist: Auf Basis der Darstellungsdatei lässt sich mittels eines geeigneten datenverarbeitenden Geräts eine Darstellung generieren und ausgeben, und zwar in mindestens einer von einem Menschen wahrnehmbaren Form, insbesondere visuell. Die ausgegebene Darstellung stellt die oder wenigstens einige der Informationen dar, die in der erzeugten Darstellungsdatei enthalten sind, oder kann wenigstens diese Informationen darstellen. Bevorzugt kann ein Betrachter der Darstellung dargestellte Informationen ausblenden oder vorgeben, dass anfangs nicht dargestellte Informationen eingeblendet werden sollen. Die Darstellung kann zusätzlich Informationen darstellen, die nicht in der Darstellungsdatei enthalten sind, insbesondere abgespeicherte Informationen über eine Person, auf die sich der Fragebogen bezieht.

Möglich ist, dass mehrere Darstellungsdateien erzeugt werden, die sich hinsichtlich ihrer Granularität oder auch hinsichtlich eines Rechners, auf dem sie ausgewertet werden können, unterscheiden.

Nachfolgend wird abkürzend die Formulierung verwendet, dass eine Darstellung auf Basis der erzeugten Darstellungsdatei ausgegeben wird oder ausgegeben werden kann.

Anmerkung: Die Begriffe "Protokolldaten", "Auswertungsdaten" und "Darstellungsdatei" können Daten bezeichnen, die als Datei dauerhaft auf einen Datenträger oder nur temporär in einem Speicher eines Rechners abgespeichert werden. Der Begriff "Datei" kann auch jeweils eine Menge mit mehreren einzelnen Dateien bezeichnen, wobei diese Dateien bevorzugt durch rechnerauswertbare Verweise miteinander verbunden sind, beispielsweise durch Internet-Links. Falls die Darstellungsdatei mehrere Dateien umfasst, wird die Darstellung auf Basis von mehreren einzelnen Dateien generiert und ausgegeben.

Wie bereits dargelegt, gibt die erfindungsgemäße Erfassungs-Anordnung einem Benutzer die Fragen eines vorgegebenen Fragebogens aus, insbesondere visuell und / oder akustisch. Der Benutzer erhält die Möglichkeit, mittels der Eingabeeinheit auf jede ausgegebene Frage jeweils eine Antwort einzugeben. Die Erfassungs-Anordnung umfasst weiterhin mindestens einen Eingabe-Parameter-Sensor, der jeweils einen vorgegebenen Eingabe-Parameter zu messen vermag. Der oder jeder Eingabe-Parameter kennzeichnet das Verhalten eines Benutzers bei einem Vorgang, bei dem der Benutzer eine Antwort auf eine ausgegebene Frage der Fragen-Gruppe eingibt und die Eingabeeinheit die angegebene Antwort erfasst.

Die Erfassungs-Anordnung vermag also automatisch mindestens einen Eingabe-Parameter und optional weitere Parameter zu messen. Dadurch vermag die Erfassungs-Anordnung nicht nur die Antworten des Benutzers zu erfassen. Zusätzlich vermag die Erfassungs-Anordnung das Verhalten des Benutzers automatisch zu messen, und zwar das Verhalten des Benutzers, während der Benutzer eine Antwort auf eine Frage der Fragen-Gruppe eingibt. Zwar möglich, aber dank der Erfindung nicht erforderlich ist, dass eine weitere Person anwesend oder aus der Ferne zugeschaltet ist, während ein Benutzer mittels der Erfassungs-Anordnung die Fragen des Fragebogens beantwortet, wobei die weitere Person den Benutzer bei der Beantwortung der Fragen beobachtet. Falls eine weitere Person anwesend ist, so braucht diese weitere Person nicht notwendigerweise das Verhalten des Benutzers bei der Beantwortung der Fragen zu notieren oder sich zu merken.

Denkbar wäre, das Verhalten des Benutzers einmal zu messen, beispielsweise bevor der Benutzer mit der Beantwortung der Fragen beginnt oder nachdem er die Beantwortung abgeschlossen hat. Diese mögliche Ausgestaltung lässt sich mit der Erfindung kombinieren. Erfindungsgemäß wird hingegen für mindestens eine, bevorzugt für jede Frage der Fragen-Gruppe gemessen, welchen Wert der Eingabe-Parameter annimmt, während der Benutzer eine Antwort auf diese Frage eingibt - genauer: welchen Wert der Eingabe-Parameter in dem Beantwortungs-Zeitraum annimmt, in dem die Frage ausgegeben wird und die Erfassungs-Anordnung die Eingabe einer Antwort oder eine sonstige Reaktion auf die Frage zu erfassen vermag. In der Regel umfasst die Fragen-Gruppe mehrere Fragen, und der Eingabe-Parameter wird für jede Frage der Fragen-Gruppe erneut gemessen. Der jeweils gemessene Wert desselben Eingabe-Parameters kann daher von Frage zu Frage variieren. Dadurch lassen sich insbesondere bestimmte Fragen mit auffälligen Eingabe-Parameter-Werten identifizieren. Ein auffälliger Eingabe-Parameter-Wert kann aus einer bestimmten Reaktion des Benutzers auf diese Frage resultieren. Dank der Erfindung lässt sich das Verhalten eines Benutzers besser messen, als wenn ein Parameter während der Beantwortung insgesamt nur einmal gemessen werden würde.

Erfindungsgemäß wird der oder jeder Eingabe-Parameter für jede Frage der Fragen-Gruppe erneut gemessen. Der Eingabe-Parameter wird nicht notwendigerweise für jede Frage des Fragebogens gemessen, sondern nur für jede Frage der gegebenen Fragen-Gruppe. Die Fragen-Gruppe lässt sich so vorgeben, dass die Antworten auf die Fragen der Fragen-Gruppe einem Menschen einen Rückschluss auf den aktuellen Zustand des Benutzers ermöglichen. Möglich ist, dass jede Frage des Fragebogens auch zur Fragen-Gruppe gehört.

Eine einzelne Bewertung des Benutzerverhaltens kann von Frage zu Frage differieren. Die Bewertung hängt dank der Erfindung nicht von Erfahrungen und subjektiven Einschätzungen eines beobachtenden Menschen ab. Vielmehr hängt die Bewertung im Wesentlichen von den vorab aufgestellten und vorgegebenen Eingabe-Parameter und Auswerteregeln ab. Die Erfindung liefert daher ein nachvollziehbares und systematisches und relativ objektives Vorgehen, um das Verhalten des Benutzers bei der Beantwortung einer Frage und / oder bei der Beantwortung der Fragen der Fragen-Gruppe zu bewerten.

Eine Darstellung, die auf Basis der erfindungsgemäß erzeugten Darstellungsdatei ausgegeben wird, umfasst für jede Frage des Fragebogens die jeweilige Antwort oder eine Kennzeichnung, dass diese Frage nicht beantwortet wurde. Außerdem zeigt die Darstellung mindestens ein Auswertungsergebnis, wobei die Erfassungs-Auswerteeinheit dieses Auswertungsergebnis durch Anwendung der oder einer Auswerteregel erzielt und als Teil der Auswertungsdaten wenigstens temporär abgespeichert hat. Bevorzugt zeigt die Darstellung für jede Frage, auf die sich ein Auswertungsergebnis bezieht, dieses Auswertungsergebnis. Die Darstellungsdatei und somit die Darstellung sind also wesentlich detaillierter, als wenn die Darstellungsdatei lediglich eine akkumulierte Bewertung des Benutzerverhaltens oder eines Zustands des Benutzers umfassen würde. Ein Mensch, insbesondere ein Arzt, vermag diese Darstellung mithilfe eines Rechners oder sonstigen geeigneten Geräts wahrzunehmen und zu verwenden. Der Mensch kann insbesondere die erfindungsgemäß erzeugte Darstellung in einem nachfolgenden Gespräch mit derjenigen Person verwenden, auf die sich die Fragen beziehen.

In einer Anwendung beziehen die Fragen des Fragebogens sich auf einen Patienten. Bevorzugt beziehen sich einige Fragen auf den körperlichen Zustand und einige Fragen auf den mentalen Zustand des Patienten. Die Erfindung reduziert die Gefahr, dass ein Arzt ein medizinisches Risiko nicht erkennt, wobei dieses Risiko bei einer geplanten medizinischen Behandlung des Patienten auftritt oder auftreten kann. Dieser Patient hat zuvor mittels der Erfassungs-Anordnung Fragen des vorgegebenen Fragebogens beantwortet. Die Verantwortung für die medizinische Behandlung liegt selbstverständlich weiterhin beim Arzt. Die Erfindung bietet ihm ein Hilfsmittel.

Die Fragen des Fragebogens können sich auch auf eine Person beziehen, wobei diese Person daraufhin untersucht werden soll, ob die Person für eine bestimmte Aufgabe oder Arbeit oder Tätigkeit geeignet ist oder nicht.

Die erfindungsgemäße Erfassungs-Anordnung lässt sich in vielen Fällen auf einem bestehenden stationären Rechner (z.B. PC, Notebook, Laptop) oder tragbaren Rechner (z. B. Smartphone, Tablet) realisieren, indem ein entsprechendes Softwareprogramm auf diesem Rechner installiert wird. In der Regel umfasst ein solcher Rechner die benötigte Eingabeeinheit, die benötigte Ausgabeeinheit und die oder wenigstens einige der Geräte, mit deren Hilfe der oder jeder Eingabe-Parameter-Sensor realisiert wird. Oft lässt sich die Erfindung dadurch realisieren, dass auf einem bestehenden Gerät Software installiert wird, welche die Erfindung ausführt. In der Regel lassen sich außerdem bei Bedarf weitere benötigte Geräte leicht ergänzen.

Bevorzugt sind die Ausgabeeinheit, die Eingabeeinheit und der oder jeder Eingabe-Parameter-Sensor auf einem Rechner realisiert, besonders bevorzugt auf einem tragbaren Rechner. Der Benutzer, der die Fragen des Fragebogens beantwortet, verwendet diesen Rechner. Bevorzugt ist auch der Protokoll-Generierer auf diesem Rechner installiert. Oft reicht es aus, dass der Benutzer ein Softwareprogramm, welches die Erfindung realisiert, herunterlädt und auf seinem Rechner installiert.

In einer Ausgestaltung ist zusätzlich die Erfassungs-Auswerteeinheit auf diesem Rechner realisiert, besonders bevorzugt als ein Softwareprogramm, das von einem Prozessor des Rechners ausgeführt werden kann. In einer anderen Ausgestaltung ist die Erfassungs-Auswerteeinheit auf einem räumlich entfernten Rechner realisiert, beispielsweise "in der Cloud". Optional sind auch der Protokoll-Generierer und / oder der Darstellungs-Generierer räumlich entfernt realisiert. Dieser räumlich entfernte Rechner lässt sich als ein Zentralrechner bezeichnen. Zwischen dem Rechner mit der Ausgabeeinheit, der Eingabeeinheit und den Eingabe-Parameter-Sensoren und optional dem Protokoll-Generierer einerseits und dem Zentralrechner andererseits ist dauerhaft oder wenigstens zeitweise eine Datenverbindung hergestellt. Diese Datenverbindung kann kabelgebunden und / oder per Funkwellen hergestellt sein. Möglich ist, dass der Fragebogen über diese Datenverbindung vom Zentralrechner an den Benutzer-Rechner mit der Eingabeeinheit und der Ausgabeeinheit übermittelt wird. Umgekehrt übermittelt in einer Ausgestaltung der Benutzer-Rechner über diese Datenverbindung die erfassten Antworten des Benutzers sowie die gemessenen Eingabe-Parameter-Werte an den Zentralrechner. Falls auf diesem Benutzer-Rechner auch der Protokoll-Generierer installiert ist, so werden bevorzugt die Protokolldaten über diese Datenverbindung an den Zentralrechner übermittelt. Der Protokoll-Generierer kann auch auf dem Zentralrechner installiert sein. Erfindungsgemäß wird mindestens ein Eingabe-Parameter vorgegeben. Dem oder mindestens einem, bevorzugt jedem Eingabe-Parameter ist jeweils mindestens ein Eingabe-Parameter-Sensor zugeordnet. Der oder jeder Eingabe-Parameter-Sensor ist ein Bestandteil der erfindungsgemäßen Erfassungs-Anordnung und misst für jede Frage der Fragen-Gruppe jeweils, welchen Wert der zugeordnete Eingabe-Parameter bei der Eingabe einer Antwort auf die Frage angenommen hat. Nachfolgend werden bevorzugte Ausgestaltungen beschrieben, wobei einige diese Ausgestaltungen vorteilhafte Eingabe-Parameter und optional bevorzugte Eingabe-Parameter-Sensoren spezifizieren. Wie bereits erwähnt, charakterisiert jeder Eingabe-Parameter das Verhalten eines Benutzers, während der Benutzer eine Antwort auf eine Frage der Fragen-Gruppe eingibt.

Bevorzugt misst die Erfassungs-Anordnung für eine Frage der Fragen-Gruppe mindestens einen der folgenden Eingabe-Parameter mittels jeweils eines geeigneten Eingabe-Parameter-Sensors:
- Ist die Frage beantwortet worden oder nicht? Eine Frage ist beantwortet worden, wenn die Eingabeeinheit der Erfassungs-Anordnung das Ereignis erfasst hat, dass eine Antwort auf die Frage eingegeben und die Eingabe abgeschlossen worden ist.
- Wie lange ist der Beantwortungs-Zeitraum für die Frage? Der Beantwortungs-Zeitraum ist der Zeitraum zwischen dem Ereignis, dass die Ausgabeeinheit die Frage ausgegeben hat, und dem Ereignis, dass die Eingabe der Antwort abgeschlossen worden ist und die Eingabeeinheit den Abschluss detektiert hat.
- Wie viele Verzögerungen sind zwischen dem Beginn und dem Abschluss des Vorgangs, eine Antwort auf die Frage einzugeben, eingetreten? Diese Verzögerungen beziehen sich auf den Vorgang, die Antwort in die Eingabeeinheit einzugeben. Beispiele für Verzögerungen sind Pausen zwischen der jeweiligen Eingabe von zwei Zeichen in eine Tastatur oder Pausen zwischen der Eingabe von zwei Worten. Optional: Wie lang sind diese Verzögerungen insgesamt und / oder durchschnittlich?
- Wie stark schwankt die Geschwindigkeit, mit der die Antwort auf die Frage in die Eingabeeinheit eingegeben worden ist? Optional: Wie stark schwankt sie mehr als eine vorgegebene untere Schranke?
- Wie oft wurde eine bereits getätigte Eingabe bei der Beantwortung der Frage wieder geändert, beispielsweise indem eingegebene Zeichen wieder gelöscht worden sind oder eine Spracheingabe zurückgenommen wurde?

Die Fragen des Fragebogens und damit auch die Fragen der Fragen-Gruppe beziehen sich auf eine Person, beispielsweise auf einen Patienten, insbesondere auf einen Patienten, dessen aktueller Zustand vor einer medizinischen Behandlung festgestellt werden soll. In einer bevorzugten und nachfolgend beschriebenen Ausgestaltung wird überprüft, ob diese Person jede Frage der Fragen-Gruppe selbst beantwortet hat oder ob die Person bei der Beantwortung mindestens einer Frage der Fragen-Gruppe von einer anderen Person unterstützt wird und / oder mindestens eine Frage allein von einer anderen Person beantwortet worden ist.

Gemäß dieser Ausgestaltung umfasst die Erfassungs-Anordnung ein Bildaufnahmegerät, insbesondere eine Digitalkamera. Viele heute verfügbare tragbare Rechner, beispielsweise Smartphones und Tablets, besitzen ein solches Bildaufnahmegerät und lassen sich als Plattform der Erfassungs-Anordnung verwenden. Das Bildaufnahmegerät gehört zu dem oder zu mindestens einem Eingabe-Parameter-Sensor. Das Bildaufnahmegerät vermag mindestens ein Bild von einem Benutzer der Erfassungs-Anordnung zu erfassen, während der Benutzer eine Antwort auf eine Frage der Fragen-Gruppe eingibt. Die Erfassungs-Anordnung ist so ausgestaltet, dass das Bildaufnahmegerät für mindestens eine, bevorzugt für jede Frage der Fragen-Gruppe jeweils mindestens ein Bild von einem Benutzer erzeugt, während die Ausgabeeinheit die Frage ausgibt und / oder der Benutzer eine Antwort auf diese Frage eingibt, vermag also im Beantwortungs-Zeitraum mindestens ein Bild zu erzeugen. Möglich ist, dass das Bildaufnahmegerät für denselben Vorgang und dieselbe Frage mehrere Bilder erzeugt, beispielsweise ein erstes Bild, während die Frage ausgegeben wird, und mindestens ein zweites Bild, während eine Eingabe einer Antwort auf die Frage erfasst wird. Bevorzugt steuert ein signalverarbeitendes Steuergerät der Erfassungs-Anordnung das Bildaufnahmegerät entsprechend an.

Gemäß dieser Ausgestaltung ist ein Eingabe-Parameter der folgende Parameter: Wird bei der Ausgabe einer Frage und / oder bei der Eingabe einer Antwort auf diese Frage der eingebende Benutzer von einer weiteren Person unterstützt? Genauer gesagt: Zeigt das oder mindestens ein Bild, das bei der Ausgabe der Frage und / oder bei der Eingabe der Antwort erzeugt worden ist, zusätzlich zur eingebenden Person (zusätzlich zum Benutzer) eine weitere Person? Das oder jedes für diese Prüfung verwendete Bild ist vom Bildaufnahmegerät erzeugt worden.

Erfindungsgemäß erzeugt der Protokoll-Generierer Protokolldaten. In einer Realisierungsform der gerade beschriebenen Ausgestaltung umfassen diese Protokolldaten für mindestens eine Frage, bevorzugt für jede Frage der Fragen-Gruppe, das oder mindestens ein Bild. Dieses Bild hat das Bildaufnahmegerät erzeugt, während die Ausgabeeinheit die Frage ausgegeben hat und / oder die Eingabeeinheit die Eingabe eine Antwort auf diese Frage erfasst hat. Optional hat das Bildaufnahmegerät in diesem Zeitraum mehrere Bilder erzeugt. Erfindungsgemäß erzeugt der Darstellungs-Generierer eine Darstellungsdatei. Hierfür verwendet der Darstellungs-Generierer die Protokolldaten. In einer Ausgestaltung umfasst die Darstellungsdatei das oder mindestens ein, bevorzugt jedes Bild, welches das Bildaufnahmegerät so wie gerade beschrieben erzeugt hat.

In einer weiteren Fortbildung der Ausgestaltung mit dem Bildaufnahmegerät umfasst die Erfassungs-Auswerteeinheit eine signalverarbeitende Bildvergleichseinheit. Die Bildvergleichseinheit ist in einer Realisierungsform auf demjenigen Gerät (Rechner) installiert, welches auch die Eingabeeinheit, die Ausgabeeinheit und den oder jeden Eingabe-Parameter-Sensor umfasst. In einer anderen Realisierungsform ist die Bildvergleichseinheit räumlich von diesem Gerät entfernt angeordnet, insbesondere auf dem oben erwähnten Zentralrechner.

Diese Bildvergleichseinheit vermag zwei Bilder miteinander zu vergleichen und zu entscheiden, ob diese beiden Bilder jeweils das Gesicht einer Person zeigen, und wenn ja, ob die beiden Bilder dieselbe Person oder zwei verschiedene Personen zeigen. Diese Fortbildung der Ausgestaltung mit dem Bildaufnahmegerät lässt sich mit anderen Fortbildungen dieser Ausgestaltung kombinieren.

In einer ersten Fortbildung der Ausgestaltung mit der Bildvergleichseinheit ist ein Eingabe-Parameter der folgende Parameter: Werden alle Fragen der Fragen-Gruppe von derselben Person beantwortet? Oder sind die Fragen von insgesamt mindestens zwei verschiedenen Personen beantwortet worden?

Optional vermag die Bildvergleichseinheit festzustellen, ob eine weitere Person sich gemäß dem Bild der Erfassungs-Anordnung und / oder dem Benutzer der Erfassungs-Anordnung zuwendet oder aber nur zufällig ins Bild geraten ist.

In einer zweiten Fortbildung der Ausgestaltung mit der Bildvergleichseinheit ist der oder ein Eingabe-Parameter der folgende Parameter: Wurde jede Frage der Fragen-Gruppe von derjenigen Person beantwortet, auf die sich der Fragebogen bezieht? Oder wurde mindestens eine Frage der Fragen-Gruppe von einer anderen Person beantwortet? Diese beiden Fortbildungen lassen sich miteinander kombinieren.

Gemäß dieser zweiten Fortbildung ist in einer rechnerauswertbaren Form mindestens ein Referenzbild vorgegeben. Das oder jedes vorgegebene Referenzbild zeigt das Gesicht einer Person, auf die sich die Fragen des vorgegebenen Fragebogens beziehen. Die Person ist beispielsweise der Patient, dessen Zustand vor einer medizinischen Behandlung untersucht und bei der Operation berücksichtigt werden soll. Die Ausgabeeinheit gibt die Fragen des Fragebogens aus. Wie oben dargelegt, erzeugt das Bildaufnahmegerät für mindestens eine, bevorzugt für jede Frage der Fragen-Gruppe jeweils mindestens ein Bild eines Benutzers der Erfassungs-Anordnung, während die Frage ausgegeben oder eine Antwort auf die ausgegebene Frage erfasst wird. In einer Realisierungsform erzeugt das Bildaufnahmegerät ein erstes Bild, während die Frage ausgegeben wird, und ein zweites Bild, während eine Antwort auf die Frage eingegeben wird.

Für jede Frage vergleicht die Bildvergleichseinheit folgende Bilder miteinander:
- das oder mindestens ein, bevorzugt jedes Bild, das bei der Ausgabe der Frage oder bei der Eingabe der Antwort auf diese Frage erzeugt worden ist, mit
- dem oder mindestens einem, bevorzugt jedem vorgegebenen Referenzbild der Person, auf die sich die Fragen beziehen.

Die Erfassungs-Auswerteeinheit vermag jede Frage zu identifizieren, die von mehreren Personen und / oder von einer anderen Person beantwortet worden ist als von derjenigen Person, die in dem oder einem Referenzbild gezeigt worden ist. Die Erfassungs-Auswerteeinheit erzeugt die Auswertungsdaten wie folgt: In den Auswertungsdaten ist jede Frage der Fragen-Gruppe gekennzeichnet, die dergestalt identifiziert worden ist. Genauer gesagt: Jede Frage ist gekennzeichnet, die von mehreren Personen und / oder von einer anderen Person beantwortet worden ist als von derjenigen Person, auf die sich der Fragebogen bezieht und die im Referenzbild gezeigt wird.

Weiter oben wurde eine Ausgestaltung beschrieben, bei der das Bildaufnahmegerät für jede Frage der Fragen-Gruppe jeweils mindestens ein Bild derjenigen Person erzeugt, welche die Erfassungs-Anordnung benutzt, während die Frage ausgegeben wird, und / oder die Erfassungs-Anordnung benutzt, um eine Antwort auf die Frage einzugeben. In einer Realisierungsform umfassen die Protokolldaten das jeweilige Bild, welches das Bildaufnahmegerät angefertigt hat, während der Benutzer eine Antwort auf eine Frage der Fragen-Gruppe eingibt. Gemäß dieser Realisierungsform erzeugt der Darstellungs-Generierer die Darstellungsdatei wie folgt: In einer Darstellung, die auf Basis der erzeugten Darstellungsdatei ausgegeben wird, wird für mindestens eine Frage zusätzlich zur erfassten Antwort auf die Frage das oder mindestens ein Bild gezeigt, wobei das Bildaufnahmegerät dieses Bild angefertigt hat, während ein Benutzer der Erfassungs-Anordnung die Antwort auf die Frage eingibt und wobei das in der Darstellungsdatei enthaltene Bild diesen Benutzer zeigt.

Möglich ist, dass die Darstellung für mindestens eine, bevorzugt für jede Frage der Fragen-Gruppe jeweils ein Bild desjenigen Benutzers zeigt, der die Antwort auf diese Frage eingegeben hat. Möglich ist auch, dass die Darstellung dann und bevorzugt nur dann die Antwort auf eine Frage zusammen mit einem Bild eines Benutzers der Erfassungs-Anordnung zeigt, wenn dieser Benutzer die Antwort auf die Frage eingegeben hat und gemäß einem Vergleichsergebnis der Bildvergleichseinheit der Benutzer nicht diejenige Person ist, die in einem vorgegebenen Referenzbild gezeigt wird, also nicht diejenige Person, auf die sich die Fragen des Fragebogens beziehen.

Die Erfindung lässt sich mit folgendem Vorgehen kombinieren: Diejenige Person, auf die sich die Fragen des Fragebogens beziehen, authentifiziert sich vorab, beispielsweise mithilfe eines Passierworts (password) oder einer biometrischen Identifikation. Die Erfassungs-Anordnung gibt nur dann die Fragen aus und erfasst die Antworten auf die Fragen, wenn der Benutzer sich erfolgreich authentifiziert hat. Diese Ausgestaltung allein verhindert aber nicht Folgendes: Diejenige Person, auf die sich der Fragebogen bezieht, authentifiziert sich erfolgreich, und anschließend beantwortet eine andere Person die Fragen. Die Ausgestaltung mit dem Bildaufnahmegerät und der Bildvergleichseinheit erkennt hingegen in der Regel dieses Vorgehen automatisch.

Bei der gerade beschriebenen Ausgestaltung umfasst die Erfassungs-Anordnung ein Bildaufnahmegerät und optional eine Bildvergleichseinheit. Gemäß der nachfolgend beschriebenen Ausgestaltung umfasst die Erfassungs-Anordnung eine Audiosignal-Erfassungseinheit und eine Audiosignal-Auswerteeinheit. Die Audiosignal-Erfassungseinheit gehört zu dem oder mindestens einem Eingabe-Parameter-Sensor, optional zu mehreren Eingabe-Parameter-Sensoren. Die Audiosignal-Auswerteeinheit gehört zur Erfassungs-Auswerteeinheit. Die Audiosignal-Erfassungseinheit vermag ein Audiosignal zu erfassen. Dieses Audiosignal wurde in einer Umgebung der Erfassungs-Anordnung erzeugt. Die Audiosignal-Auswerteeinheit vermag folgende Schritte durchzuführen:
- Die Audiosignal-Auswerteeinheit entscheidet, ob das oder mindestens ein erfasstes Audiosignal mindestens eine Sprachnachricht umfasst. Eine Sprachnachricht stammt von einem Menschen und umfasst Worte einer natürlichen Sprache. Die Sprachnachricht kann eine Antwort auf eine ausgegebene Frage umfassen. Im Gegensatz dazu kann ein sonstiges Geräusch von einem Tier oder einem Gerät in der Umgebung der Erfassungs-Anordnung stammen oder vom Wetter oder einem sonstigen Naturereignis verursacht werden.

Möglich ist, dass die Audiosignal-Erfassungseinheit mehrere Sprachnachrichten erfasst hat. Diese Sprachnachrichten können alle von derselben Person oder von mindestens zwei verschiedenen Personen stammen. Falls das erfasste Audiosignal mindestens eine Sprachnachricht umfasst, so führt die Audiosignal-Auswerteeinheit folgenden Schritt durch: Die Audiosignal-Auswerteeinheit prüft, ob die Sprachnachrichten, die im Audiosignal enthalten sind, alle von demselben Menschen oder von mindestens zwei verschiedenen Menschen stammen. Optional zählt die Audiosignal-Auswerteeinheit, von wie vielen verschiedenen Menschen die Sprachnachrichten stammen.

Gemäß dieser Ausgestaltung ist der oder ein Eingabe-Parameter der folgende Parameter: Hat nur eine Person oder haben mehrere Personen in einem Beantwortungs-Zeitraum für eine Frage gesprochen? Der Beantwortungs-Zeitraum für eine Frage
- beginnt an dem Zeitpunkt, an dem die Ausgabeeinheit die Ausgabe der Frage begonnen hat, und
- endet an dem Zeitpunkt, an dem die Eingabeeinheit einen Abschluss des Vorgangs, eine Antwort auf die Frage einzugeben, erfasst hat, oder an dem Zeitpunkt, an dem feststeht, dass die Frage nicht beantwortet worden ist.

Nachfolgend werden weitere bevorzugte Eingabe-Parameter beschrieben. Diese Eingabe-Parameter setzen jeweils einen bestimmten Bestandteil der Erfassungs-Anordnung voraus.

In einer Ausgestaltung umfasst die Eingabeeinheit der Erfassungs-Anordnung eine Tastatur. Die Tastatur wird beispielsweise auf einem berührungssensitiven Bildschirm (Touchscreen) ausgegeben. Jede Taste der Tastatur wird dann in jeweils einem Bereich des Bildschirms gezeigt, und eine Berührung dieses Bereichs bewirkt, dass die Taste betätigt worden ist. Zur Tastatur können zusätzlich oder stattdessen eine physikalische Tastatur und / oder ein Touchpad gehören. Ein Benutzer kann mithilfe dieser Tastatur eine Antwort auf eine Frage eingeben ("eintippen"). Gemäß dieser Ausgestaltung wird mindestens einer der folgenden Eingabe-Parameter mit einem entsprechenden Eingabe-Parameter-Sensor gemessen, und zwar für mindestens eine, bevorzugt für jede Frage der Fragen-Gruppe:
- Wie schnell hat ein Benutzer der Erfassungs-Anordnung Eingaben auf der Tastatur der Eingabeeinheit vorgenommen, um die Frage zu beantworten? Beispielsweise wird die Anzahl von betätigten Tasten pro Zeiteinheit oder die Latenz zwischen zwei Tasten-Betätigungen gemessen.
- Wie zielsicher und / oder wie treffsicher hat der Benutzer die Tastatur benutzt, um eine Antwort auf die Frage einzugeben? Beispielsweise wird gemessen, wie oft der Benutzer gleichzeitig zwei benachbarte Tasten betätigt hat, was in der Regel ein Fehler ist.
- Wie oft hat ein Benutzer die Eingabe eines Zeichens mittels dieser Tastatur wieder zurückgenommen, das Zeichen also gelöscht? Wie viele Zeichen hat er zurückgenommen?
- Wie stark schwankt die Geschwindigkeit, mit der der Benutzer Tasten der Tastatur benutzt hat, um eine Antwort auf die Frage einzugeben?
- Falls die Eingabeeinheit ein Touchpad umfasst: Mit welchem Berührungsdruck hat der Benutzer im Touchpad eine Taste ausgewählt?

In einer anderen Ausgestaltung umfasst die Eingabeeinheit der Erfassungs-Anordnung eine Spracheingabeeinheit, insbesondere ein Mikrophon, und einen signalverarbeitenden Spracherkenner. Der Spracherkenner gehört zu dem oder mindestens einem Eingabe-Parameter-Sensor. In einer Ausgestaltung kann ein Benutzer vorab festlegen, in welcher natürlichen Sprache er die Antworten eingeben möchte. Der Spracherkenner vermag aus einem Audiosignal, das in die Spracheingabeeinheit eingegeben wurde, eine Textnachricht zu erzeugen - oder festzustellen, dass das Audiosignal nicht einen Text in einer gesprochenen Sprache in einer verständlichen und daher auswertbaren Form umfasst. Das Audiosignal ist beispielsweise eine Reaktion des Benutzers darauf, dass die Ausgabeeinheit eine Frage des Fragebogens ausgegeben hat, und die Erfassungs-Anordnung verwendet die Textnachricht als Antwort auf diese Frage.

Möglich ist, dass die Erfassungs-Anordnung sowohl eine Tastatur als auch eine Spracheingabeeinheit und einen Spracherkenner umfasst und ein Benutzer auswählen kann, ob er die Antwort auf eine Frage mithilfe der Tastatur oder mithilfe der Spracheingabeeinheit eingeben möchte. Die Entscheidung des Benutzers, wie er eine Frage beantworten möchte, kann bevorzugt von Frage zu Frage differieren.

Gemäß der Ausgestaltung mit der Spracheingabeeinheit ist der oder mindestens ein Eingabe-Parameter für eine Frage mindestens einer der folgenden Parameter:
- Mit welcher Lautstärke gibt ein Benutzer der Erfassungs-Anordnung eine Antwort auf eine Frage in die Spracheingabeeinheit ein? Wie laut spricht also der Benutzer bei der Eingabe der Antwort?
- Mit welcher Sprechgeschwindigkeit und / oder mit welchem Sprachfluss gibt der Benutzer eine Antwort auf eine Frage in die Spracheingabeeinheit ein?
- Wie viele Verzögerungen und oder Stockungen sind aufgetreten, während der Benutzer eine Antwort auf die Frage in die Spracheingabeeinheit eingegeben hat? Wie lange dauerten diese insgesamt?
- Wie oft hat ein Benutzer eine Spracheingabe wieder zurückgenommen oder korrigiert?
- Wie stark schwanken die Lautstärke und / oder die Sprechgeschwindigkeit, mit der ein Benutzer eine Antwort auf die Frage in die Spracheingabeeinheit eingegeben hat?
- Wie verständlich ist die Spracheingabe? Insbesondere: Mit welcher Sicherheit lässt sich aus der eingegebenen Sprachnachricht eindeutig eine bestimmte Textnachricht erzeugen?

Erfindungsgemäß gibt die Ausgabeeinheit der Erfassungs-Anordnung jede Frage des Fragebogens in mindestens einer von einem Menschen wahrnehmbaren Form aus. In einer Ausgestaltung umfasst die Ausgabeeinheit eine Anzeigefläche. Die Erfassungs-Anordnung vermag zu bewirken, dass die Anzeigefläche eine Frage des Fragebogens visuell auf der Anzeigefläche darstellt und dadurch ausgibt. Die Zeichengröße, mit der die Frage dargestellt wird, lässt sich verändern und dadurch einstellen. Beispielsweise lässt sich die Größe der Zeichen auf einer Bildschirmtastatur verändern. Diese Zeichengröße kann von Frage zu Frage differieren. Ein möglicher Eingabe-Parameter für eine Frage ist die Zeichengröße auf der Anzeigefläche, die bei der Ausgabe dieser Frage eingestellt worden ist.

In einer weiteren Ausgestaltung umfasst die Ausgabeeinheit eine Sprachausgabeeinheit, insbesondere einen Lautsprecher. Die beiden Ausgestaltungen, dass die Ausgabeeinheit eine Anzeigefläche umfasst und dass die Ausgabeeinheit eine Sprachausgabeeinheit umfasst, lassen sich miteinander kombinieren. Bevorzugt kann ein Benutzer festlegen, ob eine Frage des Fragebogens auf der Anzeigefläche visuell dargestellt oder von der Sprachausgabeeinheit akustisch ausgegeben wird oder auf beide Weisen ausgegeben werden soll. Die Festlegung des Benutzers kann von Frage zu Frage differieren.

In einer Realisierungsform lässt sich die Lautstärke einstellen und verändern, wobei die Sprachausgabeeinheit eine Frage mit dieser Lautstärke ausgibt. Auch die Lautstärke kann von Frage zu Frage differieren. Ein möglicher Eingabe-Parameter für eine Frage ist die Lautstärke, die bei der akustischen Ausgabe dieser Frage eingestellt worden ist. Ein weiterer möglicher Eingabe-Parameter beschreibt, wie oft diese Lautstärke verändert worden ist, während der Fragebogen beantwortet worden ist.

In einer Ausgestaltung umfasst der oder mindestens ein Eingabe-Parameter-Sensor mindestens einen Vitalparameter-Sensor. Der oder jeder Vitalparameter-Sensor vermag jeweils einen Vitalparameter eines Benutzers der Erfassungs-Anordnung zu messen. Bevorzugt werden mindestens zwei verschiedene Vitalparameter des Benutzers gemessen. Möglich ist, dass sich zwei verschiedene Vitalparameter-Sensoren auf denselben Vitalparameter beziehen. Der oder mindestens ein, bevorzugt jeder gemessene Vitalparameter ist jeweils ein Eingabe-Parameter. Bevorzugt misst der oder mindestens ein Vitalparameter-Sensor für die oder mindestens eine, bevorzugt für jede Frage der Fragen-Gruppe erneut den Vitalparameter, während der Benutzer eine Antwort auf die Frage eingibt. Möglich ist auch, dass die Messung des Vitalparameters sich über einen Mess-Zeitraum erstreckt und mehrere Beantwortungs-Zeiträume in diesem Mess-Zeitraum liegen.

Mindestens ein möglicher Eingabe-Parameter für eine Frage ist der oder ein Vitalparameter, der bei der Beantwortung dieser Frage gemessen wird. Möglich ist, dass der Vitalparameter in einem Mess-Zeitraum gemessen wird und der Benutzer in diesem Mess-Zeitraum mehrere Fragen beantwortet. In diesem Falle bezieht sich der gemessene Wert des Vitalparameters auf jede Frage, die der Benutzer im Mess-Zeitraum beantwortet hat. Diese Fragen haben also alle denselben Vitalparameter-Wert.

Der oder mindestens ein Vitalparameter ist insbesondere die Herzschlagfrequenz oder der Blutdruck des Benutzers. Besonders bevorzugt werden diese beiden Vitalparameter gemessen. Häufig lässt sich anhand dieser beiden Vitalparameter entscheiden, ob ein Benutzer aktuell in Panik geraten ist oder nicht. Häufig vermag ein in Panik geratener Benutzer eine Frage nicht richtig zu verstehen und daher auch nicht richtig zu beantworten. Ein weitere Vitalparameter, welche von jeweils einem Eingabe-Parameter-Sensor gemessen werden, kann mindestens einer die folgenden sein:
- die Sättigung des Bluts mit Sauerstoff,
- der Blutzuckerspiegel,
- die Körpertemperatur oder
- die Atemfrequenz des Benutzers.

Die Ausgestaltung mit dem Vitalparameter-Sensor lässt sich kombinieren mit der Ausgestaltung, bei der die Erfassungs-Anordnung ein Bildaufnahmegerät umfasst. Dank dieser Kombination lässt sich feststellen, auf wen sich der gemessene Vitalparameter bezieht.

Bevorzugt umfasst die Erfassungs-Anordnung ein tragbares Gerät, insbesondere ein Smartphone oder ein Tablet. Dieses tragbare Gerät lässt sich von einem Menschen in einer Hand halten. Ein Benutzer kann dieses Gerät in einer Hand halten und in das gehaltene Gerät Antworten auf die Fragen des Fragebogens eingeben. Dieses tragbare Gerät umfasst mindestens die Ausgabeeinheit, die Eingabeeinheit und den oder mindestens einen, bevorzugt jeden Eingabe-Parameter-Sensor. Optional umfasst dieses tragbare Gerät auch den Protokoll-Generierer, die Erfassungs-Auswerteeinheit und / oder den Darstellungs-Generierer.

In einer Ausgestaltung umfasst das tragbare Gerät zusätzlich einen Bewegungssensor. Diese Bewegungssensor ist ein Eingabe-Parameter-Sensor. Der Bewegungssensor vermag die Bewegung des tragbaren Geräts im Raum zu messen und liefert bevorzugt eine Trajektorie des tragbaren Geräts in einem vorgegebenen dreidimensionalen Koordinatensystem. Beispielsweise umfasst der Bewegungssensor einen Beschleunigungssensor, wobei der Beschleunigungssensor seine eigenen drei linearen Beschleunigungen und seine eigenen drei Winkelbeschleunigungen im Raum zu messen vermag. Ein gemessener Eingabe-Parameter ist der folgende Parameter: die Bewegung im Raum des tragbaren Geräts in dem Zeitraum, in dem die Eingabeeinheit eine Antwort des Benutzers auf eine Frage der Fragen-Gruppe erfasst hat. Diese Bewegung hat der Bewegungssensor gemessen. In einer Realisierungsform ist oder umfasst der Wert des Eingabe-Parameters ein Segment der oben genannten Trajektorie, nämlich das Segment, welches das tragbare Gerät im Beantwortungs-Zeitraum zurückgelegt hat.

In einer Realisierungsform vermögen eine Auswerteeinheit des Bewegungssensors oder die Erfassungs-Auswerteeinheit das oder jedes Segment der gemessenen Bewegung auszuwerten, wobei jedes Segment die Bewegung des tragbaren Geräts bei der Beantwortung jeweils einer Frage der Fragen-Gruppe beschreibt. Durch die Auswertung lässt sich insbesondere feststellen, ob der Benutzer, der das tragbare Gerät in einer Hand hält, bei der Beantwortung der Frage zittert oder das Gerät im Raum bewegt oder aber fallen lässt. Ein Zittern gibt einen Hinweis auf den aktuellen Zustand des Benutzers. Eine erhebliche Bewegung im Raum des tragbaren Geräts kann ein Indiz dafür sein, dass der Benutzer ein Bildaufnahmegerät des tragbaren Geräts auf eine andere Person richtet, während der Benutzer eine Antwort auf die Frage eingibt. Beispielsweise zeigt das Bildaufnahmegerät den Patienten, auf den sich der Fragebogen bezieht, während ein Benutzer, der nicht der Patient ist, die Frage beantwortet. Möglich ist auch, dass der Benutzer sich an eine andere Person wendet und dabei das tragbare Gerät im Raum bewegt.

Bevorzugt umfassen die Protokolldaten und / oder die Auswertungsdaten für mindestens eine Frage der Fragen-Gruppe eine automatisch hergeleitete Beschreibung und / oder Kennzeichnung und / oder Bewertung desjenigen Segments der Bewegungsbahn, welches das tragbare Gerät in dem Beantwortungs-Zeitraum für eine Frage zurückgelegt hat. Zur Bewertung gehört beispielsweise eine maximale Beschleunigung auf dem Segment oder die Länge des Segments.

Nachfolgend wird ein weiterer möglicher Eingabe-Parameter beschrieben. Gemäß dieser Ausgestaltung lässt sich die Erfassungs-Anordnung wahlweise in einem Eingabemodus oder in einem Rückfragemodus betreiben. Standardmäßig befindet die Eingabeeinheit sich im Eingabemodus. In einer Realisierungsform kann ein Benutzer die Erfassungs-Anordnung in den Rückfragemodus umschalten. Dies kann der Benutzer für jede Frage der Fragen-Gruppe tun. In einer anderen Ausgestaltung erkennt die Erfassungs-Anordnung automatisch, ob eine Eingabe eines Benutzers eine Antwort oder eine Rückfrage ist. Bevorzugt schaltet die Erfassungs-Anordnung sich wieder in den Eingabemodus um, wenn sie eine Rückfrage des Benutzers erfasst und das Ende der Eingabe der Rückfrage festgestellt hat.

Die Erfassungs-Anordnung ist dazu ausgestaltet, eine Eingabe eines Benutzers in die Eingabeeinheit wie folgt zu verwenden:
- Dann, wenn die Ausgabeeinheit eine Frage der Fragen-Gruppe ausgibt und die Erfassungs-Anordnung im Eingabemodus ist, verwendet die Erfassungs-Anordnung die Eingabe des Benutzers als eine Antwort auf die gerade ausgegebene Frage.
- Dann, wenn die Ausgabeeinheit die Frage ausgibt und die Erfassungs-Anordnung im Rückfragemodus ist, verwendet die Erfassungs-Anordnung die Eingabe des Benutzers als eine Rückfrage zu der gerade ausgegebenen Frage.

In einer Realisierungsform kann der Benutzer die Erfassungs-Anordnung insbesondere dadurch in den Rückfragemodus umschalten, dass der Benutzer eine bestimmte Taste betätigt und / oder eine bestimmte Spracheingabe vornimmt, beispielsweise ein bestimmtes Schlüsselwort eingibt. In einer anderen Realisierungsform umfasst die Erfassungs-Auswerteeinheit einen Textanalysierer. Der Textanalysierer vermag zu erkennen, ob ein eingegebener Text eine Antwort auf eine Frage oder eine Rückfrage zu einer Frage ist. Falls der Textanalysierer entschieden hat, dass eine Eingabe eine Rückfrage ist, wird die Erfassungs-Anordnung automatisch in den Rückfragemodus umgeschaltet.

In einer Ausgestaltung kann der Benutzer eine Rückfrage per Freitext eingeben. In einen anderen Ausgestaltung bewirkt der Vorgang, dass die Erfassungs-Anordnung in den Rückfragemodus umgeschaltet wird, folgendes: Die Ausgabeeinheit gibt mindestens einen vorgegebenen erläuternden Text aus. Optional gibt die Ausgabeeinheit zunächst ein Auswahlmenü aus, wobei der Benutzer eine Alternative im Auswahlmenü auswählen kann und dadurch spezifizieren kann, worauf sich seine Rückfrage bezieht (FAQs). Anschließend gibt die Ausgabeeinheit abhängig von der ausgewählten Alternative einen erläuternden Text aus. Bevorzugt schaltet sich die Eingabeeinheit danach wieder in den Eingabemodus um.

Mehrere mögliche Eingabe-Parameter beziehen sich auf den Rückfragemodus, insbesondere die folgenden Eingabe-Parameter:
- Wie viele Rückfragen hat der Benutzer insgesamt zu einer bestimmten Frage der Fragen-Gruppe gestellt?
- Wie viele dieser Rückfragen betreffen inhaltlich den gleichen Sachverhalt?
- Wie viele dieser Rückfragen haben nichts mit der Frage zu tun? Ein Beispiel für eine solche meist nicht sinnvolle Rückfrage: Gefragt wird, welche Medikamente ein Patient aktuell zu sich nimmt. Der Patient stellt die Rückfrage, ob es heute regnen wird oder nicht.

In einer Realisierungsform umfasst die Erfassungs-Auswerteeinheit einen signalverarbeitenden Textanalysierer. Dieser Textanalysierer vermag in Verbindung mit der gerade beschriebenen Ausgestaltung automatisch zu entscheiden, ob zwei Rückfragen denselben Sachverhalt betreffen und ob eine Rückfrage eine sinnvolle Rückfrage zu einer Frage des Fragebogens ist oder nicht.

Gerade wurde eine Ausgestaltung beschrieben, bei der die Erfassungs-Anordnung wahlweise in einem Eingabemodus oder in einem Rückfragemodus betrieben werden kann. Im Rückfragemodus kann der Benutzer eine Rückfrage zu einer Frage der Fragen-Gruppe stellen. Möglich ist auch, dass der Benutzer eine Rückfrage stellen kann, bevor er mit der Beantwortung der Fragen beginnt oder nachdem er die Beantwortung der Fragen abgeschlossen hat. Eine solche Rückfrage kann sich beispielsweise auf eine geplante medizinische Behandlung beziehen, und der Benutzer fragt durch die Rückfrage nach Details der medizinischen Behandlung. Eine solche Rückfrage bezieht sich nicht notwendigerweise auf eine Frage der Fragen-Gruppe. Der gerade beschriebene optionale Textanalysierer generiert Antworten auf diese allgemeinen Rückfragen. Bevorzugt prüft der Textanalysierer außerdem, ob mindestens zwei inhaltlich übereinstimmende Rückfragen oder Rückfragen, die nichts mit den Fragen des Fragebogens zu tun haben, gestellt worden sind.

Erfindungsgemäß misst der oder mindestens ein, bevorzugt jeder, Eingabe-Parameter-Sensor für jede Frage der Fragen-Gruppe, welchen Wert der zugeordnete Eingabe-Parameter annimmt, während der Benutzer eine Antwort auf diese Frage eingibt. Der Protokoll-Generierer erzeugt die Protokolldaten. Diese Protokolldaten umfassen den oder mindestens einen, bevorzugt jeden gemessenen Eingabe-Parameter-Wert. Der Darstellungs-Generierer erzeugt eine Darstellungsdatei und verwendet hierfür die Protokolldaten.

In einer Ausgestaltung erzeugt der Darstellungs-Generierer die Darstellungsdatei wie folgt: Für mindestens eine, bevorzugt für jede Frage der Fragen-Gruppe und für mindestens einen Eingabe-Parameter umfasst die Darstellungsdatei den gemessenen Wert, den der Eingabe-Parameter bei der Beantwortung dieser Frage angenommen hat. Eine Darstellung, die auf Basis dieser Darstellungsdatei ausgegeben wird, zeigt zusammen mit einer Frage und der Antwort auf diese Frage den Eingabe-Parameter-Wert, der bei der Eingabe der Antwort gemessen worden ist.

In einer Ausgestaltung ist für den Eingabe-Parameter ein Soll-Wertebereich vorgegeben. Bei mehreren Eingabe-Parametern ist für mindestens einen, bevorzugt für jeden Eingabe-Parameter jeweils ein Soll-Wertebereich vorgegeben. Dieser Soll-Wertebereich kann nicht nur von Eingabe-Parameter zu Eingabe-Parameter, sondern auch von Frage zu Frage differieren. Beispielsweise ist ein Soll-Beantwortungs-Zeitraum vorgegeben, wobei dieser Soll-Beantwortungs-Zeitraum von Frage zu Frage differieren kann. Oder vorgegeben ist, auf welche Person sich die Fragen des Fragebogens beziehen, wobei mindestens ein Referenzbild dieser Person vorgegeben ist. Der Soll-Wertebereich ist das folgende Ergebnis, welches die oben beschriebene Bildvergleichseinheit erzielt hat: Ein Bild, das erzeugt worden ist, während die Antwort auf eine Frage der Fragen-Gruppe erfasst wird, zeigt diese Person. Oder für einen Vitalparameter ist ein Soll-Wertebereich vorgegeben. Der Soll-Wertebereich für den Vitalparameter kann von einer vorgegebenen Eigenschaft der Person abhängen, beispielsweise von seinem Alter.

Falls ein gemessener Wert für einen Eingabe-Parameter außerhalb des vorgegebenen Soll-Wertebereichs liegt, so kann dies ein Indiz dafür sein, dass die Frage unzutreffend beantwortet worden ist oder dass der Benutzer bei der Beantwortung der Frage nur geraten hat oder in Panik geraten ist oder Angstzustände aufwies. Die Erfassungs-Auswerteeinheit prüft für jede Frage der Fragen-Gruppe und für jeden Eingabe-Parameter, für den ein Soll-Wertebereich vorgegeben ist, folgendes: Liegt der Wert, den der zugeordnete Eingabe-Parameter-Sensor für diesen Eingabe-Parameter bei der Beantwortung dieser Frage gemessen hat, im Soll-Wertebereich oder außerhalb des Soll-Wertebereichs? Die Auswertungsdaten umfassen bevorzugt mindestens für jede Frage, bei der ein Eingabe-Parameter-Wert außerhalb des jeweils vorgegebenen Soll-Wertebereichs gemessen worden ist, eine Kennzeichnung dieser Frage und bevorzugt eine Kennzeichnung des gemessenen Eingabe-Parameter-Werts, der außerhalb des Soll-Wertebereichs liegt, oder wenigstens eine Aussage, dass der Wert außerhalb des Soll-Wertebereichs liegt.

Mindestens dann, wenn für eine Frage ein Eingabe-Parameter-Wert außerhalb des Soll-Wertebereichs liegt, führt der Darstellungs-Generierer folgenden Schritt durch: Der Darstellungs-Generierer erzeugt die Darstellungsdatei dergestalt, dass die Darstellungsdatei für diese Frage zusätzlich zur erfassten Antwort
- eine Kennzeichnung des Eingabe-Parameters und
- eine Kennzeichnung des Eingabe-Parameter-Werts, der bei der Beantwortung dieser Frage gemessen worden ist,
umfasst. Dieser Eingabe-Parameter-Wert liegt außerhalb des vorgegebenen Soll-Wertebereichs. Eine Darstellung, die auf Basis dieser Darstellungsdatei erzeugt worden ist, zeigt bevorzugt diese Kennzeichnung des Eingabe-Parameter und diese Kennzeichnung des Eingabe-Parameter-Werts. Diese Ausgestaltung erleichtert es einer Person, welche die Darstellung betrachtet, solche Fragen aufzufinden, bei denen ein Eingabe-Parameter-Wert außerhalb des jeweiligen Soll-Wertebereichs liegt. Ein Wert außerhalb des Soll-Wertebereichs kann ein Indiz für eine unzutreffend beantwortete Frage sein.

Die Eingabe-Parameter beziehen sich auf das Verhalten eines Benutzers in einem Beantwortungs-Zeitraum, in dem eine Frage der Fragen-Gruppe beantwortet wurde. Die Eingabe-Parameter beziehen sich in der Regel nicht auf den Inhalt einer erfassten Antwort auf eine Frage. In einer Ausgestaltung vermag die Erfassungs-Auswerteeinheit zusätzlich den Inhalt von Antworten zu bewerten. Diesbezügliche Ausgestaltungen werden nachfolgend beschrieben.

Möglich ist, dass die beiden erfassten Antworten auf zwei Fragen des Fragebogens einander widersprechen oder auf andere Weise inkonsistent sind. Dies bedeutet: In aller Regel treffen für ein und dieselbe Person nicht beide Antworten zu. Ein Beispiel: Ein Patient gibt an, aktuell mehrere verschreibungspflichtige Medikamente zu sich zu nehmen, und gibt andererseits an, in den letzten fünf Jahren niemals bei einem Arzt gewesen zu sein. Oder ein Patient gibt an, 2 m groß zu sein und 30 kg zu wiegen.

In einer Ausgestaltung umfasst die Erfassungs-Auswerteeinheit eine signalverarbeitende Inkonsistenz-Auswerteeinheit. Die Inkonsistenz-Auswerteeinheit vermag jedes Inkonsistenz-Paar in den Antworten zu detektieren. Ein Inkonsistenz-Paar besteht aus mindestens zwei Antworten, die gemäß der oder mindestens einer Inkonsistenz-Regel nicht zueinander konsistent sind oder eine ausreichend geringe Bewertung dafür aufweisen, miteinander konsistent zu sein.

In einer Realisierungsform ist der Inkonsistenz-Auswerteeinheit in einer rechnerausführbaren Form mindestens eine Inkonsistenz-Regel vorgegeben. Bevorzugt sind mehrere Inkonsistenz-Regeln vorgegeben. Jede Inkonsistenz-Regel umfasst mindestens zwei mögliche Antworten auf zwei verschiedene Fragen, wobei die beiden Antworten nicht miteinander konsistent sind, also bei korrekter Beantwortung nicht gleichzeitig für dieselbe Person gelten können. Ein Inkonsistenz-Paar besteht aus mindestens zwei Antworten, die gemäß der oder mindestens einer vorgegebenen Inkonsistenz-Regel nicht zueinander konsistent sind oder eine ausreichend geringe Bewertung dafür aufweisen, miteinander konsistent zu sein.

Möglich ist, dass eine Inkonsistenz-Regel zwei verschiedene Fragen und zwei Wertebereiche spezifiziert, nämlich jeweils einen Wertebereich für jede Frage. Für jede dieser mindestens zwei Fragen gilt: Mindestens eine mögliche Antwort auf die Frage fällt in den jeweiligen Wertebereich. In einer ersten Alternative sind zwei erfasste Antworten auf diese beiden Fragen miteinander inkonsistent, wenn die beiden Antworten in die beiden Wertebereiche fallen. In einer anderen Alternative sind die beiden Antworten inkonsistent, wenn mindestens eine Antwort außerhalb des jeweiligen Wertebereichs liegt.

Die Inkonsistenz-Auswerteeinheit vermag die oder mindestens eine, bevorzugt jede, vorgegebene Inkonsistenz-Regel auf die erfassten Antworten anzuwenden, wobei diese Antworten von mindestens einem Benutzer der Erfassungs-Anordnung stammen und sich auf die Fragen des Fragebogens und damit auf dieselbe Person beziehen. Die Inkonsistenz-Auswerteeinheit vermag durch die Anwendung der Inkonsistenz-Regeln jedes Inkonsistenz-Paar zu detektieren. Natürlich ist es möglich, dass genau ein oder gar kein Inkonsistenz-Paar detektiert wird.

Nicht immer lässt sich eindeutig entscheiden, ob zwei Antworten miteinander konsistent oder zueinander inkonsistent sind. In einer Fortbildung sind mehrere mögliche Bewertungen vorgegeben. Die mindestens zwei möglichen und in der Regel zueinander inkonsistenten Antworten einer Inkonsistenz-Regel bilden eine Prämisse, und als Konklusion nennt die Inkonsistenz-Regel eine Bewertung dafür, wie wahrscheinlich es ist, dass diese Antworten gleichzeitig auf ein und dieselbe Person zutreffen.

Gemäß der gerade beschriebenen Fortbildung vermag die Inkonsistenz-Auswerteeinheit zusätzlich für jedes detektierte Inkonsistenz-Paar eine Bewertung dieser Inkonsistenz zu ermitteln. Die Auswertungsdaten umfassen jeweils eine Kennung jedes detektierten Inkonsistenz-Paars und bevorzugt eine Bewertung dieser Inkonsistenz.

Nachfolgend wird eine zusätzliche oder alternative Realisierungsform eines Inkonsistenz-Paars beschrieben. Gemäß dieser Realisierungsform bezieht sich der Fragebogen auf eine Person, und Informationen über diese Person sind einer rechnerauswertbaren Form vorgegeben. Zu diesen Informationen gehören beispielsweise das Geburtsdatum oder die aktuelle Körpergröße oder das aktuelle Körpergewicht des Patienten. Mindestens eine Frage der Fragen-Gruppe ist eine Kontrollfrage und bezieht sich auf eine solche vorgegebene Information. Ein Inkonsistenz-Paar liegt vor, wenn die erfasste Antwort des Benutzers auf eine Kontrollfrage um mehr als eine vorgegebene Toleranz von der entsprechenden vorgegebenen Information abweicht. Ein Beispiel: Die erfasste Körpergröße oder das erfasste Körpergewicht weicht um mehr als eine Toleranz von der vorgegebenen Körpergröße bzw. dem vorgegebenen Körpergewicht ab.

Gerade wurde eine Ausgestaltung beschrieben, bei der die Inkonsistenz-Auswerteeinheit jedes Inkonsistenz-Paar zu detektieren vermag. In einer Ausführungsform wendet die Inkonsistenz-Auswerteeinheit hierfür Inkonsistenz-Regeln an. In einer anderen Ausgestaltung ist eine Stichprobe mit möglichen Antworten auf die Fragen des Fragebogens vorgegeben. Die Antworten wurden vorab eingegeben und von einer Person daraufhin bewertet, welche Antworten miteinander konsistent sind und welche Antworten nicht. Auf diese Stichprobe wird ein lernendes Verfahren angewendet. Das Ergebnis dieses lernenden Verfahrens wird angewendet, um die Antworten zu bewerten, die der Benutzer eingegeben hat.

Der Darstellungs-Generierer vermag die Darstellungsdatei wie folgt zu erzeugen: Eine Darstellung, die auf Basis der Darstellungsdatei ausgegeben wird, umfasst jeweils eine Kennzeichnung jedes detektierten Inkonsistenz-Paars, optional zusammen mit einer Kennzeichnung der Bewertung dieses Inkonsistenz-Paars. Bei mehreren erzeugten oder erzeugbaren Darstellungsdateien umfasst mindestens eine Darstellungsdatei die jeweilige Kennzeichnung jedes detektierten Inkonsistenz-Paars.

In einer Ausgestaltung ist in einer rechnerauswertbaren Form mindestens ein Test vorgegeben. Der oder jeder Test beschreibt jeweils eine Handlung. Ein Benutzer der Erfassungs-Anordnung soll diese Handlung mithilfe der Eingabeeinheit vornehmen. Beispielsweise soll der Benutzer mithilfe eines Bildschirmstifts einen durch den Test vorgegebenen Gegenstand auf einem berührungssensitiven Bildschirm der Eingabeeinheit zeichnen. Der Test spezifiziert, was der Benutzer zeichnen soll. Oder der Benutzer soll gesprochene Sprache erkennen und wiedergeben oder mindestens ein Wort sprechen oder schreiben, welches die Ausgabeeinheit visuell ausgibt. Ein Beispiel für einen solchen Test ist der sogenannte Uhrentest, bei dem ein Benutzer gebeten wird, eine Uhr zu zeichnen oder in eine vorgegebene Uhr eine bestimmte Uhrzeit einzutragen. Der Uhrentest und weitere psychometrische Tests lassen sich dafür verwenden, um zu prüfen, ob ein Patient unter einer beginnenden Demenz leidet. Eine solche Demenz ist insbesondere bei einer medizinischen Behandlung des Patienten zu berücksichtigen.

Gemäß dieser Ausgestaltung vermag die Erfassungs-Auswerteeinheit automatisch folgende Schritte zu bewirken:
- Die Ausgabeeinheit gibt den Test aus.
- Die Eingabeeinheit erfasst mindestens eine Reaktion des Benutzers auf die Ausgabe des Tests.
- Die Protokolldaten und bevorzugt auch die oder jede Darstellungsdatei umfassen die erfasste Reaktion des Benutzers, im Falle von mehreren Reaktionen mindestens eine und bevorzugt jede erfasste Reaktion.
- Eine Darstellung, die auf Basis der oder einer Darstellungsdatei ausgegeben wird, umfasst eine Darstellung der erfassten Reaktion des Benutzers auf die Ausgabe des Tests. Beispielsweise zeigt die Darstellung, was der Benutzer beim Uhrentest gezeichnet hat.

Falls mehrere solche Tests vorgegeben sind, so wählt die Erfassungs-Auswerteeinheit mindestens einen Test aus und bewirkt dessen Durchführung.

Bevorzugt hängt es von den bislang erfassten Antworten des Benutzers ab, ob der Benutzer gebeten wird, den Test durchzuführen, oder nicht gebeten wird. Bevorzugt führt die Erfassungs-Auswerteeinheit die gerade beschriebenen Schritte betreffend den Test dann durch, wenn mindestens eine der folgenden Bedingungen erfüllt ist:
- Für eine Frage der Fragen-Gruppe sind ein Eingabe-Parameter und ein Soll-Wertebereich vorgegeben. Der Wert dieses Eingabe-Parameters wird gemessen, und festgestellt wird, dass er außerhalb des vorgegebenen Soll-Wertebereichs liegt.
- Mindestens eine Frage der Fragen-Gruppe wurde von einer anderen Person oder mithilfe einer anderen Person beantwortet.
- N Fragen der Fragen-Gruppe wurden nicht beantwortet, wobei N >= 1 eine vorgegebene Anzahl ist.
- Mindestens N-mal wurde zu einer ausgegebenen Frage eine Rückfrage gestellt, die nichts mit der Frage zu tun hat, wobei N >= 1 eine vorgegebene Anzahl ist.
- Zu derselben Frage wurden N inhaltsgleiche Rückfragen gestellt, wobei N >= 1 eine vorgegebene Anzahl ist.
- Mindestens N Inkonsistenz-Paare wurden detektiert, wobei N >= 1 eine vorgegebene Anzahl ist.

Die gerade beschriebene Ausgestaltung mit dem Test wird bevorzugt kombiniert mit der weiter oben beschriebenen Ausgestaltung, bei der ein Referenzbild einer Person, auf die sich der Fragebogen bezieht, vorgegeben wird und eine Bildvergleichseinheit ein Bild eines Benutzers der Erfassungs-Anordnung mit dem Referenzbild vergleicht. Dadurch wird festgestellt, ob tatsächlich die Person, auf die sich der Fragebogen bezieht, den Test durchführte oder jemand anders. Ein Hintergrund: Insbesondere bei der Anwendung, bei der die Fragen des Fragebogens sich auf einen Patienten beziehen, der medizinisch zu behandeln ist, soll natürlich der Patient selbst den Test durchführen und nicht jemand anders.

Erfindungsgemäß umfassen die Protokolldaten für mindestens eine, bevorzugt für jede Frage der Fragen-Gruppe und für mindestens einen, bevorzugt jeden Eingabe-Parameter den Wert, den der jeweilige Eingabe-Parameter-Sensor für diese Frage und für diesen Eingabe-Parameter gemessen hat. In einer Ausgestaltung leitet die Erfassungs-Auswerteeinheit aus den gemessenen Eingabe-Parameter-Werten mindestens eine Gesamtbewertung des Verhaltens des Benutzers her, während der Benutzer mittels der Erfassungs-Anordnung die Fragen des Fragebogens beantwortet, wobei dieses Verhalten mittels der Eingabe-Parameter-Sensoren bei der Beantwortung des Fragebogens gemessen worden ist. Bevorzugt bezieht die Gesamtbewertung sich auf die Person, auf die sich die Fragen beziehen.

In einer Realisierungsform dieser Ausgestaltung wird für mindestens einen Eingabe-Parameter eine Einzelbewertung hergeleitet, optional für mehrere Eingabe-Parameter jeweils eine Einzelbewertung. Aus den Einzelbewertungen für die Eingabe-Parameter wird eine Gesamtbewertung des Benutzers hergeleitet. Die Gesamtbewertung beschreibt das Verhalten des Benutzers bei der Eingabe der Antworten auf die Fragen der Fragen-Gruppe.

Bevorzugt ist für mindestens einen Eingabe-Parameter eine Aggregierungsvorschrift vorgegeben. Die oder jede Aggregierungsvorschrift bezieht sich auf jeweils einen Eingabe-Parameter. Für verschiedene Eingabe-Parameter können unterschiedliche Aggregierungsvorschriften vorgegeben sein. Die Aggregierungsvorschrift für einen Eingabe-Parameter fasst mehrere Werte dieses Eingabe-Parameters zu einer Einzelbewertung für den Eingabe-Parameter zusammen. Diese Werte sind für unterschiedliche Fragen der Fragen-Gruppe gemessen worden. Bevorzugt ist für jede Frage der Fragen-Gruppe und für mindestens einen Eingabe-Parameter, der in einer Aggregierungsvorschrift auftritt, ein Gewichtsfaktor vorgegeben. Die Aggregierungsvorschrift spezifiziert ein gewichtetes Mittel über die gemessenen Eingabe-Parameter-Werte. Falls der Eingabe-Parameter numerische Werte liefert, so kann die Aggregierungsvorschrift auch das Minimum oder das Maximum liefern. Die Einzelbewertungen für verschiedene Eingabe-Parameter werden zu einer Gesamtbewertung zusammengefasst.

Bevorzugt beziehen die Fragen des Fragebogens sich auf eine Person. In einer Ausgestaltung sind Informationen über diese Person vorgegeben, beispielsweise das Geburtsdatum oder der Name. Zur Kontrolle werden diese Informationen trotzdem erfragt, d. h. mindestens eine Frage der Fragen-Gruppe bezieht sich auf eine solche bereits bekannte Information. In einer Ausgestaltung vergleicht die Erfassungs-Auswerteeinheit die Antwort eines Benutzers auf eine solche Frage mit der vorgegebenen Information. Beispielsweise vergleicht die Erfassungs-Auswerteeinheit das vorgegebene Geburtsdatum mit dem eingegebenen Geburtsdatum. Abweichungen zwischen einer vorgegebenen Information und einer Antwort auf eine entsprechende Frage fließen ebenfalls in die Gesamtbewertung ein.

Bevorzugt beziehen die Fragen des Fragebogens sich auf eine Person, beispielsweise auf einen Patienten, der sich einer medizinischen Behandlung unterziehen soll. Mit dieser Person soll ein Gespräch geführt werden, und zwar, nachdem der Fragebogen für diese Person ausgefüllt worden ist. In einer Ausgestaltung vermag die Erfassungs-Auswerteeinheit eine Vorhersage zu berechnen, wie lange dieses Gespräch voraussichtlich dauern wird. Diese Vorhersage erleichtert es, das Gespräch zu planen. Um diese Vorhersage zu berechnen, verwendet die Erfassungs-Auswerteeinheit einerseits bevorzugt eine vorgegebene Standarddauer eines solchen Gesprächs. Weiterhin verwendet die Erfassungs-Auswerteeinheit die Einzelbewertung eines Eingabe-Parameters, bevorzugt die jeweilige Einzelbewertung mehrerer Eingabe-Parameter, oder die Gesamtbewertung für das Verhalten des Benutzers. Optional verwendet die Erfassungs-Auswerteeinheit zusätzlich die Anzahl der detektierten Inkonsistenz-Paare und / oder die erfasste Anzahl von Korrekturen oder Rückfragen und / oder die Information, wie viele Fragen von einer anderen Person beantwortet worden sind.

Erfindungsgemäß erzeugt der Darstellungs-Generierer eine Darstellungsdatei. Auf Basis dieser Darstellungsdatei lässt sich eine Darstellung erzeugen. Die nachfolgend beschriebene Ausgestaltung erleichtert es einer Person, welche diese Darstellung betrachtet, Fragen und / oder Antworten zu identifizieren, bei denen die Erfassungs-Auswerteeinheit Auffälligkeiten entdeckt hat.

Gemäß dieser Ausgestaltung sind in einer rechnerauswertbaren Form folgendes vorgegeben:
- mindestens ein Hervorhebungs-Kriterium, bevorzugt zwei verschiedene Hervorhebungs-Kriterien, und
- mindestens ein Hervorhebungs-Element, bevorzugt mindestens zwei verschiedene Hervorhebungs-Elemente.

Das oder jedes vorgegebene Hervorhebungs-Kriterium fungiert als jeweils eine Auswerteregel und spezifiziert jeweils ein vorgegebenes Hervorhebungs-Element. Das oder jedes Hervorhebungs-Kriterium hängt von jeweils mindestens einem Eingabe-Parameter ab, wobei der oder jeder Eingabe-Parameter von dem oder einem zugeordneten Eingabe-Parameter-Sensor der Erfassungs-Anordnung gemessen worden ist oder wenigstens gemessen werden kann, während ein Benutzer der Erfassungs-Anordnung Fragen der Fragen-Gruppe beantwortet hat und seine Antworten erfasst worden sind, d.h. dass der Eingabe-Parameter im Beantwortungs-Zeitraum gemessen worden ist. Bei mehreren Eingabe-Parameter sind in einer Ausgestaltung mindestens zwei verschiedene Hervorhebungs-Kriterien und / oder mindestens zwei verschiedene Hervorhebungs-Elemente vorgegeben. Ein Hervorhebungs-Kriterium kann für einen gemessenen Eingabe-Parameter-Wert oder eine Kombination von gemessenen Eingabe-Parameter-Werten erfüllt oder nicht erfüllt sein. Falls das Hervorhebungs-Kriterium erfüllt ist, so liefert es ein Hervorhebungs-Element, ansonsten das Ergebnis, dass die Prämisse des Hervorhebungs-Kriteriums nicht erfüllt ist.

Die Erfassungs-Auswerteeinheit ist dazu ausgestaltet, für mindestens eine Frage der Fragen-Gruppe, bevorzugt für jede Frage, folgende Prüfung durchzuführen: Erfüllt für diese Frage
- der oder ein Eingabe-Parameter-Wert, der bei der Erfassung der Antwort auf diese Frage gemessen worden ist, oder
- die Kombination derjenigen Eingabe-Parameter-Werten, die bei der Erfassung der Antwort gemessen worden sind,
das oder mindestens ein vorgegebenes Hervorhebungs-Kriterium?

Die Erfassungs-Auswerteeinheit vermag die Auswertungsdaten wie folgt zu erzeugen: Die Auswertungsdaten enthalten dann, wenn eine Frage das oder ein Hervorhebungs-Kriterium erfüllt, eine Kennzeichnung der Frage und eine Kennzeichnung des Hervorhebungs-Elements, welches im erfüllten Hervorhebungs-Kriterium auftritt.

Der Darstellungs-Generierer vermag die Darstellungsdatei so zu erzeugen, dass dann, wenn eine Darstellung auf Basis der erzeugten Darstellungsdatei ausgegeben wird, folgende Wirkung erzielt wird: Falls für eine Frage das oder mindestens ein Hervorhebungs-Kriterium erfüllt ist, so stellt die Darstellung die Frage und / oder die Antwort auf die Frage zusammen mit dem Hervorhebungs-Element dar, wobei eine Kennzeichnung der Frage und eine Kennzeichnung des Hervorhebungs-Elements in den Auswertungsdaten enthalten sind. Beispiele für Hervorhebungs-Elemente sind insbesondere die folgenden: Die Antwort wird in der Darstellung hervorgehoben dargestellt, beispielsweise mit einer anderen Schriftart oder Schriftgröße oder unterstrichen oder kursiv oder mit einem Rahmen oder sonstigem grafischen Element oder mit einem Bild, das bei der Erfassung der eingegebenen Antwort erzeugt wurde.

Gemäß dieser Ausgestaltung umfasst die erfindungsgemäß erzeugte Darstellungsdatei nicht nur die erfassten Antworten, sondern auch Hervorhebungs-Elemente für die Antworten. Die Hervorhebungs-Elemente ermöglichen es einem Menschen, diejenige Person, auf die sich die Fragen beziehen, gezielt zu befragen, also "nachzuhaken". Der Mensch braucht sich nicht allein auf die erfassten Antworten zu verlassen, sondern erhält durch die Hervorhebungs-Elemente ein Hilfsmittel, insbesondere ein Hilfsmittel für die Entscheidung, welche Fragen erneut gestellt werden sollten und welche nicht erneut gestellt zu werden brauchen.

Die Erfindung betrifft weiterhin eine Anordnung, wobei die Anordnung eine erfindungsgemäße Erfassungs-Anordnung und eine datenverarbeitende Darstellungs-Anordnung umfasst. Die Darstellungs-Anordnung ist insbesondere ein tragbarer Rechner und umfasst eine Ausgabeeinheit, insbesondere einen Bildschirm oder eine sonstige Ausgabefläche. Die Darstellungs-Anordnung vermag folgende Schritte durchzuführen:
- Die Darstellungs-Anordnung erfasst eine Darstellungsdatei, wobei diese Darstellungsdatei vom Darstellungs-Generierer der Erfassungs-Anordnung erzeugt worden ist. In einer Realisierungsform ist wenigstens zeitweise eine Datenverbindung zwischen der Erfassungs-Anordnung und der Darstellungs-Anordnung hergestellt, und die Darstellungsdatei wird über diese Datenverbindung übermittelt. In einer anderen Realisierungsform bewirkt die Erfassungs-Anordnung, dass die erzeugte Darstellungsdatei in einem Datenspeicher abgespeichert wird, beispielsweise "in der Cloud", und die Darstellungs-Anordnung liest die Darstellungsdatei aus dem Datenspeicher ein.
- Die Darstellungs-Anordnung generiert eine Darstellung, wobei sie die Darstellung auf Basis der erfassten Darstellungsdatei generiert.
- Die Darstellungs-Anordnung gibt die generierte Darstellung aus, und zwar in mindestens einer von einem Menschen wahrnehmbaren Form, insbesondere visuell.

Im Folgenden wird die Erfindung anhand eines Ausführungsbeispiels beschrieben. Hierbei zeigt
- Figur 1: wie der Patient mithilfe eines Smartphones Antworten auf die Fragen eines Fragebogens eingibt und wie Protokolldaten erzeugt werden;
- Figur 2: wie Auswertungsdaten und zwei Darstellungsdateien erzeugt werden;
- Figur 3: wie ein Arzt mit dem Patienten ein Beratungsgespräch führt und hierbei eine erfindungsgemäß erzeugte Darstellung verwendet;
- Figur 4: ein Ergebnis eines Bildvergleichs: Der Patient selbst hat eine Frage beantwortet;
- Figur 5: ein anderes Ergebnis eines Bildvergleichs: Eine andere Person hat die Frage beantwortet;
- Figur 6: ein weiteres Ergebnis eines Bildvergleichs: Eine andere Person hat dem Patienten bei der Beantwortung einer Frage geholfen.

Im Ausführungsbeispiel wird die Erfindung in folgender Situation verwendet: Ein Patient beabsichtigt, sich bei einer medizinischen Operation zu unterziehen, oder ist aus einem anderen Grunde für eine medizinische Operation vorgesehen. Bei dieser Operation soll der Patient narkotisiert oder wenigstens sediert werden. Vor der Operation soll der Patient Fragen beantworten. Ein behandelnder Arzt verwendet die Antworten auf diese Fragen, um einzuschätzen, welche Risiken während oder nach der Operation auftreten können. Ein mögliches Risiko ist das folgende: Nach dem Ende der Narkotisierung oder Sedierung tritt ein postoperatives Delir auf, auch als Durchgangssyndrom oder akutes organisches Psychosyndrom bezeichnet. Ob dieses unerwünschte Ereignis auftritt oder nicht, hängt neben anderen Umständen auch vom körperlichen und mentalen Zustand des Patienten vor Beginn der Operation ab. Insbesondere möchte der behandelnde Arzt in vielen Fällen feststellen, ob der Patient an Demenz leidet und / oder ob der Patient ein Narkosemittel oder ein sonstiges Medikament nicht verträgt, welches für die Operation vorgesehen ist.

Ein Fragebogen mit mehreren Fragen ist in einer rechnerauswertbaren Form vorgegeben. Wenigstens einige dieser Fragen beziehen sich auf den gesundheitlichen Zustand, die medizinische Vorgeschichte, die Einnahme von Medikamenten, Allergien und / oder das körperliche Verhalten des Patienten. Einige Fragen können auch dafür geeignet sein, um mittels der Antworten den körperlichen oder mentalen oder psychischen Zustand des Patienten zu ermitteln. Weiterhin ist eine Fragen-Gruppe vorgegeben, die mindestens eine Frage des Fragebogens umfasst, bevorzugt mehrere Fragen, in einer Ausgestaltung alle Fragen.

Im Ausführungsbeispiel wird ein zweiphasiges Vorgehen angewendet:
In einer ersten Phase verwendet ein Benutzer eine erfindungsgemäße Erfassungs-Anordnung. Diese Erfassungs-Anordnung umfasst eine Ausgabeeinheit und eine Eingabeeinheit und wird nachfolgend näher beschrieben. Der Fragebogen ist der Erfassungs-Anordnung in einer rechnerauswertbaren Form vorgegeben. Beispielsweise ist der Fragebogen unter einer Adresse im Internet verfügbar. Die Ausgabeeinheit gibt die Fragen des Fragebogens in mindestens einer von einem Menschen wahrnehmbaren Form aus. Die Eingabeeinheit erfasst die jeweilige Antwort des Benutzers auf eine ausgegebene Frage oder detektiert, dass der Benutzer diese Frage nicht beantwortet hat.

Die Erfassungs-Anordnung generiert in einer rechnerauswertbaren Form eine Darstellungsdatei. Diese Darstellungsdatei umfasst
- jede Frage des Fragebogens,
- die jeweils erfasste Antwort des Benutzers auf die Frage oder eine Kennzeichnung, dass die Frage nicht beantwortet wurde, sowie
- weiter unten beschriebene Ergebnisse einer Auswertung, beispielsweise Hervorhebungs-Elemente, und optional weitere Informationen, die bei der Beantwortung des Fragebogens ermittelt wurden.

Nachfolgend werden die Bezeichnungen "Patient" und "Benutzer" verwendet. Der Patient ist diejenige Person, die operiert werden soll und auf den sich die Fragen des Fragebogens beziehen. Der Benutzer ist diejenige Person, welche in der ersten Phase die Erfassungs-Anordnung benutzt, um Fragen betreffend den Patienten zu beantworten. Möglich ist, dass der Benutzer der Patient selbst ist. Möglich ist auch, dass eine andere Person als der Patient die Erfassungs-Anordnung benutzt oder dass einige Fragen vom Patienten und einige Fragen von mindestens einer anderen Person mittels der Erfassungs-Anordnung beantwortet werden oder dass eine andere Person dem Patienten bei der Beantwortung einiger Fragen hilft.

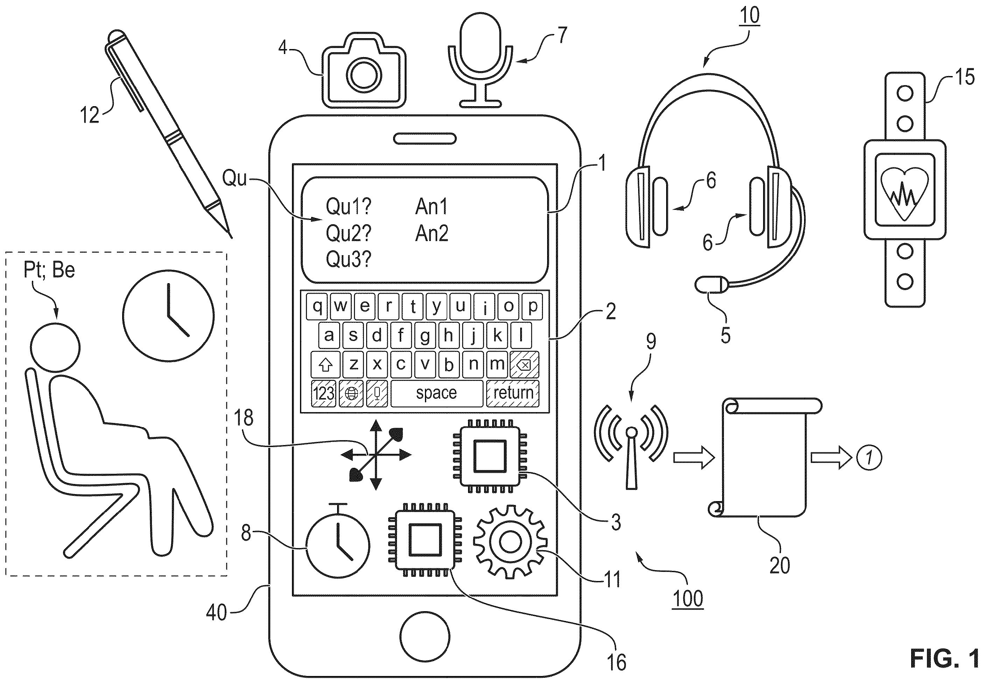

Figur 1 zeigt schematisch und beispielhaft einen Patienten Pt, wobei der Patient Pt eine Erfassungs-Anordnung 100 verwendet, um die oder wenigstens einige Fragen des Fragebogens Qu zu beantworten. In diesem Beispiel ist der Patient Pt selbst der Benutzer Be.

Die Erfassungs-Anordnung 100 ist im Ausführungsbeispiel dadurch auf einem handelsüblichen Smartphone 40 realisiert, dass ein Softwareprogramm auf dem Smartphone 40 installiert worden ist und von einem Prozessor des Smartphones 40 ausgeführt wird. Folgende Bestandteile des verwendeten Smartphones 40 und damit der Erfassungs-Anordnung 100 werden in Figur 1 schematisch dargestellt:
- eine visuelle Ausgabeeinheit 1,
- eine Bildschirmtastatur 2,
- eine Kamera 4,
- ein Mikrophon 7,
- ein Headset 10 mit zwei Lautsprechern 6 eines Kopfhörers und mit einem Mikrophon 5,
- ein Bildschirmstift 12,
- ein Prozessor, auf dem eine nachfolgend beschriebene signalverarbeitende Erfassungs-Auswerteeinheit 13 abläuft,
- ein Bewegungssensor 18,
- eine Stoppuhr 8,
- ein Betätigungselement 11,
- ein signalverarbeitendes Steuergerät (control unit) 16 und
- eine Kommunikationseinheit 9.

Die Erfassungs-Anordnung 100 gibt auf einem Bildschirm 1 des Smartphones 40 die Fragen des Fragebogens Qu visuell aus. Beispielhaft werden drei Fragen Qu1?, Qu2?, Qu3? gezeigt. Bevorzugt werden auch die erfassten Antworten auf der Ausgabeeinheit 1 dargestellt, sodass der Benutzer Be seine Eingabe visuell überprüfen und bei Bedarf korrigieren kann. Der Benutzer Be hat die ersten beiden Fragen Qu1?, Qu2? beantwortet, indem er die Antworten An1, An2 eingegeben hat. Um eine Antwort einzugeben, kann der Benutzer Be die Bildschirmtastatur 2 verwenden. Bevorzugt ist der Bildschirm 1 berührungssensitiv (ein Touch Screen), und der Benutzer Be kann eine Antwort auch mithilfe des Bildschirmstifts 12 eingeben.

Der Benutzer Be der Erfassungs-Anordnung 100 kann das Headset 10 benutzen, um sich mithilfe der Lautsprecher 6 des Headsets 10 die Fragen des Fragebogens Qu akustisch ausgeben zu lassen. Der Benutzer Be kann eine Antwort auf eine Frage des Fragebogens Qu auch mithilfe des Mikrophons 5 eingeben.

Mithilfe des Betätigungselements 11 kann der Benutzer Be Einstellungen des Smartphones 40 und damit der Erfassungs-Anordnung 100 ändern. Zu diesen Einstellungen gehören insbesondere
- ob die nächste Frage visuell oder akustisch oder sowohl visuell als auch akustisch ausgegeben werden soll,
- ob die Antwort auf die nächste Frage mittels der Bildschirmtastatur 2 oder mittels des Mikrophons 5 eingegeben werden soll,
- die Zeichengröße, mit der die Ausgabeeinheit 1 Fragen und Antworten visuell darstellt, sowie
- die Lautstärke, mit der die Lautsprecher 6 des Headsets 10 Fragen akustisch ausgibt.

Die Kamera 4 vermag Bilder von einem Benutzer Be der Erfassungs-Anordnung 100 zu erzeugen und fungiert im Ausführungsbeispiel als das Bildaufnahmegerät. Das Mikrophon 7 vermag Audiosignale zu erfassen, wobei diese Audiosignale in einer Umgebung der Erfassungs-Anordnung 100 entstanden sind, und fungiert im Ausführungsbeispiel als die Audiosignal-Erfassungseinheit.

In einer Ausgestaltung kann ein Benutzer der Erfassungs-Anordnung 100 eine Rückfrage zu einer Frage stellen, wobei diese Frage visuell auf dem Bildschirm 1 oder akustisch mithilfe der Lautsprecher 6 ausgegeben wird. Der Benutzer kann wahlweise eine Antwort auf die ausgegebene Frage oder eine Rückfrage zur Frage eingeben. In einer Ausgestaltung gibt der Benutzer zunächst vor, ob seine Eingabe als eine Antwort auf die Frage oder als eine Rückfrage verwendet werden soll. Standardmäßig befindet die Erfassungs-Anordnung 100 sich in einem Eingabemodus, und eine Eingabe wird als Antwort verwendet. Um eine Rückfrage zu stellen, betätigt der Benutzer beispielsweise eine Taste auf der Bildschirmtastatur 2, wobei die Taste beispielsweise mit einem Fragezeichen beschriftet ist. Dadurch wird die Erfassungs-Anordnung 100 in einen Rückfragemodus umgeschaltet. In einer anderen Ausgestaltung vermag ein Textanalysierer automatisch zu erkennen, ob eine Eingabe des Benutzers eine Antwort auf eine ausgegebene Frage ist oder eine Rückfrage zu der ausgegebenen Frage. Falls der Textanalysierer entschieden hat, dass die Eingabe des Benutzers eine Rückfrage ist, so bewirkt der Textanalysierer, dass die Erfassungs-Anordnung 100 in den Rückfragemodus umgeschaltet wird.

Der Benutzer gibt seine Rückfrage ein, wobei die Rückfrage beispielsweise einen medizinischen Begriff, der in der Frage auftritt, enthält. Die Erfassungs-Anordnung 100 gibt als Reaktion auf die Rückfrage eine Erläuterung aus, und zwar auf dem Bildschirm 1 oder mithilfe der Lautsprecher 6, und schaltet sich wieder in einen Eingabemodus um. Der Benutzer kann wiederholt eine Rückfrage zu derselben Frage stellen.

Wie bereits dargelegt, kann in einer Ausgestaltung der Benutzer eine Rückfrage in Form von Freitext eingeben. Ein signalverarbeitende Textanalysierer generiert automatisch eine Antwort auf die eingegebene Rückfrage und bewirkt, dass diese Antwort ausgegeben wird. In einer Ausgestaltung prüft der Textanalysierer automatisch folgendes:
- Wie viele inhaltlich übereinstimmende Rückfragen hat der Benutzer insgesamt bislang gestellt, insbesondere zu derselben Frage der Fragen-Gruppe? Viele inhaltlich übereinstimmende Rückfragen von demselben Benutzer können ein Zeichen für Unsicherheit sein.
- Ist eine Rückfrage eine sinnvolle Rückfrage zu einer Frage der Fragen-Gruppe? Oder die Rückfrage nichts mit der Frage zu tun? Nicht sinnvolle Rückfragen können ein Anzeichen für eine mentale Einschränkung des Benutzers oder auch ein Anzeichen für relativ geringe Sprachkenntnisse sein.

Mögliche Eingabe-Parameter sind die folgenden:
- Wie viele Rückfragen zu einer Frage wurden gestellt?
- Wie viele dieser Rückfragen stimmen inhaltlich über ein?
- Wie viele dieser Rückfragen haben nichts mit der Frage zu tun?

Ein signalverarbeitender Protokoll-Generierer 3 der Erfassungs-Anordnung 100 erzeugt in einer rechnerauswertbaren Form Protokolldaten 20. Die Protokolldaten 20 umfassen in einer rechnerauswertbarer Form
- die Fragen des Fragebogens Qu,
- die erfassten Antworten des Benutzers Be auf die Fragen sowie
- Informationen über das gemessene Verhalten des Benutzers Be bei der Beantwortung der Fragen.

Die Informationen über das Verhalten werden weiter unten beschrieben.

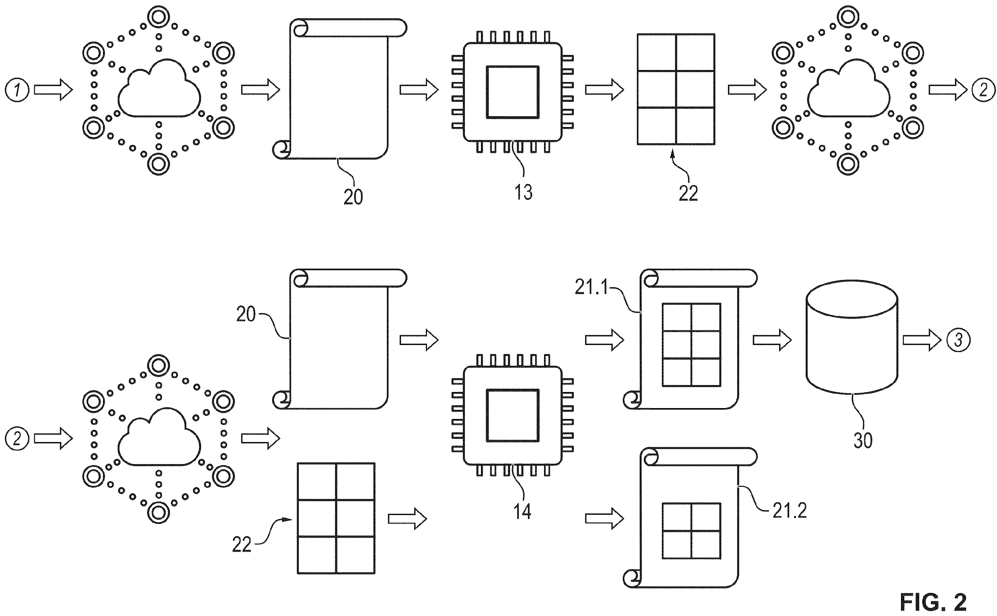

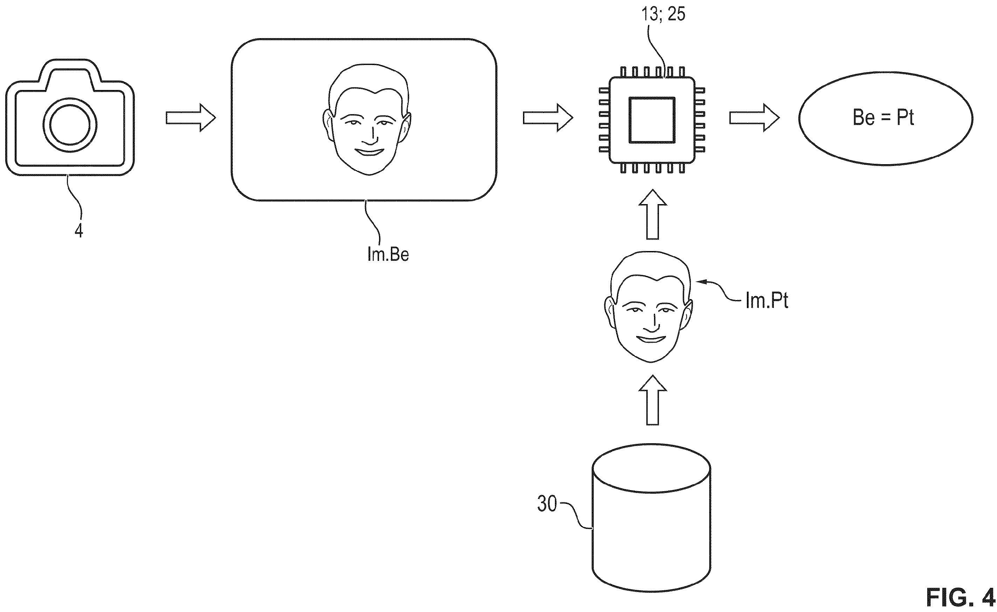

Die Erfassungs-Anordnung 100 bewirkt, dass die Kommunikationseinheit 9 die Protokolldaten 20 an einen räumlich entfernten Zentralrechner (nicht gezeigt) übermittelt, beispielsweise "in der Cloud", vgl. Figur 2. Der Zentralrechner speichert die Protokolldaten 20 in einer Datenbank 30 ab. In dieser Datenbank 30 ist bereits ein Datensatz für den Patienten Pt abgespeichert. Im Ausführungsbeispiel umfasst dieser Datensatz ein Referenzbild Im.Pt des Patienten Pt, vgl. Figur 4 bis Figur 6.

Während der Benutzer Be die Erfassungs-Anordnung 100 verwendet, um die Fragen zu beantworten, braucht kein weiterer Mensch anwesend oder aus der Ferne zugeschaltet sein. Der Benutzer Be kann die Erfassungs-Anordnung 100 an einem von ihm zu wählenden Ort verwenden und braucht insbesondere kein Krankenhaus und keine Arztpraxis aufzusuchen, um die Fragen zu beantworten. Der Benutzer Be kann ein eigenes Signalverarbeitungsgerät verwenden, beispielsweise ein eigenes Smartphone 40, auf dem der Benutzer Be das entsprechende Softwareprogramm installiert.

Ein signalverarbeitender Darstellungs-Generierer 14 der Erfassungs-Anordnung 100 erzeugt unter Verwendung der Protokolldaten 20 mindestens eine Darstellungsdatei, vgl. Figur 2. Im Beispiel von Figur 2 werden zwei verschiedene Darstellungsdateien 21.1, 21.2 gezeigt. Die oder jede Darstellungsdatei 21.1, 21.2 enthält die Fragen und die Antworten, die auch in den Protokolldaten 20 enthalten sind, sowie Auswertungen, die auf Basis des gemessenen Verhaltens des Benutzers automatisch erzeugt worden sind. Verschiedene Darstellungsdateien 21.1, 21.2 können sich insbesondere durch unterschiedliche Auswertungen und / oder unterschiedliche Detaillierungen voneinander unterscheiden.

In einer zweiten Phase trifft ein behandelnder Arzt den Patienten Pt, beispielsweise in einem Besprechungszimmer eines Krankenhauses, und führt ein Gespräch mit dem Patienten Pt. Der Fragebogen Qu, die erzeugten Protokolldaten 20, die oder jede Darstellungsdatei 21.1, 21.2 und eine Darstellung, die auf Basis der oder einer Darstellungsdatei 21.1, 21.2 ausgegeben wird, beziehen sich auf diesen Patienten Pt. Der Arzt führt das Gespräch mit dem Ziel, zu ermitteln, welche Risiken die geplante Operation für den Patienten Pt birgt oder bergen könnte.

Der Arzt verwendet während des Gesprächs einen Rechner. Dieser Rechner hat Lesezugriff auf die oder eine Darstellungsdatei 21.1, 21.2, welche die Erfassungs-Anordnung 100 erzeugt hat, und generiert eine Darstellung, die von einem Menschen visuell wahrnehmbar ist. Um diese Darstellung zu generieren, wertet der Rechner die Darstellungsdatei 21.1, 21.2 aus. Während des Gesprächs gibt dieser Rechner die generierte Darstellung visuell aus.

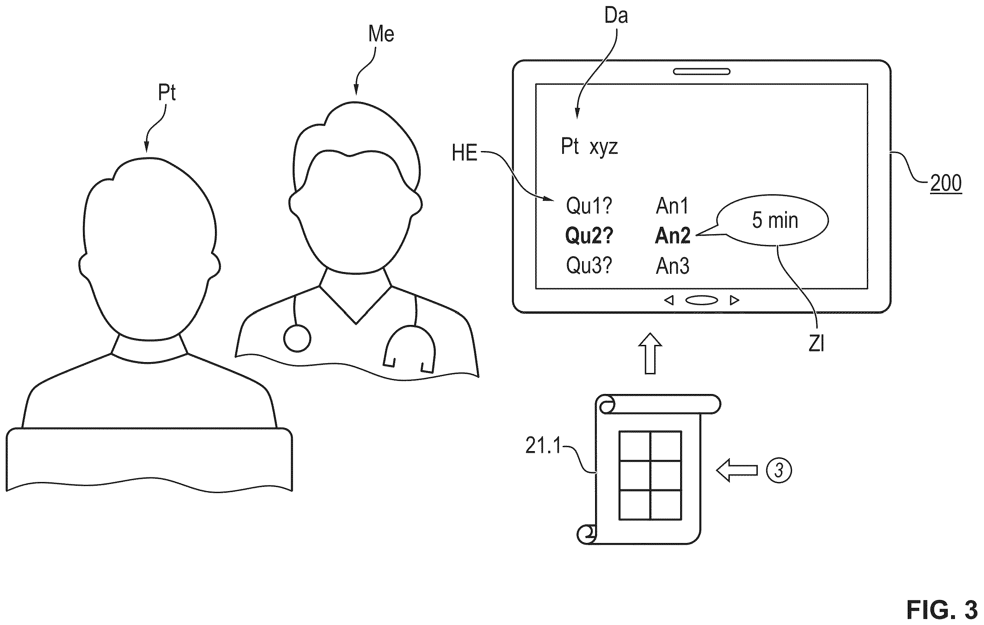

Figur 3 zeigt schematisch einen Arzt Me, der mit dem Patienten Pt das Gespräch führt. Der Arzt Me verwendet einen tragbaren Rechner in Form eines Tablets 200. Das Tablet 200 fungiert als die Ausgabe-Anordnung und hat wenigstens zeitweise Lesezugriff auf die Datenbank 30, und die Darstellungsdatei 21.1 wird an das Tablet 200 übermittelt. Das Tablet 200 wertet die übermittelte Darstellungsdatei 21.1 aus und erzeugt in einer visuell wahrnehmbaren Form eine Darstellung Da, die der Arzt Me sich betrachten und beim Gespräch verwenden kann.

Während des Gesprächs wird die erzeugte Darstellung Da dem Arzt Me in mindestens einer von einem Menschen wahrnehmbaren Form dargestellt. Die Darstellung Da zeigt erfasste Antworten des Benutzers auf Fragen des Fragebogens Qu sowie Informationen über das Verhalten des Benutzers bei der Beantwortung der Fragen und / oder Ergebnisse der Auswertung. Möglich ist, dass der Arzt Me einzelne Informationen der Darstellung einblenden und / oder ausblenden kann.

In einer Ausgestaltung unterstützen einige nachfolgend beschriebene Hervorhebungs-Elemente HE in der Darstellung Da den Arzt Me dabei, das Gespräch mit dem Patienten Pt zu führen. In einer Realisierungsform werden mittels der Hervorhebungs-Elemente HE einzelne Antworten auf Fragen des Fragebogens Qu hervorgehoben dargestellt, und dem Arzt Me wird die Möglichkeit gegeben, diese Fragen erneut zu stellen und / oder Nachfragen zu stellen. In einer Ausgestaltung werden dem Arzt Me Zusatzinformationen zu einzelnen Antworten dargestellt. Zwar möglich, aber dank der Erfindung nicht notwendig ist es, dass der Arzt Me alle Fragen des Fragebogens Qu erneut stellt. Dadurch wird Arbeitszeit des Arztes Me eingespart.

Figur 3 zeigt beispielhaft, dass die Frage Qu2 des Fragebogens Qu und die Antwort An2 hervorgehoben dargestellt werden, nämlich durch Fettdruck. Fettdruck ist ein Beispiel für ein Hervorhebungs-Element HE. Weitere Beispiele für ein Hervorhebungs-Element sind die folgenden:
- eine optische Markierung durch Unterstreichung, kursive Schrift, anderer Schrifttyp,
- eine Farbcodierung, insbesondere rot, gelb, grün abhängig von Schwellenwerten,
- ein blinkender Rahmen.

Außerdem kann sich der Arzt Me eine Zusatzinformation ZI zu einer hervorgehoben dargestellten Antwort anzeigen lassen. Beispielhaft wird die Zusatzinformation ZI angezeigt, dass die Beantwortung der Frage Qu2? 5 min gedauert hat, was in diesem Beispiel ungewöhnlich lange ist.

Soweit nicht ausdrücklich anders erwähnt, bezieht sich die nachfolgende Beschreibung auf die erste Phase.

Die Erfassungs-Anordnung 100 ist mithilfe eines signalverarbeitenden Geräts realisiert, insbesondere mithilfe eines handelsüblichen PCs, sonstigen Desktop-Rechners, Smartphones oder Tablets. Im Beispiel von Figur 1 wird ein Smartphone 40 gezeigt, das stellvertretend für andere mögliche signalverarbeitende Geräte steht. Das Smartphone 40 und damit die Erfassungs-Anordnung 100 umfassen mindestens einen Datenspeicher und einen Prozessor. Im Datenspeicher ist ein Softwareprogramm abgespeichert, beispielsweise eine App. Der Prozessor vermag das abgespeicherte Softwareprogramm zu laden und auszuführen.

Die Erfassungs-Anordnung 100 liefert als ein Ergebnis die gerade beschriebenen Protokolldaten 20. Über die Kommunikationseinheit 9 steht die Erfassungs-Anordnung 100 wenigstens zeitweise in einer Datenverbindung mit einem räumlich entfernten Zentralrechner. In einer ersten Ausgestaltung generiert das Softwareprogramm auf dem signalverarbeitenden Gerät die Protokolldaten 20. Die Kommunikationseinheit 9 bewirkt, dass die Protokolldaten 20 an den räumlich entfernten Zentralrechner übermittelt werden, vgl. Figur 2. In einer zweiten Ausgestaltung übermittelt die Kommunikationseinheit 9 ein Signal umfassend Informationen über die erfassten Antworten des Benutzers an den räumlich entfernten Zentralrechner, und ein Softwareprogramm auf diesem Rechner (nicht gezeigt) generiert die Protokolldaten 20.

Im gezeigten Beispiel sind die Erfassungs-Auswerteeinheit 13 und der Darstellungs-Generierer 14 auf dem Zentralrechner installiert, also räumlich entfernt von dem Smartphone 40. Eine mögliche Alternative ist, dass auch die Erfassungs-Auswerteeinheit 13 und / oder der Darstellungs-Generierer 14 auf dem Smartphone 40 installiert ist. Die Erfassungs-Auswerteeinheit 13 und / oder der Darstellungs-Generierer 14 können auch auf einem Gerät installiert sein, das in der zweiten Phase verwendet wird, beispielsweise auf dem Tablet 200 von Figur 3.

Das Softwareprogramm, das die Protokolldaten 20 generiert, gehört zum erfindungsgemäßen Protokoll-Generierer 3. Um die Erfindung zu implementieren, reicht es in vielen Fällen aus, den Protokoll-Generierer 3 und optional die Erfassungs-Auswerteeinheit 13 und / oder den Darstellungs-Generierer 14 auf ein bereits vorhandenes handelsübliches Gerät zu laden, beispielsweise auf ein handelsübliches Smartphone 40, und dort auszuführen. Oft kann ein Benutzer ein Gerät, welches ihm ohnehin zur Verfügung steht, verwenden, um aus dem Gerät durch Ergänzung von Software eine erfindungsgemäße Erfassungs-Anordnung 100 oder wenigstens einen Bestandteil einer erfindungsgemäßen Erfassungs-Anordnung 100 zu machen und mit dieser den Fragebogen Qu auszufüllen. Bei der zweiten Ausgestaltung, bei der ein räumlich entfernter Rechner verwendet wird, reicht es in vielen Fällen aus, Software auf dem Gerät 40, das der Benutzer verwendet, und auf dem räumlich entfernten Rechner zu installieren. Die weiteren benötigten Komponenten sind in der Regel bereits vorhanden.

Im Ausführungsbeispiel umfasst die Erfassungs-Anordnung 100 eine audiovisuelle Ausgabeeinheit 1, 6. Die Ausgabeeinheit 1, 6 umfasst zum einen eine Anzeigefläche, beispielsweise den Bildschirm 1, und zum anderen eine Sprachausgabeeinheit, insbesondere die Lautsprecher 6. Die Ausgabeeinheit vermag einerseits die Fragen des Fragebogens Qu auf der Anzeigefläche 1 visuell darzustellen und dadurch auszugeben. Möglich ist, dass die Fragen nacheinander dargestellt werden. Möglich ist auch, dass mehrere Fragen gleichzeitig dargestellt werden. Möglich ist auch, dass ein Benutzer Be auswählen kann, welche Frage er als nächstes beantworten möchte. Die Sprachausgabeeinheit (der Lautsprecher) 6 vermag die Fragen auditiv auszugeben, also durch eine Sprachausgabe. Bevorzugt kann ein Benutzer Be wählen, auf welche Art die audiovisuelle Ausgabeeinheit 1, 6 die Fragen ausgeben soll, also insbesondere, ob er eine visuelle oder eine auditive Ausgabe der Fragen wünscht. Möglich ist auch, dass eine Frage sowohl visuell als auch auditiv ausgegeben wird.

Weiterhin umfasst die Erfassungs-Anordnung 100 eine audiovisuelle Eingabeeinheit 2, 5, 12. Zum einen umfasst die Eingabeeinheit eine Tastatur, insbesondere die Tastatur 2 auf dem berührungssensitiven Bildschirm (Touchscreen) 1, und / oder eine Auswahleinheit, wobei der Benutzer Be mithilfe der Auswahleinheit eine Alternative aus einem Auswahlmenü auswählen kann, bevorzugt mithilfe des berührungssensitiven Bildschirms 2 oder mithilfe einer Maus oder eines Touchpads. Das Auswahlmenü zeigt mögliche Antworten auf eine ausgegebene Frage. Der berührungssensitive Bildschirm 1 kann zugleich als Anzeigefläche 1 der Ausgabeeinheit fungieren, so wie dies von einem handelsüblichen Smartphone oder Tablet bekannt ist. Zur Eingabeeinheit 2, 5, 12 gehört weiterhin der Bildschirmstift 12.

Zum anderen umfasst die Eingabeeinheit 2, 5, 12 des Ausführungsbeispiels eine Spracheingabeeinheit, wobei die Spracheingabeeinheit das Mikrophon 5 umfasst. Im Ausführungsbeispiel umfasst die Erfassungs-Anordnung 100 das Headset 10 mit einem Kopfhörer, der die beiden Lautsprecher 6 der Sprachausgabeeinheit trägt, und das Mikrophon 5, das zur Spracheingabeeinheit gehört.

Die Eingabeeinheit 2, 5, 12 erfasst die jeweilige Eingabe eines Benutzers Be, wobei der Benutzer Be die Eingabe als Reaktion auf die Ausgabe einer Frage tätigt. Bevorzugt kann der Benutzer Be auswählen, auf welche Art er die Antworten auf die Fragen eingeben will, also insbesondere, ob er sie visuell oder auditiv eingeben will.

Möglich ist, dass der Benutzer Be folgendes vorgibt: Die Ausgabeeinheit 1, 6 soll die Fragen visuell auf der Anzeigefläche 1 ausgeben, und der Benutzer Be gibt die Eingabe mithilfe der Spracheingabeeinheit 5 ein. Oder umgekehrt soll die Ausgabeeinheit 1, 6 die Fragen mithilfe der Sprachausgabeeinheit 6 ausgeben, und der Benutzer Be beantwortet diese Fragen durch eine Eingabe auf der Anzeigefläche 1 und / oder der Tastatur 2 und / oder mit dem Bildschirmstift 12.

Die Erfassungs-Anordnung 100 des Ausführungsbeispiels umfasst weiterhin das Steuergerät 16 und ein Bildaufnahmegerät, insbesondere die Digitalkamera 4. Das Bildaufnahmegerät 4 ist auf einen Benutzer der Erfassungs-Anordnung 100 gerichtet, insbesondere auf einen Benutzer Be, der auf die Anzeigefläche 1 der Ausgabeeinheit blickt. Die Digitalkamera 4 vermag ein Bild von diesem Benutzer Be anzufertigen, bevorzugt eine Videosequenz.

Für jede Frage der Fragen-Gruppe ermittelt das Steuergerät 16 einen Beantwortungs-Zeitraum, wobei der Beantwortungs-Zeitraum
- mit der Ausgabe der Frage beginnt und
- endet, sobald die Eingabeeinheit 2, 5, 12 erfasst hat, dass eine Antwort auf diese Frage eingegeben worden und die Eingabe abgeschlossen ist, oder detektiert hat, dass der Benutzer Be diese Frage nicht beantwortet hat.

Um den Beantwortungs-Zeitraum zu ermitteln, verwendet das Steuergerät 16 die Stoppuhr 8.

Beispielsweise ist eine Maximal-Zeitspanne vorgegeben, und eine Frage gilt als nicht beantwortet, wenn innerhalb der Maximal-Zeitspanne nicht die Eingabe einer Antwort auf die Frage erfasst worden ist. Oder die Eingabeeinheit 2, 5, 12 hat explizit eine Benutzereingabe erfasst, die Frage nicht zu beantworten.

Das Steuergerät 16 steuert im Ausführungsbeispiel das Bildaufnahmegerät 4 so an, dass das Bildaufnahmegerät 4 in jedem Beantwortungs-Zeitraum für eine Frage der Fragen-Gruppe jeweils mindestens ein Bild des Benutzers Be erzeugt. Optional erzeugt das Bildaufnahmegerät 4 ein erstes Bild, während die Ausgabeeinheit 1, 6 die Frage ausgibt, und mindestens ein zweites Bild, während ein Benutzer Be eine Antwort auf die ausgegebene Frage eingibt. Diese Ausgestaltung berücksichtigt die Möglichkeit, dass zwar der Patient Pt die ausgegebene Frage liest oder hört, dann aber eine andere Person die ausgegebene Frage beantwortet.

Im Ausführungsbeispiel erzeugt das angesteuerte Bildaufnahmegerät 4 mindestens so viele Bilder, wie die Fragen-Gruppe Fragen umfasst. Jedes Bild zeigt jeweils einen Benutzer Be beim Vorgang, eine Antwort auf eine Frage einzugeben. Diese Ausgestaltung berücksichtigt die Möglichkeit, dass die Fragen der Fragen-Gruppe von mindestens zwei verschiedene Benutzer beantwortet werden.

In einer Ausgestaltung umfasst die Erfassungs-Anordnung 100 mindestens einen Vitalparameter-Sensor 15, insbesondere einen Sensor für die folgende Vitalparameter:
- den Blutdruck,
- den Puls-Sensor (Sensor für die Frequenz des Herzschlags),
- die Leitfähigkeit der Haut,
- die Sauerstoffsättigung des Blutes (den Gehalt an SpO₂).

Mithilfe des Blutdrucks und des Pulses lässt sich häufig entscheiden, ob ein Mensch in Panik geraten ist oder nicht. Der oder jeder Vitalparameter-Sensor 15 vermag jeweils einen Vitalparameter eines Benutzers Be der Erfassungs-Anordnung 100 zu messen. Bevorzugt steuert das Steuergerät 16 den oder jeden Vitalparameter-Sensor 15 so an, dass der Vitalparameter-Sensor 15 den Vitalparameter mindestens einmal im oben beschriebenen Beantwortungs-Zeitraum, der zum Beantworten einer Frage der Fragen-Gruppe verwendet wird, misst. Wenn der Fragebogen Qu beantwortet worden ist, liegen gemäß dieser Ausgestaltung mindestens so viel Vitalparameter-Werte vor, wie die Fragen-Gruppe Fragen aufweist. Möglich ist auch, dass der Vitalparameter-Sensor 15 den Vitalparameter des Benutzers Be in regelmäßigen Abständen misst und dass festgestellt wird, welcher Beantwortungs-Zeitraum in welchen Mess-Zeitraum des Vitalparameter-Sensors 15 fällt.

Die Erfassungs-Anordnung 100 umfasst weiterhin eine signalverarbeitende Erfassungs-Auswerteeinheit 13, die in einer Realisierungsform als Teil des Softwareprogramms realisiert ist, welches vom Prozessor ausgeführt werden kann. In einer anderen Realisierungsform ist wenigstens ein Teil der Erfassungs-Auswerteeinheit 13 auf einem Rechner installiert, der räumlich vom Smartphone 40 entfernt ist, vgl. Figur 2. Bestandteile dieser Erfassungs-Auswerteeinheit 13 werden nachfolgend beschrieben.

In einer Ausgestaltung umfasst die Erfassungs-Auswerteeinheit 13 eine Bildauswerteeinheit. Die Bildauswerteeinheit vermag die Bilder des Bildaufnahmegeräts 4 auszuwerten. Durch die Auswertung vermag die Bildauswerteeinheit insbesondere folgendes zu entscheiden:
- Ist für jede Frage folgende Bedingung erfüllt: In dem oder mindestens einem Bild, das das Bildaufnahmegerät 4 bei der Beantwortung der Frage erzeugt hat, das Gesicht des Benutzers Be zu sehen, ohne dass dessen Gesicht verdeckt oder maskiert ist oder sich im Dunkeln befindet?
- Wurde jede Frage von demselben Benutzer Be beantwortet? Oder wurden die Fragen insgesamt von mindestens zwei verschiedenen Benutzern beantwortet?
- Ist im oben beschriebenen Beantwortungs-Zeitraum für eine Frage zusätzlich zum Benutzer Be eine weitere Person in dem oder einem Bild, das im Beantwortungs-Zeitraum aufgenommen worden ist, zu sehen? Wenn ja: Ist diese weitere Person zum Benutzer Be gewandt und / oder bleibt in der Nähe des Benutzers stehen, scheint also dem Benutzer Be bei der Beantwortung der Frage zu unterstützen? Oder ist diese weitere Person nur "zufällig" ins Bild geraten?
- Welche Augenbewegungen hat der Benutzer Be durchgeführt, während er eine Frage beantwortet hat? Hat der auffallend viel oder auffallend weniger Augenbewegungen durchgeführt?

Möglich ist auch folgende Situation: Ein Benutzer Be richtet sprachlich eine Frage an eine andere Person, die andere Person beantwortet diese Frage oder richtet eine Anweisung an den Benutzer Be, und der Benutzer Be verwendet die Antwort oder Anweisung dieser anderen Person, um mit der Eingabeeinheit 2, 5, 12 eine Frage des Fragebogens Qu zu beantworten. Die andere Person kann sich in der Nähe des Benutzers Be aufhalten, oder der Benutzer Be telefoniert mit der anderen Person.

Diese Situation lässt sich in vielen Fällen durch die nachfolgend beschriebene Ausgestaltung entdecken. Gemäß dieser Ausgestaltung umfasst das Smartphone 40 eine Audiosignal-Erfassungseinheit mit dem Mikrophon 7, und die Erfassungs-Auswerteeinheit 13 umfasst eine signalverarbeitende Audiosignal-Auswerteeinheit. Gemäß dieser Ausgestaltung vermag die Erfassungs-Anordnung 100 automatisch folgende Schritte durchzuführen:
- Die Audiosignal-Erfassungseinheit 7 erfasst ein Audiosignal, welches in einer Umgebung der Erfassungs-Anordnung 100 entstanden ist.
- Die Audiosignal-Auswerteeinheit entscheidet, ob das erfasste Audiosignal mindestens eine Sprachnachricht umfasst, also gesprochene Sprache.

Falls das Audiosignal mindestens eine Sprachnachricht umfasst, so werden mindestens einige der folgenden Schritte durchgeführt:
- Die Audiosignal-Auswerteeinheit entscheidet, ob die detektierte Sprachnachricht eine gesprochene Antwort auf eine Frage ist oder umfasst, wobei der Benutzer Be diese Antwort in die Spracheingabeeinheit 5 eingibt, oder nicht. Insbesondere entscheidet die Audiosignal-Auswerteeinheit, ob eine Sprachnachricht erfasst worden ist, während der Benutzer Be die Tastatur 2 oder eine andere visuelle Eingabeeinheit verwendet.
- Die Audiosignal-Auswerteeinheit entscheidet, ob eine detektierte Sprachnachricht sich auf eine Frage des Fragebogens Qu bezieht oder quasi ein Hintergrundgeräusch ist. Beispiele für Hintergrundgeräusche sind: ein gleichzeitig geführtes Gespräch, ein Selbstgespräch des Benutzers Be oder ein Geräusch, das von einem Tier oder einem Gerät verursacht wird und daher keine natürliche Sprache umfasst.
- Die Audiosignal-Auswerteeinheit zählt, von wie vielen verschiedenen Menschen die Sprachnachrichten des Audiosignals insgesamt stammen. Mindestens prüft die Audiosignal-Auswerteeinheit, ob die Sprachnachrichten alle von derselben Person oder von insgesamt zwei verschiedenen Personen stammen.

Durch die nachfolgend beschriebene Ausgestaltung wird festgestellt, ob der Patient Pt selbst alle Fragen beantwortete oder ob mindestens eine Frage von einer anderen Person beantwortet wurde. Gemäß dieser Ausgestaltung ist in einer rechnerauswertbaren Form mindestens ein Referenzbild Im.Pt vorgegeben, wobei das oder mindestens ein Referenzbild Im.Pt das Gesicht des Patienten Pt zeigt. Der vorgegebene Fragebogen Qu bezieht sich auf diesen Patienten Pt. Das oder jedes Referenzbild Im.Pt ist bevorzugt in einem Datenspeicher der Erfassungs-Anordnung 100 oder in einem räumlich entfernten Datenspeicher abgespeichert, im Ausführungsbeispiel im Datenspeicher 30, und ist dem Patienten Pt und damit dem Fragebogen Qu zugeordnet.

Wie bereits dargelegt, erzeugt das angesteuerte Bildaufnahmegerät 4 für jede Frage der Fragen-Gruppe jeweils mindestens ein Bild eines Benutzers Be, während dieser Benutzer Be die Frage beantwortet. Dieses Bild wird nachfolgend als das Benutzerbild Im.Be für die Frage bezeichnet.

Figur 4 bis Figur 6 veranschaulichen die nachfolgend beschriebene vorteilhafte Ausgestaltung. Wie bereits dargelegt, ist in der Datenbank 30 ein Datensatz über den Patienten Pt abgespeichert. Dieser Datensatz umfasst ein rechnerauswertbares Referenzbild Im.Pt des Patienten Pt. Die Kamera 4 hat mindestens ein Benutzerbild Im.Be erzeugt, welches einen Benutzer Be der Erfassungs-Anordnung 100 zeigt, während dieser Benutzer Be eine Antwort auf eine Frage eingibt.

Die Erfassungs-Auswerteeinheit 13 umfasst eine signalverarbeitende Bildvergleichseinheit 25. Bevorzugt prüft die Bildvergleichseinheit 25 zunächst, ob das oder mindestens ein vorgegebenes Referenzbild Im.Pt ausreichend deutlich das Gesicht eines Menschen zeigt. Falls mehrere Referenzbilder vorgegeben sind, prüft die Bildvergleichseinheit 25 bevorzugt, ob alle diese Referenzbilder das Gesicht desselben Menschen zeigen. Die Bildvergleichseinheit 25 vergleicht also mindestens zwei Bilder miteinander.

Falls beides der Fall ist, vergleicht die Bildvergleichseinheit 25 das oder jedes Benutzerbild Im.Be mit dem oder jedem vorgegebenen Referenzbild Im.Pt. Als Ergebnis des Bildvergleichs entscheidet die Bildvergleichseinheit 25 automatisch, welche Fragen der Patient Pt selbst und welche Fragen mindestens eine andere Person beantwortet hat. Mit Be (Benutzer der Erfassungs-Anordnung 100) wird die Person bezeichnet, die eine Antwort auf eine Frage eingibt.

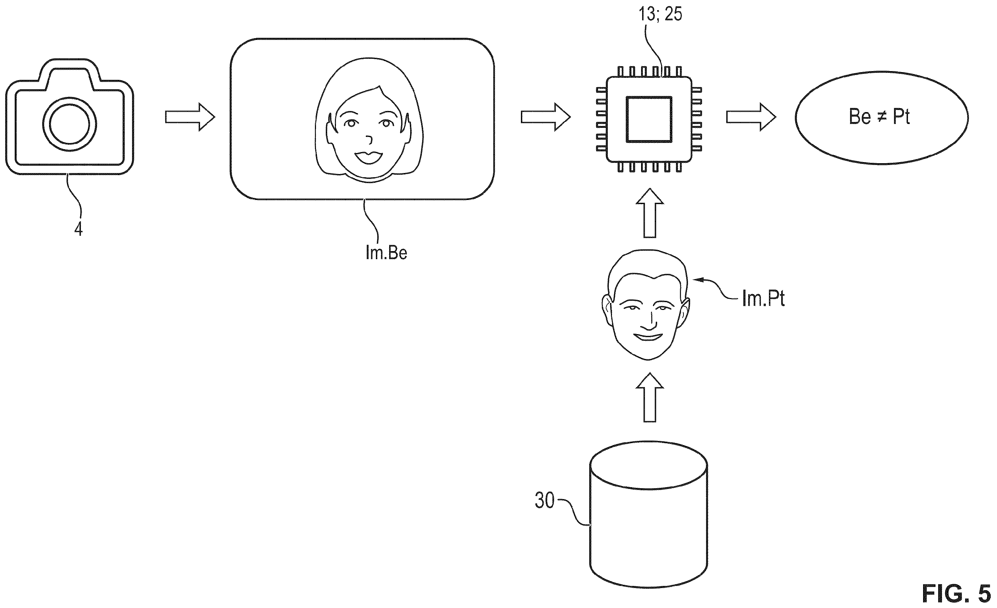

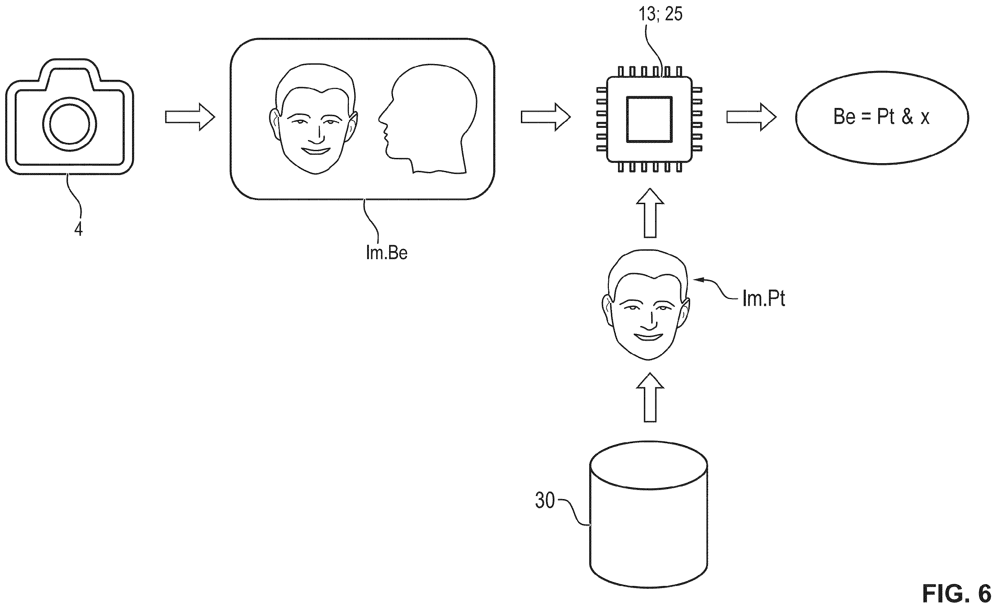

Im Beispiel von Figur 4 entscheidet die Bildvergleichseinheit 25, dass der Patient Pt selbst eine Antwort auf eine Frage in die Erfassungs-Anordnung 100 eingegeben hat (Ergebnis Be = Pt). Im Beispiel von Figur 5 entscheidet die Bildvergleichseinheit 25, dass eine andere Person eine Antwort eingegeben hat (Ergebnis Be ≠ Pt). Im Beispiel von Figur 6 entscheidet die Bildvergleichseinheit 25, dass eine andere Person x dem Patienten Pt bei der Beantwortung der Frage geholfen hat (Ergebnis Be = Pt & x).

Ein weiterer Eingabe-Parameter wird mithilfe des Bewegungssensors 18 gemessen. Der Bewegungssensor 18 vermag die Bewegung des Smartphones 40 im Raum zu messen, bevorzugt mit einer vorgegebenen Abtastrate. Bevorzugt misst der Bewegungssensor 18 die drei linearen Beschleunigungen und die drei Winkelbeschleunigungen in einem vorgegebenen dreidimensionalen Koordinatensystem. Der Bewegungssensor 18 liefert als ein Ergebnis eine Trajektorie des Smartphones 40 im dreidimensionalen Koordinatensystem. Weiter oben wurde beschrieben, dass für jede Frage der Fragen-Gruppe jeweils ein Beantwortungs-Zeitraum detektiert wird. Für jeden Beantwortungs-Zeitraum umfasst die Trajektorie jeweils ein Segment, nämlich dasjenige Segment der Trajektorie, entlang der das Smartphone in den Beantwortungs-Zeitraum bewegt worden ist. Möglich ist natürlich, dass das Smartphone 40 in mindestens einem Beantwortungs-Zeitraum überhaupt nicht bewegt worden ist und daher das Segment ein Punkt ist. Das Segment oder mindestens eine Eigenschaft, beispielsweise die Länge oder die räumliche Ausdehnung, des Segments ist der gemessene Wert, den der entsprechende Eingabe-Parameter für eine Frage annimmt. Mithilfe des Bewegungssensors 18 lassen sich insbesondere folgende beiden Situationen detektieren:
- Der Benutzer Be oder wenigstens eine Hand des Benutzers Be, die das Smartphone 40 hält, hat bei der Beantwortung einer Frage gezittert.
- Der Benutzer Be hat das Smartphone 40 im Beantwortungs-Zeitraum erheblich bewegt. Dies kann ein Indiz dafür sein, dass ein von der Kamera 4 aufgenommenes Bild Im.Be jemanden anders zeigt als die Person, die tatsächlich eine Antwort auf die Frage eingegeben hat.
- Das Smartphone 40 ist dem Benutzer Be aus der Hand gefallen.

Im Ausführungsbeispiel sind mehrere messbare Eingabe-Parameter vorgegeben. Jeder vorgegebene Eingabe-Parameter nimmt für jede Frage der Fragen-Gruppe jeweils einen Wert an oder kann wenigstens einen Wert annehmen. Dieser Wert kennzeichnet das Verhalten des Benutzers Be bei der Beantwortung (oder Nicht-Beantwortung) dieser Frage. Für zwei verschiedene Fragen kann der Eingabe-Parameter zwei unterschiedliche Werte annehmen.

Die Erfassungs-Anordnung 100 umfasst mindestens einen Eingabe-Parameter-Sensor. Jeder Eingabe-Parameter-Sensor ist jeweils mindestens einem Eingabe-Parameter zugeordnet. Der Eingabe-Parameter-Sensor vermag für jede Frage jeweils zu messen, welchen Wert der zugeordnete Eingabe-Parameter während des oben beschriebenen Beantwortungs-Zeitraums für diese Frage annimmt. Bevorzugt umfasst die Erfassungs-Anordnung 100 für jeden vorgegebenen Eingabe-Parameter jeweils einen Eingabe-Parameter-Sensor. Möglich ist, dass ein Eingabe-Parameter-Sensor mindestens zwei verschiedene Eingabe-Parameter zu messen vermag. Möglich ist umgekehrt, dass zwei verschiedene Eingabe-Parameter-Sensoren verwendet werden, um denselben Eingabe-Parameter zu messen. Beispielhafte Eingabe-Parameter-Sensoren wurden weiter oben bereits beschrieben.

Außerdem ist im Ausführungsbeispiel mindestens ein Hervorhebungs-Kriterium vorgegeben. Das oder jedes Hervorhebungs-Kriterium spezifiziert jeweils ein Hervorhebungs-Element als Funktion mindestens eines verwendeten Eingabe-Parameter. Jedes Hervorhebungs-Kriterium liefert also als Funktion der gemessenen Werte der Eingabe-Parameter jeweils ein Hervorhebungs-Element.

Wie bereits dargelegt, erzeugt der Protokoll-Generierer 3 die Protokolldaten 20, und der Darstellungs-Generierer 14 erzeugt mindestens eine Darstellungsdatei 21.1, 21.2. Hierfür verwendet der Darstellungs-Generierer 14 die Protokolldaten 20. Falls auf Basis dieser Darstellungsdatei 21.1, 21.2 eine Darstellung Da visuell ausgegeben wird, so stellt im Ausführungsbeispiel diese Darstellung Da eine Kennung des Patienten Pt, auf den sich der Fragebogen Qu bezieht, sowie für jede Frage des Fragebogens Qu mindestens folgendes dar:
- die Frage und
- die erfasste Antwort auf diese Frage oder eine Kennzeichnung, dass diese Frage nicht beantwortet wurde.

Für jede Frage der Fragen-Gruppe stellt die Darstellung Da im Ausführungsbeispiel weiterhin folgendes dar: Falls der Eingabe-Parameter-Wert oder die Kombination von Eingabe-Parameter-Werten, die bei der Beantwortung der Frage gemessen worden sind, ein vorgegebenes Hervorhebungs-Kriterium erfüllen, so wird die Antwort zusammen mit dem Hervorhebungs-Element dieses Hervorhebungs-Kriteriums dargestellt.

Beispielhaft werden nachfolgend einige mögliche Hervorhebungs-Elemente beschrieben:
- Ein Hervorhebungs-Element für eine Frage kennzeichnet die erfasste Antwort auf diese Frage, hebt insbesondere die Antwort in der Darstellung Da hervor, beispielsweise durch eine andere Form der Zeichen (z.B. andere Schriftgröße, anderer Font, Fettdruck) oder eine Unterstreichung oder einen besonderen farblichen Hintergrund oder ein Symbol.
- Ein Hervorhebungs-Element für eine Frage umfasst ein Benutzerbild Im.Be desjenigen Benutzers Be, der die Antwort auf diese Frage eingegeben hat, wobei das Bildaufnahmegerät 4 dieses Bild Im.Be während des oben beschriebenen Beantwortungs-Zeitraums erzeugt hat.
- Ein Hervorhebungs-Element für eine Frage umfasst eine Kennzeichnung, ob der Patient Pt selbst oder eine andere Person Be diese Frage beantwortet hat.
- Ein Hervorhebungs-Element für eine Frage umfasst eine Kennzeichnung, ob eine weitere Person dem Benutzer Be bei der Beantwortung der Frage geholfen hat.
- Ein Hervorhebungs-Element kennzeichnet einen gemessenen Wert eines sonstigen Eingabe-Parameters, wobei dieser Wert während oder nach der Beantwortung dieser Frage gemessen worden ist. Beispielsweise kennzeichnet das Hervorhebungs-Element eine Frage und / oder eine Antwort, wenn der gemessene Eingabe-Parameter-Wert außerhalb eines Soll-Wertebereichs liegt, wobei dieser Soll-Wertebereich für diese Frage und für diesen Eingabe-Parameter vorgegeben ist. Das Hervorhebungs-Element umfasst beispielsweise ein Symbol, insbesondere ein Ausrufezeichen.

Einige Eingabe-Parameter wurden bereits weiter oben beschrieben. Zu diesen bereits beschriebenen Eingabe-Parametern zählen die folgenden Messgrößen, die sich auf eine Frage des Fragebogens Qu beziehen:
- Wurde die Frage beantwortet oder nicht?
- Hat der Patient Pt selbst oder eine andere Person diese Frage beantwortet? Dies ist der Fall, wenn der Bildvergleich das Ergebnis liefert, dass das Benutzerbild Im.Be eine andere Person zeigt als den Patienten Pt, der im vorgegebenen Referenzbild Im.Pt gezeigt wird.
- Hat eine weitere Person dem Benutzer Be bei der Beantwortung der Frage geholfen? Dies ist der Fall, wenn in dem gerade beschriebenen Benutzerbild Im.Be zusätzlich zum Benutzer Be eine weitere Person sichtbar ist und / oder wenn die Audiosignal-Auswerteeinheit eine sprachliche Hilfe oder Anweisung bei der Beantwortung einer Frage detektiert hat.
- Wie viele Rückfragen hat der Benutzer Be zu der Frage gestellt?
- Wie viele Rückfragen stimmen inhaltlich überein?
- Wie viele Rückfragen haben nichts mit der Frage zu tun?
- Welchen Wert nahm der oder ein Vitalparameter bei der Beantwortung der Frage ein? Der Vitalparameter ist insbesondere der Blutdruck oder der Puls.

Nachfolgend werden weitere messbare Eingabe-Parameter beschrieben. Auch diese weiteren Eingabe-Parameter sind Messgrößen, die sich auf eine Frage der Fragen-Gruppe beziehen. Dies sind insbesondere einige der folgenden Messgrößen:
- Wie lange ist ein Beantwortungs-Zeitraum, wobei dieser Zeitraum mit der Ausgabe der Frage beginnt und dann endet, wenn der Benutzer Be bestätigt hat, die Antwort auf die Frage vollständig eingegeben zu haben, oder wenn feststeht, dass die Frage nicht beantwortet wurde? In einer Ausgestaltung wird für jede Frage zusätzlich eine Soll-Zeitdauer vorgegeben, und die Erfassungs-Auswerteeinheit 13 prüft, ob die gemessene Dauer des Beantwortungs-Zeitraums in dieser Soll-Zeitdauer liegt oder nicht. Sowohl eine sehr lange als auch eine sehr schnelle Beantwortung der Frage können ein Indiz für eine unzutreffend oder durch Raten beantwortete Frage sein.
- Mit welchem Bewegungsmuster, mit welcher Mimik und Gestik und mit welchem Gesichtsausdruck hat der Benutzer Be die Frage beantwortet? Wie stark hat der Benutzer Be den Kopf bewegt, während er die Antwort auf die Frage eingegeben hat? Hierfür verwendet die Bildauswerteeinheit mindestens ein Benutzerbild, welches das Bildaufnahmegerät 4 erzeugt hat, während der Benutzer Be die Antwort auf die Frage eingegeben hat.
- Mit wie vielen Verzögerungen und Stockungen hat der Benutzer Be seine Antwort auf die Frage eingegeben? Dies gilt sowohl für eine Eingabe per Tastatur 2 als auf einer Eingabe per Spracheingabeeinheit 5.
- Falls der Benutzer Be sich eine Frage visuell auf der Anzeigefläche 1 ausgeben lässt: Welche Schriftgröße oder Zeichengröße oder Hintergrundfarbe oder Farbe des ausgegebenen Textes hat der Benutzer mithilfe des Betätigungselements 11 eingestellt?
- Falls der Benutzer Be die Tastatur 2 verwendet, um eine Antwort einzugeben: Mit welcher Stärke und / oder mit welcher Treffsicherheit und mit welcher Eingabegeschwindigkeit hat der Benutzer die Tastatur 2 betätigt?
- Falls der Benutzer Be die Sprachausgabeeinheit 6 verwendet, um sich eine Frage sprachlich ausgeben zu lassen: Welche Lautstärke hat der Benutzer Be für die Ausgabe dieser Frage mithilfe des Betätigungselements 11 eingestellt?
- Falls der Benutzer die Spracheingabeeinheit 5 verwendet, um eine Antwort einzugeben: Mit welcher Lautstärke und mit welcher Sprechgeschwindigkeit hat der Benutzer Be die Antwort auf die Frage eingegeben? Wie stark schwankte die Lautstärke bei der Eingabe?
- Wie sicher vermag die Spracherkennungseinheit aus der Spracheingabe eine Zeichenfolge zu erzeugen? Mit anderen Worten: Wie verständlich war die Spracheingabe des Benutzers Be?
- Wie viele Änderungen, insbesondere Korrekturen, hat der Benutzer Be bei der Eingabe der Antwort vorgenommen? Auch dies gilt sowohl bei einer Eingabe mittels der Tastatur 2 als auf einer Spracheingabe in das Mikrophon 5.

Wie bereits dargelegt, erzeugt der Protokoll-Generierer 3 in der oben beschriebenen ersten Phase die Protokolldaten 20. Der Darstellungs-Generierer 14 erzeugt aus den Protokolldaten 20 mindestens eine Darstellungsdatei 21.1, 21.2. Die oder jede Darstellungsdatei 21.1, 21.2 und damit eine Darstellung Da, die auf Basis der Darstellungsdatei 21.1, 21.2 ausgegeben wird, umfasst im Ausführungsbeispiel jede Frage des Fragebogens Qu und für jede Frage jeweils die Antwort oder die Aussage, dass keine Antwort erfasst wurde. Im Ausführungsbeispiel umfasst die oder jede Darstellungsdatei 21.1, 21.2 weiterhin den oder jeden gemessenen Eingabe-Parameter-Wert zusammen mit einer Kennzeichnung derjenigen Frage, auf den sich dieser Eingabe-Parameter-Wert bezieht.

Die Erfassungs-Auswerteeinheit 13 wendet auf die Eingabe-Parameter-Werte, die in den Protokolldaten 20 enthalten sind, mindestens eine Auswerteregel an. Die oder jede Auswerteregel ist in einer rechnerauswertbaren Form vorgegeben und bezieht sich auf jeweils mindestens einen Eingabe-Parameter. Möglich ist, dass eine Auswerteregel sich auf eine Kombination von mindestens zwei Eingabe-Parametern bezieht. Die Anwendung einer Auswerteregel liefert ein Auswertungsergebnis. Durch die Anwendung erzeugt die Erfassungs-Auswerteeinheit 13 Auswertungsdaten 22. Im Folgenden werden beispielhaft Auswerteregeln und Auswertungsdaten 22 beschrieben. Der Darstellungs-Generierer 14 verwendet die Protokolldaten 20 und die Auswertungsdaten 22, um die oder jede Darstellungsdatei 21.1, 21.2 zu erzeugen.

Weiter oben wurde mit Bezug auf Figur 4 bis Figur 6 eine Ausgestaltung beschrieben, bei der eine Bildvergleichseinheit 25 prüft, ob der Patient Pt selbst oder eine andere Person Be eine Antwort auf eine Frage in die Erfassungs-Anordnung 100 eingegeben hat. In einer Ausgestaltung umfassen die Auswertungsdaten 22 für jede Frage, die nicht vom Patienten Pt selbst beantwortet worden ist, eine Kennzeichnung und optional ein Bild derjenigen Person Be, die tatsächlich diese Frage beantwortet hat. Optional umfasst die oder mindestens eine Darstellungsdatei 21.1, 21.2 für jede Frage, die nicht vom Patienten Pt selbst beantwortet wurde, ein Bild derjenigen Person Be, die diese Frage beantwortet hat. Dieses Bild wurde von der Kamera 4 erzeugt und wird in Figur 4 bis Figur 6 mit Im.Be bezeichnet.

Falls für eine Frage das oder ein Hervorhebungs-Kriterium erfüllt ist, umfassen die Auswertungsdaten 22 und damit die oder mindestens eine Darstellungsdatei 21.1, 21.2 und die Darstellung Da zusätzlich das vom Hervorhebungs-Kriterium spezifizierte Hervorhebungs-Element.

Nachfolgend wird beispielhaft beschrieben, wie die Hervorhebungs-Elemente erzeugt werden. In einer Realisierungsform werden mindestens zwei verschiedene mögliche Einstufungen vorgegeben. Jede Einstufung legt ein Maß dafür fest, wie groß die Sicherheit ist, dass die Antwort zutreffend ist. Einerseits wird vorab jeder möglichen Einstufung jeweils ein Hervorhebungs-Element zugeordnet. Beispielsweise wird der Einstufung, dass die Antwort mit hoher Sicherheit zutreffend ist, ein Standard-Hervorhebungs-Element zugeordnet, welches dazu führt, dass die Antwort in der Darstellung Da nicht hervorgehoben dargestellt wird. Der oder jeder anderen Einstufung wird jeweils ein abweichendes Hervorhebungs-Element zugeordnet, welches dazu führt, dass in der erzeugten Darstellungsdatei 21.1 und in der ausgegebenen Darstellung Da die Antwort auf die Frage hervorgehoben gekennzeichnet wird. Möglich ist, dass mindestens zwei verschiedene Einstufungen, die von der Einstufung "mit hoher Sicherheit zutreffend" abweichen, vorgegeben werden und daher auch mindestens zwei verschiedenen Hervorhebungs-Elemente, die vom Standard-Hervorhebungs-Element abweichen, verwendet werden.

Jedes vorgegebene Hervorhebungs-Kriterium spezifiziert jeweils eine Einstufung und damit ein Hervorhebungs-Element als Funktion mindestens eines Eingabe-Parameters, optional mehrerer Eingabe-Parameter. Wie oben dargelegt, misst die Erfassungs-Anordnung 100, welchen Wert die tatsächlich verwendeten Eingabe-Parameter bei der Eingabe einer Antwort auf eine Frage tatsächlich annehmen. Idealerweise liegt für jede Antwort auf eine Frage der Fragen-Gruppe und für jeden verwendeten Eingabe-Parameter jeweils ein Wert vor. Die Erfassungs-Auswerteeinheit 13 wendet für jede erfasste Antwort das oder jedes Hervorhebungs-Kriterium auf die gemessenen Eingabe-Parameter-Werte an. Die Anwendung liefert eine Einstufung und damit ein Hervorhebungs-Element.

Die Hervorhebungs-Kriterien werden vorab aufgestellt. In einer Realisierungsform hat das oder jedes Hervorhebungs-Kriterium die Form einer Regel. Diese Regel spezifiziert eine Einstufung und damit ein Hervorhebungs-Element als Funktion eines Wertebereichs eines Eingabe-Parameters oder einer Kombination von mehreren Wertebereichen mehrerer Eingabe-Parameter. Ein Wertebereich kann auch nur aus einem einzigen möglichen Wert bestehen. In einer anderen Realisierungsform wird ein lernendes Verfahren auf eine vorgegebene Stichprobe angewendet. Die Stichprobe umfasst mehrere Stichprobenelemente. Jedes Stichprobenelement besteht einerseits aus mindestens einem Eingabe-Parameter-Wert, optional mehreren Eingabe-Parameter-Werten, und andererseits einer Einstufung.

In einer Ausgestaltung ist für mindestens einen vorgegebenen Eingabe-Parameter ein Soll-Wertebereich vorgegeben. Beispielsweise sind für jede Frage eine Mindest-Zeitdauer und eine Höchst-Zeitdauer für die Beantwortung dieser Frage, also für den oben beschriebenen Beantwortungs-Zeitraum, vorgegeben. Oder ein Soll-Wertebereich für die Lautstärke der Spracheingabeeinheit 5 oder für die Schriftgröße der Anzeigefläche 1 oder für einen Vitalparameter sind vorgegeben. Der Soll-Wertebereich kann für denselben Eingabe-Parameter von Frage zu Frage differieren. Beispielsweise kann die jeweilige Höchst-Zeitdauer des Beantwortungs-Zeitraums für verschiedene Fragen unterschiedlich sein. Möglich ist auch, dass der Soll-Wertebereich individuell und abhängig von einer Eigenschaft des Patienten Pt, auf den sich die Fragen beziehen, generiert wird, beispielsweise abhängig von dessen Alter oder von einer bekannten Krankheitsvorgeschichte.

Die Erfassungs-Auswerteeinheit 13 entscheidet für diesen Eingabe-Parameter und für jede Frage, ob der gemessene Wert des Eingabe-Parameter bei der Beantwortung dieser Frage in den Soll-Wertebereich fällt oder nicht. Mindestens dann, wenn für eine Frage der gemessene Wert außerhalb des Soll-Wertebereichs fällt, umfassen die Auswertungsdaten 22 eine Kennzeichnung der Frage und eine Kennzeichnung des Eingabe-Parameter-Werts, der außerhalb des vorgegebenen Soll-Wertebereichs liegt. Der Darstellungs-Generierer 14 erzeugt die Darstellungsdatei 21.1, 21.2 wie folgt: Zusätzlich zur erfassten Antwort und zum optionalen Hervorhebungs-Kriterium umfasst die Darstellungsdatei 21.1, 21.2 eine Kennzeichnung des Eingabe-Parameters und eine Kennzeichnung des Werts, wenigstens aber eine Kennzeichnung, dass der Wert außerhalb des Soll-Wertebereichs liegt. Diese Ausgestaltung erleichtert es einem Menschen noch mehr, Auffälligkeiten bei der Beantwortung des Fragebogens Qu zu detektieren.

Bislang wurde beschrieben, wie die Erfassungs-Anordnung 100 das Verhalten eines Benutzers bei der Beantwortung des Fragebogens Qu misst und bewertet.

In einer Ausgestaltung bewertet die Erfassungs-Auswerteeinheit 13 zusätzlich automatisch den Inhalt der Antworten. Dies wird nachfolgend näher beschrieben.

In einer Ausgestaltung ist mindestens eine Frage des Fragebogens Qu so formuliert, dass ein Benutzer Be die Frage mit Freitext beantwortet oder beantworten kann, beispielsweise dann, wenn vorgegebene Antwortmöglichkeiten nicht zutreffen.

In einer Ausgestaltung umfasst die Erfassungs-Auswerteeinheit 13 einen Textanalysierer. Der Textanalysierer analysiert einen Freitext, der als Antwort auf eine Frage eingegeben wurde. Bevorzugt ist in diesem Falle als Soll-Wertebereich vorgegeben, dass der Freitext einen Zustand eines gesunden Menschen beschreibt. Beispielsweise ist vorgegeben, dass ein gesunder Mensch m Treppenstufen binnen n Minuten hinaufsteigen kann, ohne außer Atem zu gelangen. Folgendes sind Beispiele für einen Wert, der außerhalb des Soll-Wertebereichs liegt:
- Der tatsächlich eingegebene Freitext beschreibt einen Zustand eines nicht vollständig gesunden Menschen. Beispielsweise beschreibt im obigen Beispiel mit dem Treppensteigen der Freitext, dass der Patient Pt mehr als n Minuten benötigt hat oder außer Atem geraten ist.
- Der tatsächlich eingegebene Freitext hat nichts mit der Frage zu tun, ist also eine invalide Antwort auf die Frage. Ein Extremfall: Gefragt wird, welche Medikamente der Patient Pt einnimmt, und die Antwort lautet: Heute ist schönes Wetter.

Möglich ist, dass die beiden Antworten auf zwei verschiedene Fragen des Fragebogens Qu nicht zusammenpassen oder wenigstens selten tatsächlich auf dieselbe Person gleichzeitig zutreffen oder aber ein Indiz für eine besondere Situation sind. Beispielsweise kommt es sehr selten vor, dass eine Person 2 m groß ist, aber weniger als 30 kg wiegt. Oder ein Patient Pt gibt an, mindestens ein rezeptpflichtiges Medikament einzunehmen, war aber gemäß seiner Antwort in den letzten Jahren niemals beim Arzt. Oder eine Frage bezieht sich auf den das Geburtsdatum und eine andere Frage auf das aktuelle Lebensalter des Patienten Pt, und die beiden Antworten passen nicht zusammen.

Falls für denselben Patienten Pt zwei derartige in der Regel einander widersprechende Antworten vorliegen, so ist dies an die Indiz dafür, dass mindestens eine Antwort falsch beantwortet wurde, was versehentlich oder vorsätzlich oder aufgrund einer Beeinträchtigung geschehen sein kann. Die nachfolgende Ausgestaltung erleichtert es einem Arzt Me, der die ausgegebene Darstellung Da analysiert, derartige einander in der Regel widersprechende Antworten herauszufinden.

Gemäß dieser Ausgestaltung ist in einer rechnerauswertbaren Form mindestens eine Inkonsistenz-Regel vorgegeben. In einer Realisierungsform bezieht sich die oder jede Inkonsistenz-Regel auf eine Fragen-Kombination, wobei die Fragen-Kombination mindestens zwei Fragen des Fragebogens Qu umfasst. Beispielsweise gibt es für jede Frage der Fragen-Kombination eine vorgegebene Menge von möglichen Antworten, und der Benutzer Be wählt aus dieser Menge eine der möglichen Antworten aus, um die Frage zu beantworten. Die möglichen Antworten auf die Fragen der Fragen-Kombination spannen einen Merkmalsraum auf. Die oder jede Inkonsistenz-Regel spezifiziert die Fragen der Fragen-Kombination sowie einen Soll-Wertebereich im Merkmalsraum der möglichen Antworten auf die Fragen der Fragen-Kombination. Die Fragen der Fragen-Kombination wurden inkonsistent beantwortet, wenn die Antworten im Merkmalsraum und dort außerhalb dieses Soll-Wertebereichs liegen.

Die Erfassungs-Auswerteeinheit 13 wendet automatisch die Inkonsistenz-Regeln auf die erfassten Antworten an. Diese erfassten Antworten sind in einer rechnerauswertbaren Form in den Protokolldaten 20 enthalten. Durch diese Anwendung detektiert die Erfassungs-Auswerteeinheit 13 jedes Inkonsistenz-Paar oder stellt fest, dass es kein Inkonsistenz-Paar gibt. Ein Inkonsistenz-Paar umfasst die Antworten, mit denen die Fragen einer Fragen-Kombination einer Inkonsistenz-Regel beantwortet worden sind, wobei diese Antworten außerhalb des oben beschriebenen Soll-Wertebereichs liegen. Die Erfassungs-Auswerteeinheit 13 erzeugt die Auswertungsdaten 22 so, dass die Auswertungsdaten jeweils eine Kennzeichnung jedes detektierten Inkonsistenz-Paars umfassen. Der Darstellungs-Generierer 14 verwendet die Kennzeichnungen der detektierten Inkonsistenz-Paare und generiert die Darstellungsdatei 21.1, 21.2 wie folgt: Die Darstellungsdatei 21.1, 21.2 umfasst zusätzlich zu den Antworten jeweils eine Kennzeichnung jedes detektierten Inkonsistenz-Paars. Die Darstellung Da, die auf Basis der Darstellungsdatei 21.1, 21.2 ausgegeben wird, umfasst eine Kennzeichnung des oder jedes detektierten Inkonsistenz-Paars. Beispielsweise werden die mindestens zwei zueinander inkonsistenten Antworten mit dem gleichen Symbol hervorgehoben gekennzeichnet.

In einer Ausgestaltung ist in einer rechnerausführbaren Form mindestens ein Test vorgegeben, bevorzugt ein psychometrischer Test. Ein Beispiel für einen solchen Test ist der sogenannte Uhrentest, bei dem ein Benutzer gebeten wird, eine Uhr zu zeichnen und / oder in eine Uhr eine vorgegebene Uhrzeit (Stellung der beiden Zeiger der Uhr) einzutragen. Beispielsweise wird der Benutzer gebeten, mithilfe des Stifts 12 auf dem Bildschirm 1 eine Uhr zu zeichnen oder in eine dargestellte Uhr die beiden Zeigerstellungen für eine vorgegebene Uhrzeit einzutragen. Falls die Reaktion des Benutzers auf die Bitte, eine Uhr zu zeichnen, deutlich von einer realen Uhr abweicht oder die gezeichneten Zeigerstellungen von den korrekten Zeigerstellungen erheblich abweichen, so ist dies ein Indiz für eine Demenz. Gemäß der Ausgestaltung vermag die Ausgabeeinheit 1, 6 den Test auszugeben. Die Eingabeeinheit 2, 5, 12 vermag die Reaktion des Benutzers Be auf diesen Test zu erfassen.

Bevorzugt löst die Erfassungs-Auswerteeinheit 13 mindestens dann automatisch den Schritt aus, einen vorgegebenen Test auf der Ausgabeeinheit 1, 6 auszugeben und eine Reaktion des Benutzers zu erfassen, wenn mindestens eine der folgenden Ereignisse detektiert worden sind:
- Ein gemessener Wert eines Eingabe-Parameters liegt außerhalb des Soll-Wertebereichs für diesen Eingabe-Parameter und optional für diese Frage.
- Mindestens einer Frage wurde von einem anderen Benutzer Be als von dem Patienten Pt beantwortet.
- Eine eingegebene Antwort auf eine Frage hat nichts mit der Frage zu tun, insbesondere eine als Freitext eingegebene Antwort.
- Ein Inkonsistenz-Paar wurde detektiert, also zwei Antworten auf zwei Fragen des Fragebogens Qu, wobei die Antworten zueinander inkonsistent sind.

Möglich ist, dass es vom Inkonsistenz-Paar und / oder von dem Eingabe-Parameter-Wert außerhalb des Soll-Wertebereichs abhängt, welcher Test auf der Ausgabeeinheit 1, 6 ausgegeben wird.

In einer Ausgestaltung umfassen die Protokolldaten 20 und bevorzugt auch die Darstellungsdatei 21.1, welche der Darstellungs-Generierer 14 erzeugt hat, eine rechnerauswertbare Repräsentation des Testergebnisses, insbesondere eine Bilddatei. Die Repräsentation des Testergebnisses zeigt, welche Reaktion des Benutzers Be die Eingabeeinheit 2, 5, 12 als Reaktion darauf, dass der Test auf der Ausgabeeinheit 1, 6 ausgegeben wurde, erfasst hat. Eine Darstellung Da, die auf Grundlage der erzeugten Darstellungsdatei 21.1 visuell ausgegeben wird, umfasst eine visuelle Darstellung dieser Repräsentation des Testergebnisses.

In einer Ausgestaltung fasst die Erfassungs-Auswerteeinheit 13 mehrere gemessene Werte eines Eingabe-Parameters zu einer Einzelbewertung für diesen Eingabe-Parameter zusammen. Die Erfassungs-Auswerteeinheit 13 fasst die Einzelbewertungen für verschiedene Eingabe-Parameter zu einer Gesamtbewertung zusammen. Die Gesamtbewertung kennzeichnet das Verhalten des Patienten Pt. Die gemessenen Werte beziehen sich auf unterschiedliche Fragen. Die oder mindestens eine Auswerteregel bezieht sich auf diesen Eingabe-Parameter und umfasst eine Aggregierungsvorschrift. Die Aggregierungsvorschrift fasst mehrere Werte dieses Eingabe-Parameters zur Einzelbewertung zusammen. Die Werte beziehen sich auf unterschiedliche Fragen der Fragen-Gruppe. In einer Ausgestaltung spezifiziert die Aggregierungsvorschrift ein gewichtetes Mittel der Eingabe-Parameter-Werte. Für diesen Eingabe-Parameter und für jede Frage der Fragen-Gruppe ist jeweils ein Gewichtsfaktor vorgegeben, und diese vorgegebenen Gewichtsfaktoren treten in der Aggregierungsvorschrift auf.

Nachfolgend wird beispielhaft dargelegt, welche Eingabe-Parameter und sonstige Größen in die Gesamtbewertung des Verhaltens des Patienten Pt einfließen. In diesem Beispiel ist eine hohe Gesamtbewertung ein Anzeichen dafür, dass der Patient Pt möglicherweise an einer beginnenden Demenz oder an einer sonstigen körperlichen oder mentalen Einschränkung leidet. Folgende Eingabe-Parameter und sonstige Größen erhöhen die Gesamtbewertung:
- falls der Patient älter als 70 Jahre ist, jedes Lebensjahr oberhalb des vollendeten 70. Lebensjahres,
- wie oft eine eingegebene Antwort auf eine Frage nichts mit der Frage zu tun hat, also eine invalide Antwort auf die Frage ist,
- wie viele Inkonsistenz-Paare detektiert worden sind, wobei mehrere Inkonsistenz-Paare deutlich höher bewertet werden als ein einziges Inkonsistenz-Paar (man kann sich ja einmal vertun),
- wie viele Antworten auf eine Frage des Fragebogens Qu inhaltlich nichts mit der Frage zu tun haben,
- wie viele Rückfragen der Patient Pt insgesamt gestellt hat,
- wie viele Rückfragen zu einer Frage inhaltlich nichts mit dieser Frage zu tun haben,
- wie viele Fragen der Patient Pt nicht oder wenigstens nicht allein beantwortet hat,
- eine geringe Treffsicherheit des Patienten Pt bei der Benutzung der Tastatur 2,
- eine schwer verständliche Spracheingabe,
- mindestens ein Vitalparameter, der bei der Beantwortung der Fragen gemessen worden ist.

Wie bereits erwähnt, erzeugt die Erfassungs-Auswerteeinheit 13 Auswertungsdaten 22. Der Darstellungs-Generierer 14 erzeugt die oder mindestens eine Darstellungsdatei 21.1, 21.2. Die Auswertungsdaten 22 und daher die Darstellungsdatei 21.1, 21.2 umfassen die Gesamtbewertung. In der zweiten Stufe verwendet der Arzt Me die Darstellung Da, die auf Basis der Darstellungsdatei 21.1 erzeugt worden ist, vgl. Figur 3, um das Gespräch mit dem Patienten Pt zu führen.

Im Ausführungsbeispiel berechnet die Erfassungs-Auswerteeinheit 13 zusätzlich eine Vorhersage, wie lange das Gespräch voraussichtlich dauern wird, welches der Arzt Me in der zweiten Stufe mit dem Patienten Pt durchführt. Um diese Vorhersage zu treffen, verwendet die Erfassungs-Auswerteeinheit 13 einerseits eine vorgegebene Standarddauer für ein solches Gespräch. Die Erfassungs-Auswerteeinheit 13 vergrößert die erwartete Dauer des Gesprächs mit dem Patienten Pt, wenn der Patient Pt eine hohe Gesamtbewertung aufweist. Im Ausführungsbeispiel werden außerdem die Beantwortungs-Zeiträume, die bei der Beantwortung der Fragen der Fragen-Gruppe detektiert worden sind, verwendet, um die erwartete Dauer des Gesprächs mit dem Patienten Pt vorherzusagen. Wie oben dargelegt, ist ein Eingabe-Parameter die Länge des Beantwortungs-Zeitraums, wobei der Beantwortungs-Zeitraum der Zeitraum zwischen den beiden folgenden Ereignissen ist:
- Die Ausgabeeinheit 1, 6 hat eine Frage ausgegeben.
- Die Eingabeeinheit 2, 5, 12 hat den Vorgang detektiert, dass der Benutzer Be die Eingabe einer Antwort auf diese Frage abgeschlossen hat.

Aus diesen Beantwortungs-Zeiträumen wird eine Einzelbewertung für den Eingabe-Parameter Beantwortungs-Zeitraum hergeleitet. Die Einzelbewertung ist ein gewichtetes Mittel über die Beantwortungs-Zeiträume, die bei der Beantwortung der Fragen der Fragen-Gruppe detektiert worden sind. Diese Einzelbewertung wird im Ausführungsbeispiel verwendet, um die Zeitdauer für das Gespräch vorherzusagen.

Weiter oben wurde beschrieben, wie automatisch eine Gesamtbewertung des Patienten Pt hergeleitet wird. In einer Ausgestaltung hängt die Vorhersage, wie lange das Gespräch dauern wird, zusätzlich von dieser Gesamtbewertung ab.

Nachfolgend werden beispielhaft weitere Größen genannt, die in die Vorhersage einfließen oder einfließen können, wie lange das Gespräch mit dem Patienten Pt dauern wird.
- Ein Eingabe-Parameter beschreibt, ob der Patient Pt selbst und ohne fremde Hilfe oder ein anderer Benutzer eine Antwort auf eine Frage der Fragen-Gruppe eingegeben hat. In die vorhergesagte Dauer fließt folgende Größe ein: der prozentuale Anteil derjenigen Fragen, die der Patient Pt selbst ohne fremde Hilfe beantwortet hat, und / oder der Anteil derjenigen Fragen, die der Patient Pt nicht selbst und allein beantwortet hat, an der Anzahl der Fragen der Fragen-Gruppe.
- die Anzahl der Rückfragen,
- die Anzahl von inhaltlich gleichen Rückfragen, auch zu verschiedenen Fragen,
- die Anzahl der nicht sinnvollen Rückfragen,
- die Anzahl der nicht validen Antworten.

In der oben beschriebenen zweiten Phase verwendet ein Arzt Me eine Darstellung Da, um das Gespräch mit dem Patienten Pt zu führen, vgl. Figur 3. Diese Darstellung Da wurde auf Basis der Darstellungsdatei 21.1 erzeugt.

In einer Realisierungsform liegt dem Arzt Me die Darstellung Da in ausgedruckter Form vor. In einer anderen Realisierungsform verwendet der Arzt ein weiteres signalverarbeitendes Gerät, um sich die Darstellung Da ausgeben zu lassen. Das weitere signalverarbeitende Gerät bevorzugt ebenfalls ein tragbarer Rechner, beispielsweise das Tablet 200, vgl. Figur 3, und umfasst eine Ausgabefläche, auf der die Darstellung Da ausgegeben wird. Nachfolgend wird das weitere Gerät als "Ausgabe-Anordnung" bezeichnet.

In einer Ausgestaltung kann sich der behandelnde Arzt Me auf der Ausgabefläche der Ausgabe-Anordnung 200 eine Begründung dafür anzeigen lassen, warum eine Frage der Fragen-Gruppe und / oder die Antwort auf diese Frage mit einem Hervorhebungs-Element versehen und dadurch hervorgehoben ist. Der behandelnde Arzt Me wählt die Frage aus. Als Reaktion darauf, dass eine Frage ausgewählt ist, gibt die Ausgabe-Anordnung 200 zusätzlich Folgendes aus: den oder jeden Eingabe-Parameter und den gemessenen Wert, wobei die Eingabe-Parameter-Werte in der ersten Phase gemessen worden sind und in der zweiten Phase ausgegeben werden und dazu geführt haben, dass das jeweilige Hervorhebungs-Kriterium erfüllt ist. Insbesondere werden jeder Eingabe-Parameter und sein gemessener Wert ausgegeben, wenn der Eingabe-Parameter-Wert außerhalb des für diesen Eingabe-Parameter und für diese Frage vorgegebenen Soll-Wertebereich liegt. Beispielhaft wird in Figur 3 eine entsprechende Zusatzinformation ZI angedeutet, nämlich dass die Beantwortung der Frage Qu2? 5 min gedauert hat.

In einer Ausgestaltung erzeugt die Ausgabe-Anordnung 200 eine rechnerauswertbare Kopie des Fragebogens Qu. Diese Kopie umfasst die Fragen des Fragebogens Qu und die Antworten, die der Benutzer Be der Erfassungs-Anordnung 100 in der ersten Phase eingegeben hat, aber nicht notwendigerweise die Hervorhebungs-Elemente oder die gemessenen Eingabe-Parameter-Werte. Der fragende Arzt Me kann in der Kopie die Antworten des Patienten Pt durch eigene Eingaben überschreiben und dadurch insbesondere unzutreffende Antworten des Benutzers Be korrigieren. Hierfür verwendet der Arzt die Ausgabe-Anordnung 200. Diese Ausgestaltung erspart es dem fragenden Arzt Me, selbst erneut alle Antworten auf die Fragen des Fragebogens Qu einzugeben, auch solche Fragen, die dem behandelnden Arzt Me als richtig beantwortet erscheinen.

### Bezugszeichenliste

| | |
|---|---|
| 1 | berührungssensitiver Bildschirm, gehört zur visuellen Ausgabeeinheit der Erfassungs-Anordnung 100 |
| 2 | Bildschirm-Tastatur, gehört zur Eingabeeinheit der Erfassungs-Anordnung 100 |
| 3 | Protokoll-Generierer, erzeugt die Protokolldaten 20, ist ein Programm, das auf dem Prozessor der Erfassungs-Anordnung 100 abläuft |
| 4 | Kamera der Erfassungs-Anordnung 100, fungiert als das Bildaufnahmegerät und als ein Eingabe-Parameter-Sensor |
| 5 | Mikrophon des Headsets 10, fungiert als eine Spracheingabeeinheit |
| 6 | Lautsprecher des Headsets 10, fungieren als Sprachausgabeeinheit, vermögen die Fragen des Fragebogens Qu akustisch auszugeben |
| 7 | Mikrophon der Erfassungs-Anordnung 100, gehört zur Audiosignal-Erfassungseinheit |
| 8 | Stoppuhr der Erfassungs-Anordnung 100, gehört zu einem Eingabe-Parameter-Sensor, misst die Zeitdauer zur Beantwortung einer Frage |
| 9 | Kommunikationseinheit der Erfassungs-Anordnung 100, übermittelt die erzeugten Protokolldaten 20 an einen Zentralrechner, der Schreibzugriff auf die Datenbank 30 hat |
| 10 | Headset, umfasst das Mikrophon 5 und die beiden Lautsprecher 6, gehört zum Smartphone 40 |
| 11 | Betätigungselement, ermöglicht es einem Benutzer, Einstellungen des Smartphones 40 und damit der Erfassungs-Anordnung 100 zu ändern |
| 12 | Bildschirmstift, ermöglicht es einem Benutzer, Eingaben auf dem Bildschirm 1 vorzunehmen |
| 13 | Erfassungs-Auswerteeinheit, erzeugt durch Auswertung der Protokolldaten 20 die Auswertungsdaten 22 |
| 14 | Darstellungs-Generierer, erzeugt unter Verwendung der Protokolldaten 20 und der Auswertungsdaten 22 mindestens eine Darstellungsdatei 21.1, 21.2 |
| 15 | Vitalparameter-Sensor, misst die Herzschlagfrequenz des Benutzers Be, ist ein Eingabe-Parameter-Sensor |
| 16 | Steuergerät des Smartphones 40, steuert die Kamera 4 an |
| 18 | Bewegungssensor, misst die Bewegung des Smartphones 40 im Raum, umfasst bevorzugt einen Beschleunigungssensor |
| 20 | Protokolldaten, umfassen die erfassten Antworten auf die Fragen des Fragebogens Qu und die gemessenen Eingabe-Parameter-Werte, werden vom Protokoll-Generierer 3 erzeugt, werden in Datenspeicher 30 abgespeichert, |
| 21.1, 21.2 | Darstellungsdatei, vom Darstellungs-Generierer 14 unter Verwendung der Protokolldaten 20 und der Auswertungsdaten 22 erzeugt, lässt sich visuell als Darstellung Da ausgeben |
| 22 | Auswertungsdaten, von der Erfassungs-Auswerteeinheit 13 durch Auswertung der Protokolldaten 20 erzeugt |
| 25 | Bildvergleichseinheit, vergleicht ein Bild Im.Be, das die Kamera 4 von einem Benutzer Be der Erfassungs-Anordnung 100 erzeugt hat, mit einem vorgegebenen Referenzbild Im.Pt des Patienten Pt, gehört zur Erfassungs-Auswerteeinheit |
| 30 | Datenbank, in der das Referenzbild Im.Pt abgespeichert ist und in der die Protokolldaten 20 abgespeichert werden |
| 40 | Smartphone, gehört zur Erfassungs-Anordnung 100, umfasst die Eingabeeinheit 2, 5, 12, die Ausgabeeinheit 1, 6, jeden Eingabe-Parameter-Sensor 4, 7, 8, 15, 18, den Protokoll-Generierer 3, das Steuergerät 16 und die Kommunikationseinheit 9, erzeugt die Protokolldaten 20 |
| 100 | Erfassungs-Anordnung, umfasst das Smartphone 40, die Erfassungs-Auswerteeinheit 13 und den Darstellungs-Generierer 14, erzeugt die oder jede Darstellungsdatei 21.1,21.2 |
| 200 | Tablet, wird vom Arzt Me benutzt, stellt auf Basis der Darstellungsdatei 21.1 die Darstellung Da dar, fungiert als die Ausgabe-Anordnung |
| An1, An2 | Antworten auf die Fragen Qu1?, Qu2? |
| Be | Benutzer, verwendet die Erfassungs-Anordnung 100, um die Fragen Qu1?, Qu2?, Qu3?des Fragebogens Qu zu beantworten, kann der Patient Pt oder eine andere Person sein |
| Da | Darstellung, enthält die Fragen Qu1?, Qu2?, Qu3? des Fragebogens Qu und die Antworten An1, An2 auf diese Fragen, stellt die Darstellungsdatei 21.1 visuell wahrnehmbar dar, wird auf dem Tablet 200 dargestellt |
| HE | Hervorhebungs-Element |
| Im.Be | Bild von einem Benutzer der Erfassungs-Anordnung 100, von der Kamera 4 erzeugt |
| Im.Pt | Referenzbild des Patienten Pt, in der Datenbank 30 abgespeichert |
| Me | Arzt, verwendet einen tragbaren Rechner in Form des Tablets 200 |
| Pt | Patient, auf den sich die Fragen des Fragebogens Qu beziehen, kann der Benutzer Be sein |
| Qu | Fragebogen, der auf der Ausgabeeinheit 1 visuell ausgegeben wird, umfasst die Fragen Qu1?, Qu2?, Qu3? |
| Qu1?, Qu2?, Qu3? | Fragen des Fragebogens Qu |
| ZI | Zusatzinformation zu einer hervorgehoben dargestellten Antwort |

## Patentansprüche

1. Erfassungs-Anordnung (100) zum Erfassen von Benutzereingaben,
wobei die Erfassungs-Anordnung (100)
- eine Ausgabeeinheit (1, 6),
- eine Eingabeeinheit (2, 5, 12),
- einen Eingabe-Parameter-Sensor (4, 7, 8, 15, 18),
- einen signalverarbeitenden Protokoll-Generierer (3),
- eine signalverarbeitende Erfassungs-Auswerteeinheit (13, 25) und
- einen signalverarbeitenden Darstellungs-Generierer (14)
umfasst,
wobei in rechnerauswertbarer Form ein Fragebogen (Qu) mit mehreren Fragen und eine Fragen-Gruppe vorgegeben sind,
wobei die Fragen-Gruppe mindestens eine Frage des Fragebogens (Qu) umfasst,
wobei ein Eingabe-Parameter vorgegeben ist,
wobei der Eingabe-Parameter ein messbares Verhalten eines Benutzers (Be) der Erfassungs-Anordnung (100) bei der Eingabe einer Antwort auf eine Frage der Fragen-Gruppe beschreibt,
wobei die Ausgabeeinheit (1, 6) dazu ausgestaltet ist, die Fragen des Fragebogens (Qu) in einer von einem Menschen wahrnehmbaren Form an den Benutzer (Be) auszugeben,
wobei die Eingabeeinheit (2, 5, 12) dazu ausgestaltet ist, eine Antwort des Benutzers (Be) auf eine Frage, die im Fragebogen (Qu) enthalten und mittels der Ausgabeeinheit (1, 6) ausgegeben worden ist, zu erfassen,
wobei der Eingabe-Parameter-Sensor (4, 7, 8, 15, 18)
- dem vorgegebenen Eingabe-Parameter zugeordnet ist und
- dazu ausgestaltet ist, für jede Frage der Fragen-Gruppe jeweils zu messen, welchen Wert der zugeordnete Eingabe-Parameter annimmt, während die Eingabeeinheit (2, 5, 12) eine Antwort des Benutzers (Be) auf diese Frage erfasst,
wobei der Protokoll-Generierer (3) dazu ausgestaltet ist, Protokolldaten (20) zu erzeugen,
wobei die Protokolldaten (20) in einer rechnerauswertbaren Form für jede Frage des Fragebogens (Qu)
- eine Kennzeichnung der Frage und
- eine Kennzeichnung der erfassten Antwort oder eine Kennzeichnung, dass keine Antwort auf diese Frage erfasst worden ist,
umfassen,
wobei die Protokolldaten (20) weiterhin in einer rechnerauswertbaren Form für jede Frage der Fragen-Gruppe und für den Eingabe-Parameter jeweils eine Kennzeichnung des gemessenen Werts, den der Eingabe-Parameter bei der Erfassung der Antwort auf die Frage angenommen hat, umfassen,
wobei die Erfassungs-Auswerteeinheit (13, 25) dazu ausgestaltet ist,
- für mindestens eine Frage der Fragen-Gruppe auf den Wert, den der Eingabe-Parameter bei der Erfassung der Antwort auf die Frage angenommen hat,
optional auf mindestens zwei Eingabe-Parameter-Werte für zwei verschiedene Fragen,
eine in einer rechnerauswertbaren Form vorgegebene Auswerteregel anzuwenden,
- durch die Anwendung der Auswerteregel ein Auswertungsergebnis zu erzeugen und
- Auswertungsdaten (22) zu erzeugen,
wobei die Auswertungsdaten (22) in einer rechnerauswertbaren Form das oder jedes erzeugte Auswertungsergebnis umfassen,
wobei der Darstellungs-Generierer (14) dazu ausgestaltet ist, unter Verwendung der Protokolldaten (20) und der Auswertungsdaten (22) eine Darstellungsdatei (21.1, 21.2) zu erzeugen,
wobei die erzeugte Darstellungsdatei (21.1, 21.2)
- für jede Frage der Fragen-Gruppe die erfasste Antwort oder die Aussage, dass keine Antwort erfasst worden ist, sowie das oder jedes für diese Frage erzeugte Auswertungsergebnis umfasst und
- dergestalt ausgestaltet ist, dass auf Basis der Darstellungsdatei (21.1, 21.2) eine Darstellung (Da) in einer von einem Menschen wahrnehmbaren Form ausgegeben werden kann, insbesondere visuell ausgegeben werden kann.

2. Erfassungs-Anordnung (100) nach Anspruch 1,
**dadurch gekennzeichnet, dass**
mindestens einer der folgenden Eingabe-Parameter vorgegeben ist:
- ob eine Frage beantwortet worden ist oder nicht, wobei eine Frage beantwortet worden ist, wenn die Eingabeeinheit (2, 5, 12) den Abschluss einer Eingabe einer Antwort auf die Frage erfasst hat,
- wie lange der Beantwortungs-Zeitraum für diese Frage ist,
- wie viele Verzögerungen bei der Eingabe und / oder Schwankungen der Eingabegeschwindigkeit im Beantwortungs-Zeitraum für die Frage aufgetreten sind,
- wie oft eine bereits getätigte Eingabe im Beantwortungs-Zeitraum wieder geändert worden ist,
wobei der Beantwortungs-Zeitraum für eine Frage
- an dem Zeitpunkt beginnt, an dem die Ausgabe der Frage abgeschlossen wurde, und
an dem Zeitpunkt endet, an dem die Eingabe einer Antwort auf die Frage beendet wurde oder feststeht, dass die Frage nicht beantwortet ist.

3. Erfassungs-Anordnung (100) nach einem der vorhergehenden Ansprüche,
**dadurch gekennzeichnet, dass**
der Eingabe-Parameter-Sensor (4, 7, 8, 15, 18) ein Bildaufnahmegerät (4) umfasst,
wobei das Bildaufnahmegerät (4) dazu ausgestaltet ist, für die oder mindestens eine, bevorzugt für jede Frage der Fragen-Gruppe jeweils mindestens ein Bild (Im.Be), bevorzugt eine Bilder-Abfolge, dergestalt zu erzeugen,
dass das erzeugte Bild (Im.Be) einen Benutzer (Be) der Erfassungs-Anordnung (100) zeigt, während die Ausgabeeinheit (1, 6) die Frage ausgibt und / oder während der Benutzer (Be) mit der Eingabeeinheit (2, 5, 12) eine Antwort auf die Frage eingibt.

4. Erfassungs-Anordnung (100) nach Anspruch 3,
**dadurch gekennzeichnet, dass**
der oder ein vorgegebener Eingabe-Parameter ist,
- ob bei der Eingabe der Antwort auf eine Frage in einem Bild (Im.Be) mindestens eine Person dargestellt wird und / oder
- ob im Bild (Im.Be) bei der Eingabe der Antwort auf eine Frage zusätzlich zum die Antwort eingebenden Benutzer (Be) eine weitere Person dargestellt wird,
wobei das Bildaufnahmegerät (4) dieses Bild (Im.Be) erzeugt hat, während die Ausgabeeinheit (1, 6) die Frage ausgibt und / oder während der Benutzer (Be) mit der Eingabeeinheit (2, 5, 12) eine Antwort auf die Frage eingibt.

5. Erfassungs-Anordnung (100) nach Anspruch 3 oder Anspruch 4,
**dadurch gekennzeichnet, dass**
der Protokoll-Generierer (3) dazu ausgestaltet ist, die Protokolldaten (20) dergestalt zu erzeugen,
dass die Protokolldaten (20) für mindestens eine Frage der Fragen-Gruppe das oder mindestens ein Bild (Im.Be) umfassen,
wobei das Bildaufnahmegerät (4) dieses Bild (Im.Be) erzeugt hat, während diese Frage ausgegeben und / oder eine Antwort auf diese Frage eingegeben worden ist, und
wobei bevorzugt der Darstellungs-Generierer (14) dazu ausgestaltet ist, die oder eine Darstellungsdatei (21.1, 21.2) dergestalt zu erzeugen,
dass eine auf Basis der Darstellungsdatei (21.1, 21.2) ausgegebene Darstellung (Da) für mindestens eine Frage der Fragen-Gruppe das Bild (Im.Be) zeigt, welches im Beantwortungs-Zeitraum für diese Frage erzeugt worden ist und von den Protokolldaten (20) umfasst ist.

6. Erfassungs-Anordnung (100) nach einem der Ansprüche 3 bis 5,
**dadurch gekennzeichnet, dass**
die Fragen des Fragebogens (Qu) sich auf eine Person (Pt) beziehen,
die Erfassungs-Auswerteeinheit (13, 25) eine signalverarbeitende Bildvergleichseinheit (25) umfasst und
in rechnerauswertbarer Form ein Referenzbild (Im.Pt) des Gesichts derjenigen Person (Pt), auf die sich die Fragen beziehen, vorgegeben ist,
wobei die Bildvergleichseinheit (25) dazu ausgestaltet ist, für die oder jede Frage der Fragen-Gruppe
- das Bild (Im.Be), welches das Bildaufnahmegerät (4) im Beantwortungs-Zeitraum für diese Frage erzeugt hat, mit dem vorgegebenen Referenzbild (Im.Pt) zu vergleichen und
- auf Basis des Vergleichs zu entscheiden, ob die Person (Pt), die im Referenzbild (Im.Pt) gezeigt wird, oder eine andere Person (Be) eine erfasste Antwort auf die Frage eingegeben hat, und
wobei die Erfassungs-Auswerteeinheit (13, 25) dazu ausgestaltet ist,
- jede Frage zu identifizieren, die von einer anderen Person (Be) als von der im Referenzbild (Im.Pt) gezeigten Person (Pt) beantwortet worden ist, und
- die Auswertungsdaten (22) dergestalt zu erzeugen,
dass die Auswertungsdaten (22) jeweils eine Kennzeichnung jeder Frage, die als eine von einer anderen Person (Be) beantwortete Frage identifiziert worden ist, umfassen.

7. Erfassungs-Anordnung (100) nach Anspruch 6,
**dadurch gekennzeichnet, dass**
der Darstellungs-Generierer (14) dazu ausgestaltet ist, die Darstellungsdatei (21.1, 21.2) dergestalt zu erzeugen,
dass in einer auf Basis der Darstellungsdatei (21.1, 21.2) ausgegebenen Darstellung (Da) jede Frage der Fragen-Gruppe gekennzeichnet ist, die von einer anderen Person (Be) als von der im Referenzbild (Im.Pt) gezeigten Person (Pt) beantwortet worden ist, und
bevorzugt die Darstellung (Da) zusätzlich das Bild (Im.Be) zeigt, welches vom Bildaufnahmegerät (4) während des Beantwortungs-Zeitraums für diese Frage erzeugt worden ist.

8. Erfassungs-Anordnung (100) nach einem der vorhergehenden Ansprüche,
**dadurch gekennzeichnet, dass**
der Eingabe-Parameter-Sensor (4, 7, 8, 15, 18) eine Audiosignal-Erfassungseinheit (7) umfasst und
die Erfassungs-Auswerteeinheit (13, 25) eine Audiosignal-Auswerteeinheit umfasst,
wobei die Audiosignal-Erfassungseinheit (7) dazu ausgestaltet ist, ein Audiosignal zu erfassen, das in einer Umgebung der Erfassungs-Anordnung (100) entstanden ist,
wobei die Audiosignal-Auswerteeinheit dazu ausgestaltet ist,
- zu entscheiden, ob ein erfasstes Audiosignal mindestens eine Sprachnachricht umfasst oder nicht, und
- dann, wenn das Audiosignal mindestens eine Sprachnachricht umfasst, zu zählen, von wie vielen verschiedenen Menschen die Sprachnachrichten, die im Audiosignal enthalten sind, insgesamt stammen, und
wobei der oder ein Eingabe-Parameter ist, wie viele Personen in einem Beantwortungs-Zeitraum für eine Frage insgesamt gesprochen haben.

9. Erfassungs-Anordnung (100) nach einem der vorhergehenden Ansprüche,
**dadurch gekennzeichnet, dass**
die Eingabeeinheit (2, 5, 12) eine Tastatur (2) umfasst und
der oder ein vorgegebener Eingabe-Parameter ist,
- wie schnell ein Benutzer (Be) der Erfassungs-Anordnung (100) im Beantwortungs-Zeitraum für eine Frage die Tastatur (2) benutzt, um eine Antwort auf die Frage einzugeben,
- wie zielsicher und / oder treffsicher der Benutzer (Be) die Tastatur (2) im Beantwortungs-Zeitraum für die Frage benutzt, um eine Antwort auf die Frage einzugeben,
- wie oft ein Benutzer (Be) eine Eingabe mit der Tastatur (2) gelöscht und / oder korrigiert hat,
- welche Schwankungen bei der Geschwindigkeit der Eingabe mittels der Tastatur (2) aufgetreten sind.

10. Erfassungs-Anordnung (100) nach einem der vorhergehenden Ansprüche,
**dadurch gekennzeichnet, dass**
die Eingabeeinheit (2, 5, 12) eine Spracheingabeeinheit (5) umfasst und
der oder ein Eingabe-Parameter-Sensor (4, 7, 8, 15, 18) einen Spracherkenner umfasst,
wobei die Spracheingabeeinheit (5) dazu ausgestaltet ist, ein Audiosignal zu erfassen,
wobei der Spracherkenner dazu ausgestaltet ist, aus einem Audiosignal eine Textnachricht zu erzeugen, und
der oder ein vorgegebener Eingabe-Parameter ist,
- mit welcher Lautstärke ein Benutzer (Be) der Erfassungs-Anordnung (100) im Beantwortungs-Zeitraum für eine Frage eine Antwort auf die Frage in die Spracheingabeeinheit (5) eingegeben hat,
- mit welcher Sprechgeschwindigkeit und / oder mit welchem Sprachfluss der Benutzer (Be) im Beantwortungs-Zeitraum für die Frage eine Antwort auf die Frage in die Spracheingabeeinheit (5) eingegeben hat,
- wie oft ein Benutzer eine Spracheingabe zurückgenommen und / oder korrigiert hat,
- wie viele Verzögerungen und / oder Stockungen und / oder welche Schwankungen der Lautstärke und / oder der Sprechgeschwindigkeit während der Eingabe der Antwort auf eine Frage in die Spracheingabeeinheit (5) aufgetreten sind,
- mit welcher Zuverlässigkeit oder Sicherheit der Spracherkenner aus dem Audiosignal eine Textnachricht erzeugt hat.

11. Erfassungs-Anordnung nach einem der vorhergehenden Ansprüche,
**dadurch gekennzeichnet, dass**
die Ausgabeeinheit (1, 6) eine Anzeigefläche (1) umfasst,
wobei die Ausgabeeinheit (1, 6) dazu ausgestaltet ist, eine Frage des Fragebogens (Qu) auf der Anzeigefläche (1) visuell mit einer von einem Benutzer (Be) der Erfassungs-Anordnung (100) einstellbaren Zeichengröße darzustellen, und
wobei ein vorgegebener Eingabe-Parameter ist,
welche Zeichengröße auf der Anzeigefläche (1) bei der Ausgabe der Frage eingestellt worden ist.

12. Erfassungs-Anordnung (100) nach einem der vorhergehenden Ansprüche,
**dadurch gekennzeichnet, dass**
die Ausgabeeinheit (1, 6) eine Sprachausgabeeinheit (6) umfasst,
wobei die Sprachausgabeeinheit (6) dazu ausgestaltet ist, eine Frage des Fragebogens (Qu) mit einer von einem Benutzer (Be) der Erfassungs-Anordnung (100) einstellbaren Lautstärke auszugeben, und
wobei ein vorgegebener Eingabe-Parameter ist,
welche Lautstärke für die Sprachausgabeeinheit (6) bei der Ausgabe einer Frage eingestellt worden ist.

13. Erfassungs-Anordnung (100) nach einem der vorhergehenden Ansprüche,
**dadurch gekennzeichnet, dass**
der oder ein vorgegebener Eingabe-Parameter einen Vitalparameter umfasst und
der oder ein Eingabe-Parameter-Sensor ein Vitalparameter-Sensor (15) ist,
wobei der Vitalparameter-Sensor (15) dazu ausgestaltet ist, einen Vitalparameter eines Benutzers (Be) der Erfassungs-Anordnung (100) zu messen,
insbesondere die Herzschlagfrequenz oder den Blutdruck, und
wobei bevorzugt der Vitalparameter-Sensor (15) dazu ausgestaltet ist, für jede Frage der Fragen-Gruppe jeweils zu messen, welchen Wert der Vitalparameter annimmt, während die Eingabeeinheit (2, 5, 12) eine Antwort des Benutzers (Be) auf diese Frage erfasst.

14. Erfassungs-Anordnung (100) nach einem der vorhergehenden Ansprüche,
**dadurch gekennzeichnet, dass**
die Erfassungs-Anordnung (100) ein tragbares Gerät (40) umfasst,
wobei das tragbare Gerät (40) dazu ausgestaltet ist, von einem Menschen (Be) in einer Hand gehalten zu werden,
wobei mindestens die Ausgabeeinheit (1, 6), die Eingabeeinheit (2, 5, 12) und der Eingabe-Parameter-Sensor (4, 7, 8, 15, 18) Bestandteile des tragbaren Geräts (40) sind,
wobei das tragbare Gerät (40) weiterhin als einen Eingabe-Parameter-Sensor einen Bewegungssensor (18) umfasst,
wobei der Bewegungssensor (18) dazu ausgestaltet ist, eine Bewegung des tragbaren Geräts (40) im Raum zu messen, und
wobei der oder ein Eingabe-Parameter die gemessene Bewegung des tragbaren Geräts (40) im Raum im Beantwortungs-Zeitraum für eine Frage ist.

15. Erfassungs-Anordnung (100) nach einem der vorhergehenden Ansprüche,
**dadurch gekennzeichnet, dass**
die Erfassungs-Anordnung (100) für jede Frage der Fragen-Gruppe jeweils wahlweise in einem Eingabemodus oder in einem Rückfragemodus betreibbar ist,
wobei die Erfassungs-Anordnung (100) so ausgestaltet ist, dass die Erfassungs-Anordnung dann, wenn die Ausgabeeinheit (1, 6) eine Frage der Fragen-Gruppe ausgibt,
eine Eingabe in die Eingabeeinheit (2, 5, 12)
- dann, wenn die Erfassungs-Anordnung (100) im Eingabemodus betrieben wird, als Antwort auf eine Frage verwendet und
- dann, wenn die Erfassungs-Anordnung (100) im Rückfragemodus betrieben wird, als Rückfrage auf die ausgegebene Frage verwendet,
wobei der oder ein vorgegebener Eingabe-Parameter ist,
- wie viele Rückfragen der Benutzer zu einer Frage der Fragen-Gruppe gestellt hat,
- wie viele Rückfragen mit dem gleichen Inhalt der Benutzer zu derselben Frage der Fragen-Gruppe gestellt hat.

16. Erfassungs-Anordnung (100) nach einem der vorhergehenden Ansprüche,
**dadurch gekennzeichnet, dass**
für den Eingabe-Parameter ein Soll-Wertebereich vorgegeben ist, optional jeweils ein Soll-Wertebereich pro Frage der Fragen-Gruppe,
wobei die Erfassungs-Auswerteeinheit (13, 25) dazu ausgestaltet ist,
- für jede Frage der Fragen-Gruppe und für den gemessenen Eingabe-Parameter zu entscheiden,
ob der Wert des Eingabe-Parameters, der bei der Beantwortung der Frage gemessen worden ist, innerhalb oder außerhalb des vorgegebenen Soll-Wertebereichs des Eingabe-Parameters liegt, und
- mindestens dann, wenn der Wert außerhalb des Soll-Wertebereichs liegt, die Auswertungsdaten (22) dergestalt zu erzeugen,
dass die Auswertungsdaten (22) eine Kennzeichnung der Frage und eine Kennzeichnung des außerhalb des Soll-Wertebereichs liegenden Werts umfassen.

17. Erfassungs-Anordnung (100) nach Anspruch 16,
**dadurch gekennzeichnet, dass**
der Darstellungs-Generierer (14) dazu ausgestaltet ist, die Darstellungsdatei (21.1, 21.2) dergestalt zu erzeugen,
dass mindestens dann, wenn der gemessene Eingabe-Parameter-Wert außerhalb des vorgegebenen Soll-Wertebereichs liegt,
eine auf Basis der Darstellungsdatei (21.1, 21.2) ausgegebene Darstellung (Da) zusätzlich zur erfassten Antwort eine Kennzeichnung des Eingabe-Parameters und des gemessenen Eingabe-Parameter-Werts umfasst.

18. Erfassungs-Anordnung (100) nach einem der vorhergehenden Ansprüche,
**dadurch gekennzeichnet, dass**
die Erfassungs-Auswerteeinheit (13, 25) zusätzlich eine signalverarbeitende Inkonsistenz-Auswerteeinheit umfasst,
wobei die Inkonsistenz-Auswerteeinheit dazu ausgestaltet ist, automatisch
- zu entscheiden, ob die erfassten Antworten in sich konsistent sind oder mindestens zwei erfasste Antworten zueinander inkonsistent sind, und
- jedes Inkonsistenz-Paar zu detektieren, wobei ein Inkonsistenz-Paar aus mindestens zwei Antworten besteht, die zueinander inkonsistent sind, und
wobei die Erfassungs-Auswerteeinheit (13, 25) dazu ausgestaltet ist, die Auswertungsdaten (22) dergestalt zu erzeugen, dass die erzeugten Auswertungsdaten (22) jeweils eine Kennzeichnung jedes detektierten Inkonsistenz-Paars umfassen, und
wobei bevorzugt der Darstellungs-Generierer (14) dazu ausgestaltet ist, die Darstellungsdatei (21.1, 21.2) dergestalt zu erzeugen,
dass eine auf Basis der Darstellungsdatei (21.1, 21.2) ausgegebene Darstellung (Da) jeweils eine Kennzeichnung jedes detektierten Inkonsistenz-Paars umfasst.

19. Erfassungs-Anordnung (100) nach Anspruch 18,
**dadurch gekennzeichnet, dass**
in einer rechnerauswertbaren Form eine Inkonsistenz-Regel vorgegeben ist und
die Inkonsistenz-Auswerteeinheit dazu ausgestaltet ist,
- die vorgegebene Inkonsistenz-Regel auf die erfassten Antworten eines Benutzers (Be) der Erfassungs-Anordnung (100) auf die Fragen des Fragebogens (Qu) anzuwenden,
- durch die Anwendung zu entscheiden, ob die erfassten Antworten in sich konsistent sind oder mindestens zwei erfasste Antworten zueinander inkonsistent sind, und
- jedes Inkonsistenz-Paar zu detektieren, wobei ein Inkonsistenz-Paar aus mindestens zwei Antworten besteht, die gemäß der Inkonsistenz-Regel zueinander inkonsistent sind.

20. Erfassungs-Anordnung (100) nach einem der Ansprüche 16 bis19,
**dadurch gekennzeichnet, dass**
in einer rechnerausführbaren Form ein Test vorgegeben ist,
wobei der Test eine Handlung beschreibt, die ein Benutzer (Be) der Erfassungs-Anordnung (100) mithilfe der Eingabeeinheit (2, 5, 12) vornehmen soll,
wobei die Erfassungs-Auswerteeinheit (13, 25) dazu ausgestaltet ist,
mindestens dann, wenn ein gemessener Wert des oder eines Eingabe-Parameters außerhalb des für diesen Eingabe-Parameter vorgegebenen Soll-Wertebereichs liegt oder wenn mindestens ein Inkonsistenz-Paar detektiert worden ist,
- zu bewirken, dass die Ausgabeeinheit (1, 6) den Test ausgibt,
- zu bewirken, dass die Eingabeeinheit (2, 5, 12) eine Reaktion des Benutzers (Be) auf die Ausgabe des Tests erfasst, und
- die Auswertungsdaten (22) dergestalt zu erzeugen, dass die Auswertungsdaten (22) eine rechnerauswertbare Repräsentation der erfassten Reaktion des Benutzers (Be) auf die Ausgabe des Tests umfassen, und
wobei bevorzugt der Darstellungs-Generierer (14) dazu ausgestaltet ist, die Darstellungsdatei (21.1, 21.2) dergestalt zu erzeugen,
dass eine auf Basis der Darstellungsdatei (21.1, 21.2) ausgegebene Darstellung (Da) die von den Auswertungsdaten (22) umfasste Repräsentation der erfassten Reaktion des Benutzers (Be) umfasst.

21. Erfassungs-Anordnung (100) nach einem der vorhergehenden Ansprüche,
**dadurch gekennzeichnet, dass**
die oder mindestens eine Auswerteregel sich auf den oder einen Eingabe-Parameter bezieht und eine Aggregierungsvorschrift spezifiziert,
wobei die Aggregierungsvorschrift mehrere Werte dieses Eingabe-Parameters, die für unterschiedliche Fragen der Fragen-Gruppe gemessen worden sind, zu einer Einzelbewertung für diesen Eingabe-Parameter zusammenfasst,
wobei bevorzugt für jede Frage der Fragen-Gruppe jeweils ein Gewichtsfaktor vorgegeben ist und die oder mindestens eine, bevorzugt jede Aggregierungsvorschrift diesen Gewichtsfaktor enthält.

22. Erfassungs-Anordnung (100) nach Anspruch 21,
**dadurch gekennzeichnet, dass**
die Erfassungs-Auswerteeinheit (13, 25) dazu ausgestaltet ist, eine Vorhersage für die Dauer eines Gesprächs zu berechnen,
wobei dieses Gespräch mit einer Person (Pt) geführt werden soll, auf die sich die Fragen des ausgegebenen Fragebogens (Qu) beziehen,
wobei die Erfassungs-Auswerteeinheit (13, 25) dazu ausgestaltet ist, für die Berechnung der Vorhersage die jeweilige Einzelbewertung des oder eines Eingabe-Parameters, bevorzugt mehrere Eingabe-Parameter zu verwenden,
wobei die Erfassungs-Auswerteeinheit (13, 25) bevorzugt dazu ausgestaltet ist, für die Berechnung der Vorhersage zusätzlich
- eine vorgegebene Standarddauer eines solchen Gesprächs und / oder
- die Anzahl von detektierten Inkonsistenz-Paaren und / oder
- die Anzahl von erfassten Korrekturen oder Rückfragen bei der Beantwortung der Fragen
zu verwenden,
wobei bevorzugt der oder ein Eingabe-Parameter, dessen Einzelbewertung verwendet wird, einer der folgenden Eingabe-Parameter ist:
- der Beantwortungs-Zeitraum, der zwischen der Ausgabe einer Frage und dem Abschluss der Eingabe der Antwort auf diese Frage verstreicht,
- die Anzahl der Rückfragen,
- die Anzahl der Fragen, die nicht oder nicht ausschließlich von derjenigen Person beantwortet worden sind, auf die sich die Fragen des ausgegebenen Fragebogens (Qu) beziehen,
- ein gemessener Vitalparameter.

23. Erfassungs-Anordnung (100) nach einem der vorhergehenden Ansprüche,
**dadurch gekennzeichnet, dass**
als die oder eine Auswerteregel ein Hervorhebungs-Kriterium vorgegeben ist, bevorzugt mindestens zwei verschiedene Hervorhebungs-Kriterien vorgegeben sind,
wobei das oder jedes Hervorhebungs-Kriterium abhängig von mindestens einem Eingabe-Parameter jeweils ein Hervorhebungs-Element (HE) spezifiziert,
wobei die Erfassungs-Auswerteeinheit (13, 25) dazu ausgestaltet ist, für jede Frage der Fragen-Gruppe zu prüfen, ob für die Frage
- der oder ein Eingabe-Parameter-Wert, der bei der Erfassung der Antwort auf diese Frage gemessen worden ist, oder
- die Kombination von Eingabe-Parameter-Werten, die bei der Erfassung der Antwort gemessen worden sind,
das Hervorhebungs-Kriterium erfüllt ist,
wobei die Erfassungs-Auswerteeinheit (13, 25) weiterhin dazu ausgestaltet ist,
dann, wenn für eine Frage der Fragen-Gruppe das Hervorhebungs-Kriterium erfüllt ist,
die Auswertungsdaten (22) dergestalt zu erzeugen, dass die Auswertungsdaten (22) eine Kennzeichnung der Frage und eine Kennzeichnung des erfüllten Hervorhebungs-Elements (HE) umfassen,
wobei bevorzugt der Darstellungs-Generierer (14) dazu ausgestaltet ist, die Darstellungsdatei (21.1, 21.2) dergestalt zu erzeugen,
dass eine auf Basis der Darstellungsdatei (21.1, 21.2) ausgegebene Darstellung (Da) die Antwort auf diese Frage und dann, wenn das Hervorhebungs-Kriterium erfüllt ist, zusätzlich das im erfüllten Hervorhebungs-Kriterium spezifizierte Hervorhebungs-Element (HE) darstellt.

24. Anordnung umfassend
- eine Erfassungs-Anordnung (100) nach einem der vorhergehenden Ansprüche und
- eine datenverarbeitende Darstellungs-Anordnung (200),
wobei die Darstellungs-Anordnung (200) dazu ausgestaltet ist,
- die vom Darstellungs-Generierer (14) der Erfassungs-Anordnung (100) erzeugte Darstellungsdatei (21.1, 21.2) zu erfassen und
- auf Basis der erfassten Darstellungsdatei (21.1, 21.2) eine Darstellung (Da) zu generieren und in einer von einem Menschen wahrnehmbaren Form, insbesondere visuell, auszugeben.

25. Erfassungs-Verfahren zum Erfassen von Benutzereingaben,
wobei das Erfassungs-Verfahren unter Verwendung einer Erfassungs-Anordnung (100) durchgeführt wird,
wobei die Erfassungs-Anordnung (100)
- eine Ausgabeeinheit (1, 6),
- eine Eingabeeinheit (2, 5, 12),
- einen Eingabe-Parameter-Sensor (4, 7, 8, 15, 18)
- einen signalverarbeitenden Protokoll-Generierer (3),
- eine signalverarbeitende Erfassungs-Auswerteeinheit (13, 25) und
- einen signalverarbeitenden Darstellungs-Generierer (14)
umfasst,
wobei in rechnerauswertbarer Form ein Fragebogen (Qu) mit mehreren Fragen und eine Fragen-Gruppe vorgegeben werden,
wobei die Fragen-Gruppe mindestens eine Frage des Fragebogens (Qu) umfasst,
wobei ein Eingabe-Parameter vorgegeben wird,
wobei der vorgegebene Eingabe-Parameter ein messbares Verhalten eines Benutzers (Be) der Erfassungs-Anordnung (100) bei der Eingabe einer Antwort auf eine Frage der Fragen-Gruppe beschreibt,
wobei der Eingabe-Parameter-Sensor (4, 7, 8, 15, 18) einem vorgegebenen Eingabe-Parameter zugeordnet ist,
wobei das Erfassungs-Verfahren die Schritte umfasst, dass
die Ausgabeeinheit (1, 6) die Fragen des Fragebogens (Qu) in einer von einem Menschen wahrnehmbaren Form an den Benutzer (Be) ausgibt,
die Eingabeeinheit (2, 5, 12) eine Antwort des Benutzers (Be) auf eine Frage, die im Fragebogen (Qu) enthalten und mittels der Ausgabeeinheit (1, 6) ausgegeben worden ist, erfasst,
der Eingabe-Parameter-Sensor (4, 7, 8, 15, 18) für die oder jede Frage der Fragen-Gruppe jeweils misst, welchen Wert der zugeordnete Eingabe-Parameter annimmt, während die Ausgabeeinheit (1, 6) eine Antwort des Benutzers (Be) auf diese Frage erfasst,
der Protokoll-Generierer (3) Protokolldaten (20) erzeugt,
wobei die Protokolldaten (20) in einer rechnerauswertbaren Form für jede Frage des Fragebogens (Qu)
- eine Kennzeichnung der Frage und
- eine Kennzeichnung der erfassten Antwort oder eine Kennzeichnung, dass keine Antwort auf diese Frage erfasst worden ist,
umfassen,
wobei die Protokolldaten (20) weiterhin in einer rechnerauswertbaren Form für jede Frage der Fragen-Gruppe und für den Eingabe-Parameter eine Kennzeichnung des gemessenen Werts, den der Eingabe-Parameter bei der Erfassung der Antwort auf die Frage angenommen hat, umfassen,
die Erfassungs-Auswerteeinheit (13, 25)
- für mindestens eine Frage der Fragen-Gruppe auf den Wert, den der Eingabe-Parameter bei der Erfassung der Antwort auf die Frage angenommen hat,
optional auf mindestens zwei Eingabe-Parameter-Werte für zwei verschiedene Fragen,
eine in einer rechnerauswertbaren Form vorgegebene Auswerteregel anwendet,
- durch die Anwendung der Auswerteregel ein Auswertungsergebnis erzeugt und
- Auswertungsdaten (22) erzeugt,
wobei die Auswertungsdaten (22) in einer rechnerauswertbaren Form das oder jedes erzeugte Auswertungsergebnis umfassen, und
der Darstellungs-Generierer (14) unter Verwendung der Protokolldaten (20) und der Auswertungsdaten (22) eine Darstellungsdatei (21.1, 21.2) erzeugt,
wobei die erzeugte Darstellungsdatei (21.1, 21.2) für jede Frage der Fragen-Gruppe
- die jeweils erfasste Antwort oder die Aussage, dass keine Antwort erfasst worden ist, sowie das oder jedes für diese Frage erzeugte Auswertungsergebnis umfasst und
- dergestalt ausgestaltet ist, dass auf Basis der Darstellungsdatei (21.1, 21.2) eine Darstellung (Da) in einer von einem Menschen wahrnehmbaren Form ausgegeben werden kann, insbesondere visuell ausgegeben werden kann.

26. Computerprogramm, welches
dazu ausgestaltet ist, auf einem Rechner ausgeführt zu werden, und
bei der Ausführung auf dem Rechner bewirkt, dass mithilfe des Rechners ein Erfassungs-Verfahren nach Anspruch 25 zum Erfassen von Benutzereingaben durchgeführt wird.
